# EUROPEAN PATENT APPLICATION

(11) **EP 4 840 885 A1**
(43) Date of publication of application: **18.11.2026**
(21) Application number: 25739186.2
(22) Date of filing: 13.01.2025
(51) Int. Cl.: C07K 16/28, C07K 16/22, A61P 27/02, A61P 35/00, A61K 39/00

(54) **BISPECIFIC ANTIBODY SPECIFICALLY BINDING TO C-KIT AND VEGF, AND USE THEREOF**

(30) Priority: 12.01.2024 KR 20240005633; 10.01.2025 KR 20250004411
(71) Applicant: NOVELTY NOBILITY Inc., Gyeonggi-do, 13477 (KR)
(72) Inventor: PARK, Sang Gyu, Seongnam-si, Gyeonggi-do 13477 (KR); NA, Tae Young, Seongnam-si, Gyeonggi-do 13628 (KR)
(74) Representative: SONN Patentanwälte GmbH & Co KG
(86) International application number: PCT/KR2025/000744
(87) International publication number: WO 2025/151018

(57) **Abstract**

The present invention relates to a bispecific antibody specifically binding to c-Kit and VEGF and, more specifically, to: a bispecific antibody capable of inhibiting or neutralizing the activity or activation of c-Kit and VEGF by binding to c-Kit and VEGF with high affinity; a nucleic acid molecule encoding the bispecific antibody; a vector comprising the nucleic acid molecule; a method for preparing the bispecific antibody; a pharmaceutical composition for preventing or treating angiogenic diseases, the composition including the bispecific antibody as an active ingredient; a method for preventing or treating angiogenic diseases using the bispecific antibody; and a c-Kit and/or VEGF detection or angiogenic disease diagnosis kit comprising the bispecific antibody.

## Description

### TECHNICAL FIELD

This application claims priority to and the benefit of Korean Patent Application No. 10-2024-0005633, filed on January 12, 2024, and all contents disclosed in the specification and drawings of the application are incorporated in this application.

The present invention relates to a bispecific antibody that specifically binds to c-Kit and VEGF, and more specifically to a bispecific antibody that can bind to c-Kit and VEGF with high affinity and inhibit or neutralize the activity or activation of c-Kit and VEGF, a nucleic acid molecule encoding the bispecific antibody, a vector and a host cell including the nucleic acid molecule, a method for producing the bispecific antibody, a pharmaceutical composition for preventing or treating an angiogenic disease including the bispecific antibody as an active ingredient, a method for preventing or treating an angiogenic disease using the bispecific antibody, and a kit for detecting c-Kit and/or VEGF or diagnosing an angiogenic disease including the bispecific antibody.

### BACKGROUND ART

Angiogenesis is the process by which new capillaries are formed from existing microvessels. Normally, it occurs only during embryonic development, wound healing and cyclical changes in the female reproductive system, and rarely occurs under other normal conditions. However, if angiogenesis is not regulated autonomously and continues to grow pathologically, it causes various diseases. Diseases related to angiogenesis include hemangioma, angiofibroma, vascular malformation and cardiovascular diseases such as arteriosclerosis, vascular adhesions and edematous sclerosis; ophthalmic diseases caused by angiogenesis include age-related macular degeneration, corneal transplant neovascularization, angiogenic glaucoma, diabetic retinopathy, corneal diseases caused by neovascularization, macular degeneration, pterygium, retinal degeneration, retrolental fibroplasia and granular conjunctivitis; chronic inflammatory diseases such as arthritis; and dermatological diseases such as psoriasis, telangiectasia, pyogenic granuloma, seborrheic dermatitis and acne. The growth and metastasis of cancer cells are necessarily dependent on angiogenesis.

VEGF is a powerful angiogenesis promoter, and the expression thereof is increased in various cancer cells. VEGF plays an important role in the growth and metastasis of cancer cells by activating tyrosine kinase by binding to VEGF receptors on the surface of vascular endothelial cells, thereby inducing angiogenesis. Therefore, VEGF inhibitors are used to treat various cancers and macular degeneration by blocking angiogenesis. Currently, as recombinant antibody drugs, there are bevacizumab as an anticancer agent, ranibizumab and aflibercept as macular degeneration treatments, and sunitinib and sorafenib as kinase inhibitors. However, there are many patients who are refractory to the VEGF inhibitors, and various bispecific antibodies are being developed to supplement the same.

Meanwhile, when hypoxia occurs, the expression of c-KIT in endothelial cells increases, and the production of new blood vessels by SCF (stem cell factor) in endothelial cells increases. This pathological angiogenesis is a major cause of blindness in various eye diseases such as wet macular degeneration and diabetic retinopathy. When hypoxia occurs, the expression of HIF-1α (hypoxia inducible factor-1α) increases along with SCF and c-KIT, and in this case, SCF stabilizes HIF-1α to prolong hypoxia. If the hypoxic condition persists, a vicious cycle occurs in which VEGF/VEGFR increases again, and thus, blocking the SCF signal with a c-KIT inhibitor can remove the factor that stimulates angiogenesis by inhibiting HIF-1α. Accordingly, the inventors of the present invention developed an anti-c-Kit antibody that specifically binds to c-Kit and demonstrated its excellent angiogenesis inhibition effect (Patent Document 1).

Under this background, the inventors of the present invention developed a bispecific antibody that specifically binds to c-Kit and VEGF, and confirmed that the antibody can act as a therapeutic agent for angiogenic diseases, particularly macular degeneration, thereby completing the present invention.

### [Related Art Documents]

### [Patent Documents]

(Patent Document 0001) Korean Patent Laid-Open Publication No. 10-2020-0040407

### DISCLOSURE

### TECHNICAL PROBLEM

An object of the present invention is to provide a bispecific antibody that specifically binds to c-Kit and VEGF.

Another object of the present invention is to provide a nucleic acid molecule encoding the bispecific antibody.

Still another object of the present invention is to provide a vector including the nucleic acid molecule and a host cell including the vector.

Still another object of the present invention is to provide a method for producing a bispecific antibody that specifically binds to c-Kit and VEGF using the host cell.

Another object of the present invention is to provide a pharmaceutical composition for preventing or treating an angiogenic disease, including the bispecific antibody that specifically binds to c-Kit and VEGF described above as an active ingredient.

Still another object of the present invention is to provide a method for preventing or treating an angiogenic disease, using the bispecific antibody that specifically binds to c-Kit and VEGF described above.

Still another object of the present invention is to provide the use of the bispecific antibody specifically binding to c-Kit and VEGF described above in the manufacture of a medicament for preventing or treating an angiogenic disease.

Still another object of the present invention is to provide a kit for detecting c-Kit and/or VEGF or diagnosing an angiogenic disease, by using a bispecific antibody that specifically binds to c-Kit and VEGF.

However, the technical problems to be solved by the present invention are not limited to the problems mentioned above, and other problems that are not mentioned will be clearly understood by a person skilled in the art from the description below.

### TECHNICAL SOLUTION

In order to solve the above-described problems, the present invention provides a bispecific antibody that specifically binds to c-Kit and VEGF, including (a) and (b) below: (a) a first antigen binding site that specifically binds to c-Kit, including a heavy chain variable region including a heavy chain CDR1 including an amino acid sequence of SEQ ID NO: 2, a heavy chain CDR2 including an amino acid sequence of SEQ ID NO: 4, and a heavy chain CDR3 including an amino acid sequence of SEQ ID NO: 6; and a light chain variable region including a light chain CDR1 including an amino acid sequence of SEQ ID NO: 13, a light chain CDR2 including an amino acid sequence of SEQ ID NO: 15, and a light chain CDR3 including an amino acid sequence of SEQ ID NO: 17; and (b) a second antigen binding site that specifically binds to VEGF, wherein a soluble extracellular domain of VEGF and an Fc domain of IgG are fused.

In the present invention, the first antigen binding site may include a heavy chain variable region including an amino acid sequence having 90% or more sequence homology with an amino acid sequence of SEQ ID NO: 27 and a light chain variable region including an amino acid sequence having 90% or more sequence homology with an amino acid sequence of SEQ ID NO: 28.

In the present invention, the soluble extracellular domain may include immunoglobulin-like domain 2 of a first VEGF receptor and immunoglobulin-like domain 3 of a second VEGF receptor.

In the present invention, the second antigen binding site may include an amino acid sequence of SEQ ID NO: 21.

In the present invention, the second antigen binding site may be bound to a heavy chain C-terminus or N-terminus of the first antigen binding site by a peptide linker; or the second antigen binding site may be bound to a light chain C-terminus of the first antigen binding site by a peptide linker.

In the present invention, the peptide linker may be GGGGG (SEQ ID NO: 20), GGGGGG (SEQ ID NO: 67), GGGGGGG (SEQ ID NO: 68), GGGGGGGG (SEQ ID NO: 23), GGGGGGGGG (SEQ ID NO: 69) or GGGGGGGGGG (SEQ ID NO: 70).

In the present invention, an Fc domain of the first antigen binding site may include: (a) wild-type IgG Fc; (b) an Fc variant including L234A and L235E amino acid substitutions; (c) an Fc variant including L235A and G237A amino acid substitutions; or (d) an Fc variant including L234A, L235A, I253A, H310A, P329G and H435A amino acid substitutions, wherein residues are numbered according to the EU index of Kabat.

In the present invention, the bispecific antibody may be a tetravalent antibody.

In the present invention, the first antigen binding site may be a full-length divalent antibody, and the second antigen binding site may consist of two VEGF antagonists in which a soluble extracellular domain of VEGF and an Fc domain of IgG are fused.

In addition, the present invention provides a nucleic acid molecule, encoding the bispecific antibody that specifically binds to c-Kit and VEGF described above.

In addition, the present invention provides a recombinant vector, including the nucleic acid molecule described above and a transformant, including the recombinant vector.

Additionally, the present invention provides a method for producing a bispecific antibody that specifically binds to c-Kit and VEGF, including steps (a) and (b) below: (a) culturing the transformant described above; and (b) recovering a bispecific antibody that specifically binds to c-Kit and VEGF from a culture.

Furthermore, the present invention provides a pharmaceutical composition for preventing or treating an angiogenic disease, including the bispecific antibody that specifically binds to c-Kit and VEGF described above as an active ingredient.

In addition, the present invention provides a method for preventing or treating an angiogenic disease, including administering a therapeutically effective amount of the bispecific antibody that specifically binds to c-Kit and VEGF described above to a subject in need thereof.

In addition, the present invention provides the bispecific antibody that specifically binds to c-Kit and VEGF described above for use in preventing or treating an angiogenic disease.

In addition, the present invention provides the use of the bispecific antibody that specifically binds to c-Kit and VEGF described above in the manufacture of a medicament for preventing or treating an angiogenic disease

In the present invention, the angiogenic disease may be selected from the group consisting of cancer, leukemia, ocular vascular disease, rheumatoid arthritis, psoriasis, chronic wound, chronic inflammation, hemangioma, angiofibroma, vascular malformation, arteriosclerosis, vascular adhesion, vasculitis, pyogenic granuloma, blistering disease, pulmonary hypertension, asthma, nasal polyp, infectious disease, inflammatory bowel disease, periodontal disease, peritoneal adhesion, endometrium, uterine hemorrhage, ovarian cyst, osteomyelitis, osteopathy, sepsis and autoimmune disease.

In the present invention, the cancer may be selected from the group consisting of bone cancer, lung cancer, head cancer, neck cancer, thyroid cancer, parathyroid cancer, non-small cell lung cancer, stomach cancer, liver cancer, pancreatic cancer, skin cancer, cutaneous or intraocular melanoma, rectal cancer, proanal cancer, colon cancer, uterine cancer, breast cancer, ovarian cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, adrenal cancer, soft tissue sarcoma, urethral cancer, penile cancer, prostate cancer, bladder cancer, kidney cancer, ureteral cancer, renal cell carcinoma, renopelvic carcinoma, central nervous system tumor, central nervous system lymphoma, spinal cord tumor, glioblastoma, brainstem glioma and pituitary adenoma.

In the present invention, the ocular vascular disease may be selected from the group consisting of diabetic retinopathy, macular degeneration, age-related macular degeneration, glaucoma, glaucomatous retinitis pigmentosa, choroidal neovascularization, retinopathy of immaturity, corneal dystrophy and retinal separation.

Additionally, the present invention provides a kit for diagnosing an angiogenic disease, including the bispecific antibody that specifically binds to c-Kit and VEGF.

### ADVANTAGEOUS EFFECTS

The bispecific antibody according to the present invention that specifically binds to c-Kit and VEGF binds to c-Kit and/or VEGF with high affinity and has an excellent effects of inhibiting hypoxia- or inflammation-induced venous endothelial barrier destruction, SCF-dependent c-Kit signaling and the secretion of various cytokines (IL-6, VEGF, Ang-2, IL-8, and MCP-1). Accordingly, the bispecific antibody according to the present invention can be utilized for the treatment of various angiogenic diseases by significantly inhibiting the creation of abnormal or excessive neovascularization.

### DESCRIPTION OF DRAWINGS

FIG. 1 is a mimetic diagram showing the structure of a bispecific candidate antibody A that specifically binds to c-Kit and VEGF according to the present invention.
FIG. 2 shows the results of evaluating changes in antibody-dependent cell-mediated cytotoxicity according to the type of Fc variant.
FIGS. 3A to 3C are mimetic diagrams showing the structures of bispecific Candidate Antibody B (FIG. 3A), Candidate Antibody C (FIG. 3B) and Candidate Antibody B-1 (FIG. 3C) that specifically bind to c-Kit and VEGF according to the present invention.
FIGS. 4A and 4B are mimetic diagrams showing the structures of bispecific Candidate Antibody D (FIG. 4A) and Candidate Antibody E (FIG. 4B) that specifically bind to c-Kit and VEGF according to the present invention.
FIGS. 5A and 5B show the results of ELISA for determining the binding ability of a bispecific candidate antibody that specifically binds to c-Kit and VEGF according to the present invention to c-Kit and VEGF.
FIGS. 6A and 6B show the results of determining the binding affinity of a bispecific candidate antibody that specifically binds to c-Kit and VEGF according to the present invention by surface plasmon resonance to c-Kit and VEGF.
FIG. 7 shows the results of determining the thermal stability of a bispecific candidate antibody that specifically binds to c-Kit and VEGF according to the present invention using DSF.

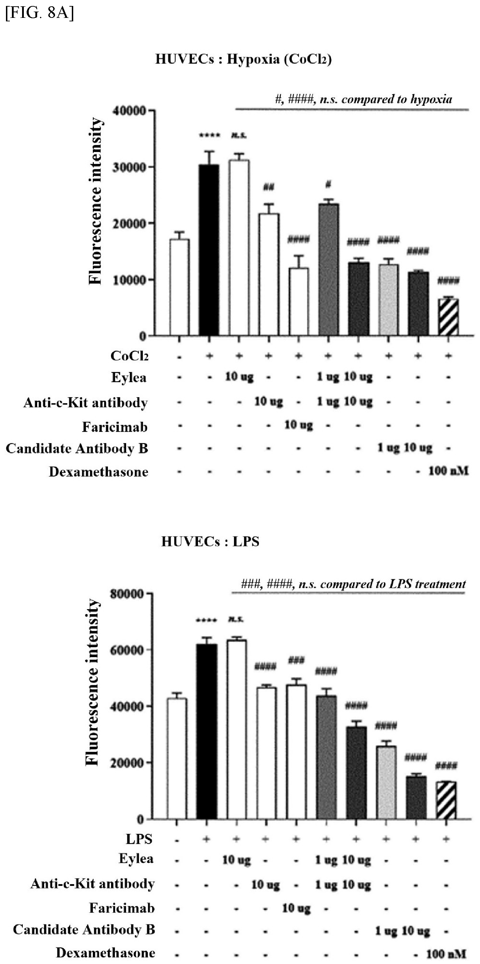

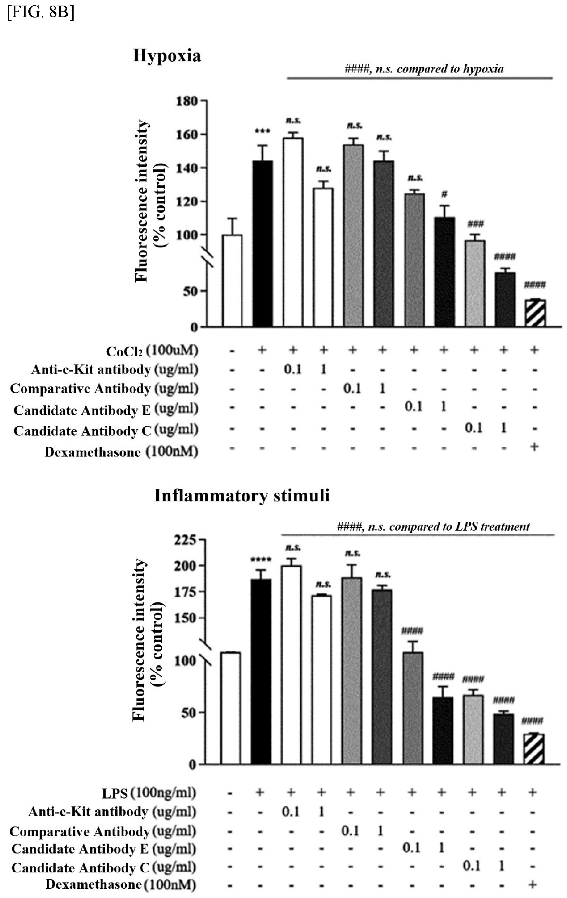

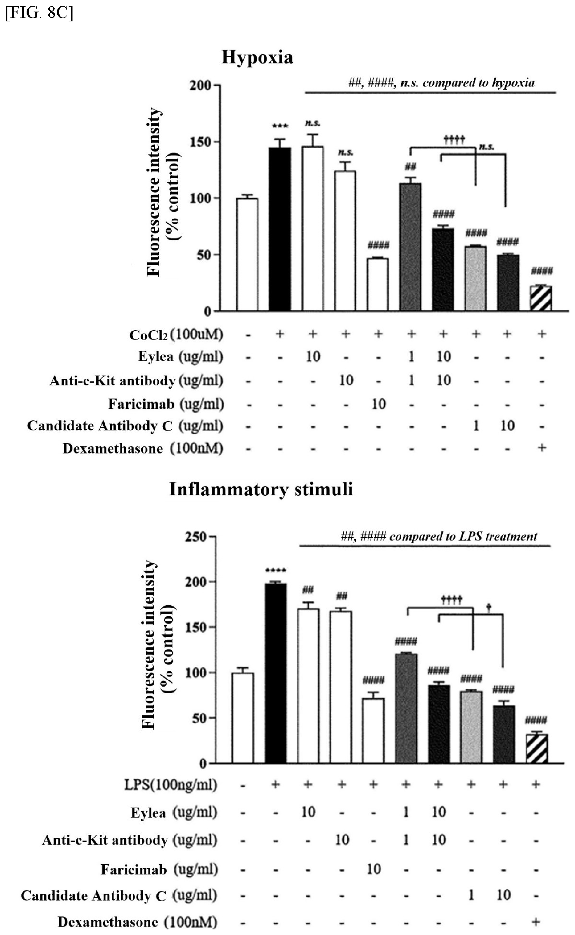

FIGS. 8A to 8C show the inhibitory effect of vascular permeability according to treatment with a bispecific candidate antibody specifically binding to c-Kit and VEGF according to the present invention in HUVECs induced under hypoxic conditions or with inflammation (FIGS. 8A and 8B: *** *P* <0.001, **** *P* <0.0001 *vs.* vehicle; # *P* <0.05, ### *P* <0.001 and #### *P* <0.0001 *vs.* 1% O₂ or LPS; *ns* ., no significance (*n* =3), FIG. 8C: *** *P* <0.001, **** *P* <0.0001 *vs.* vehicle; ## *P* <0.005, #### *P* <0.0001 *vs.* 1% O₂ or LPS; † *P* <0.05, †††† *P* <0.0001 *vs.* 1% O₂ or each concentration in LPS; *ns*., not significant).
FIGS. 9A to 9D show the effect of inhibiting SCF-dependent c-Kit signaling in HRMEC or LAD2 induced under hypoxic conditions or inflammation, according to treatment with a bispecific candidate antibody that specifically binds to c-Kit and VEGF according to the present invention.
FIGS. 10A and 10B show the cytokine (IL-8, MCP-1, IL-6) inhibitory effect according to treatment with bispecific Candidate Antibody A that specifically binds to c-Kit and VEGF according to the present invention in HRMEC or ARPE-19 cells induced with hypoxia or inflammation (** *P* <0.01, *** *P* <0.001*,* and **** *P* <0.0001 vs. vehicle; # *P* <0.01, ## *P* <0.005, and ### *P* <0.001 vs. 1% O₂ or LPS).
FIGS. 11A to 11R show the inhibitory effects of cytokines (VEGF, IL-6, IL-8, MCP-1 and Ang-2) according to the treatment of Candidate Antibody B in hypoxic or inflammation-induced HRMEC or ARPE-19 cells (** *P* <0.01, *** *P* <0.001, and **** *P* <0.0001 *vs.* vehicle; *# P* <0.01, ##*P*<0.005, ### P <0.001, and #### *P<0.0001 vs.* 1% O₂ or LPS treatment; *ns.,* not significant).
FIGS. 12A to 12Ishow the inhibitory effect of the secretion of cytokines (IL-6, VEGF, Ang-2, IL-8, and MCP-1) according to the treatment of Candidate Antibody C or Candidate Antibody E in hypoxic or inflammation-induced HRMEC or ARPE-19 cells (*** *P* <0.001, **** *P* <0.0001 *vs.* vehicle; # *P* <0.05, ## *P* <0.005, ### *P* <0.001, and #### *P* <0.0001 *vs.* 1% O₂ or LPS; *ns.,* not significant).
FIGS. 13A to 13Ishow the comparison of the inhibitory effects of cytokines (IL-6, VEGF, Ang-2, IL-8, and MCP-1) according to the treatment of Candidate Antibody C alone or in combination with Eylea and anti-c-Kit antibody in hypoxic or inflammation-induced HRMEC or ARPE-19 cells (*** *P* <0.001, **** *P* <0.0001 *vs.* vehicle; # *P* <0.05, ### *P* <0.001, #### *P* <0.0001 *vs.* 1% O₂ or LPS; ††† *P* <0.001, †††† *P* <0.0001 *vs.* each concentration in 1% O₂ or LPS; *ns.,* not significant).
FIGS. 14A and 14B show the comparison of the inhibitory effects of vascular permeability according to the treatment of Candidate Antibody C alone or in combination with Eylea and anti-c-Kit antibody on in mice with induced choroidal neovascularization.
FIG. 14C shows the comparison of the effects of reducing choroidal neovascularization lesions according to the treatment of Candidate Antibody C alone or in combination with Eylea and anti-c-Kit antibody in mice with induced choroidal neovascularization.
FIG. 15A shows the comparison of the inhibitory effects of vascular permeability according to the treatment of Candidate Antibody A alone at different concentrations (0.5 1, and 2 µg/µL/eye, respectively) and treatment of Eylea (aflibercept) alone at different concentrations (2 and 20 µg/µL/eye, respectively) in mice with induced choroidal neovascularization (** P<0.05, ** P<0.01*, and **** *P<0.0001* vs. G2).
FIG. 15B shows the comparison of the effects of reducing choroidal neovascularization lesions according to the treatment of Candidate Antibody A alone at different concentrations (0.5, 1 and 2 µg/µL/eye, respectively) and Eylea (aflibercept) alone at different concentrations (2 and 20 µg/µL/eye, respectively) on mice with induced choroidal neovascularization (****P<0.001* and *****P<0.0001* vs. G2).

### BEST MODE

Hereinafter, the present invention will be described in more detail.

All technical terms used in the present invention, unless otherwise defined, are used with the same meanings as commonly understood by a person skilled in the art in the relevant field of the present invention. In addition, although preferred methods or samples are described in the present specification, similar or equivalent ones are also included in the scope of the present invention.

The term "antibody" in the present invention is used in the broadest sense and includes various antibody structures, including but not limited to monoclonal antibodies, polyclonal antibodies, monospecific and multispecific antibodies (e.g. bispecific antibodies), and antibody fragments (so long as they exhibit the desired antigen binding activity).

The term "monoclonal antibody" in the present invention refers to an antibody obtained from a substantially homogeneous population of antibodies, that is, the individual antibodies including the population are identical and/or bind to the same epitope, with the possible exception of mutant antibodies, including those containing naturally occurring mutations or those arising during the production of the monoclonal antibody preparation, and these mutations are generally present in minor amounts. Unlike polyclonal antibody preparations, which typically include different antibodies directed against different determinants (epitopes), each monoclonal antibody of a monoclonal antibody preparation is directed against a single determinant on an antigen.

The term "monospecific" antibody, as used herein, refers to an antibody having more than one binding site, each of which binds to the same epitope on the same antigen. The term "bispecific" means that the antibody is capable of specifically binding to at least two distinct antigenic determinants, for example, two binding sites formed by a pair of antibody heavy chain variable domains (VH) and antibody light chain variable domains (VL), each of which binds to different antigens or different epitopes on the same antigen. Such bispecific antibodies are in a 2+2 format (including two binding sites for a first and a second antigen or epitope). Typically, a bispecific antibody includes two antigen binding sites, each of which is specific for a different antigenic determinant.

The term "valency" in the present invention indicates the presence of a specified number of binding sites in an antigen binding molecule. Likewise, the terms "bivalent", "tetravalent" and "hexavalent" indicate the presence of two binding sites, four binding sites and six binding sites, respectively, in an antigen binding molecule. A bispecific antibody according to the present invention may be "tetravalent."

The terms "full length antibody", "intact antibody" and "whole antibody" are used interchangeably herein to refer to antibodies having a structure substantially similar to a native antibody structure. "Native antibodies" refer to naturally occurring immunoglobulin molecules having a variety of structures. For example, native IgG-class antibodies are heterotetrameric glycoproteins of about 150,000 daltons, consisting of two light chains and two heavy chains disulfide-bonded. From N-terminus to C-terminus, each heavy chain has a variable region (VH), called a variable heavy domain or heavy chain variable domain, followed by three constant domains (CH1, CH2 and CH3), called heavy chain constant regions. Similarly, from N-terminus to C-terminus, each light chain has a variable region (VL), called the variable light domain or light chain variable domain, followed by a light chain constant domain (CL), called the light chain constant region. The heavy chains of antibodies may be assigned to one of five types, called α (IgA), δ (IgD), ε (IgE), γ (IgG), or µ (IgM), some of which can be further classified into subtypes, such as γ1 (IgG1), γ2 (IgG2), γ3 (IgG3), γ4 (IgG4), α1 (IgA1) and α2 (IgA2). The light chains of antibodies may be assigned to one of two types, called kappa (κ) and lambda (λ), based on the amino acid sequence of their constant domains.

As indicated above, the variable region allows the antibody to selectively recognize and specifically bind to an epitope on an antigen. That is, a combination of the antibody's VL domain and VH domain, or a subset of the complementarity determining region (CDR), forms a variable region that defines a three-dimensional antigen-binding site. This quaternary antibody structure forms an antigen binding site that is present at the end of each arm of the Y. More specifically, the antigen binding site is defined by three CDRs on each of the VH and VL chains (i.e., CDR-H1, CDR-H2, CDR-H3, CDR-L1, CDR-L2, and CDR-L3). In some cases, for example, when a predetermined immunoglobulin molecule is derived from a camelid species, or is engineered based on a camelid immunoglobulin, the complete immunoglobulin molecule may be composed solely of the heavy chain, without the light chain. See, for example, Hamers-Casterman et al., Nature 363: 446-448 (1993).

The terms "CDR-H", "HCDR" and "CDRH" are used interchangeably herein to refer to the VH chain of a CDR (e.g., CDR-H1, HCDR1 and CDRH1 refer to the VH1 of a CDR). The terms "CDR-L", "LCDR" and "CDRL" are used interchangeably herein to refer to the VL chain of a CDR (e.g., CDR-L1, LCDR1 and CDRL1 refer to the VL1 of a CDR).

In naturally occurring antibodies, the 6 "complementarity determining regions" or "CDRs" present in each antigen binding domain are short non-contiguous amino acid sequences that are specifically positioned to form the antigen binding domain as the antibody assumes a three-dimensional conformation in an aqueous environment. The remaining amino acids in the antigen binding domain, referred to as the "framework" region, exhibit less intermolecular variability. The framework region primarily adopts a β-sheet conformation, with the CDRs forming loops that connect and, in some cases, it forms part of the β-sheet structure. Therefore, the framework region functions to form a scaffold that positions the CDRs in the correct orientation by interchain, non-covalent interactions. The antigen binding domain formed by the positioned CDRs defines a surface that is complementary to the epitopes on the immunoreactive antigen. This complementary surface facilitates non-covalent binding of the antibody to its cognate epitope. The amino acids including the CDR and framework regions, respectively, for any given heavy or light chain variable region are readily identifiable by a person skilled in the art, as these have been precisely defined (see www.bioinf.org.uk: Dr. Andrew C.R. Martin's Group; "Sequences of Proteins of Immunological Interest," Kabat, E., et al., U.S. Department of Health and Human Services, (1983); and Chothia and Lesk, J. MoI. Biol., 196: 901-917 (1987)).

Where there are two or more definitions for a term used and/or accepted in the corresponding technical field, the definition of the term as used herein is intended to encompass all such meanings unless the meaning expressly states otherwise. As a specific example, the term "complementarity determining region" ("CDR") is used to describe the non-contiguous antigen binding sites found within the variable regions of both heavy and light chain polypeptides. Such specific regions are described in Kabat et al., U.S. Dept. of Health and Human Services, "Sequences of Proteins of Immunological Interest" (1983) and Chothia et al., J. MoI. Biol. 196: 901-917 (1987), the entire contents of which are incorporated herein by reference. The CDR definitions according to Kabat and Chothia include overlapping or subsets of amino acid residues when compared to one another. Nonetheless, the application of the definitions to refer to CDRs of antibodies or variants thereof is intended to be within the scope of the terms as defined and used herein. Appropriate amino acid residues including the CDRs defined by each of the references cited above are provided in Table 1 below for comparison. The exact number of residues including a particular CDR will vary depending on the sequence and size of the CDR. A person skilled in the art may routinely determine whether a residue includes a particular CDR by considering the variable region amino acid sequence of the antibody.

**[Table 1]**

| | Kabat | Chothia |
|---|---|---|
| CDR-H1 | 31-35 | 26-32 |
| CDR-H1 | 50-65 | 52-58 |
| CDR-H1 | 95-102 | 95-102 |
| CDR-L1 | 24-34 | 26-32 |
| CDR-L2 | 50-56 | 50-52 |
| CDR-L3 | 89-97 | 91-96 |

Kabat et al. also defined a numbering system for variable domain sequences that can be applied to any antibody. A person skilled in the art may unambiguously assign the "Kabat numbering" system to any variable domain sequence without relying on any experimental data beyond the sequence itself. As used herein, "Kabat numbering" refers to the numbering system set forth in Kabat et al., U.S. Dept. of Health and Human Services, "Sequence of Proteins of Immunological Interest" (1983). The antibodies disclosed herein may be derived from any animal source, including birds and mammals. Preferably, the antibodies are human, murine, donkey, rabbit, goat, guinea pig, camel, llama, horse or chicken.

As used herein, the term "heavy chain constant region" includes an amino acid sequence derived from an immunoglobulin heavy chain. As indicated above, a person skilled in the art will appreciate that the heavy chain constant region may be modified to vary the amino acid sequence from a naturally occurring immunoglobulin molecule.

The heavy chain constant region of the antibodies disclosed herein may be derived from different immunoglobulin molecules. The heavy chain constant region of the antibodies according to the present invention may be designated as one of the five types of immunoglobulins, called α (IgA), δ (IgD), ε (IgE), γ (IgG), or µ (IgM), as described above. In addition, depending on the amino acid composition of the hinge region and the number and position of heavy chain disulfide bonds, immunoglobulins of the same type may be classified into different subclasses (isotypes). For example, if the heavy chain constant region of the antibody of the present invention is γ (IgG), the subclass (isotype) may be further classified into γ1 (IgG1), γ2 (IgG2), γ3 (IgG3) or γ4 (IgG4), and if the heavy chain constant region of the antibody of the present invention is α (IgA), the subclass (isotype) may be further classified into α1 (IgA1) or α2 (IgA2).

As used herein, the term "light chain constant region" includes an amino acid sequence derived from an antibody light chain. Preferably, the light chain constant region includes at least one of a constant kappa domain or a constant lambda domain.

A "light chain-heavy chain pair" refers to a collection of light chains and heavy chains that can form a dimer through a disulfide bond between the CL domain of the light chain and the CH1 domain of the heavy chain.

"Antibody fragment" or "antigen binding fragment" refers to a molecule other than an intact antibody, which includes a portion of an intact antibody that binds to an antigen to which the intact antibody binds. Immunologically functional immunoglobulin fragments include, but are not limited to, Fab, Fab', F(ab')₂, xFab, scFab, dsFv, Fv, scFv-Fc, scFab-Fc, diabodies, minibodies, scAbs, dAbs, half-IgG or combinations thereof. The term "Fab" as used in Fab, Fab', F(ab')₂, xFab and scFab may include conventional Fab fragments and chimeric Fab-like domains as described in PCT/CN2018/106766 (Wuxibody). In addition, it may be derived from any mammal, including but not limited to human, mouse, rat, camelid or rabbit. A functional portion of an antibody, such as one or more of the CDRs described herein, may be covalently linked to a second protein or small molecule compound, thereby enabling it to be used as a targeted therapeutic for a specific target. The term "antibody fragment" includes aptamers, spiegelmers and diabodies. The term "antibody fragment" also includes any synthetic or genetically engineered protein that acts like an antibody by binding to a specific antigen and forming a complex.

Antibody fragments may be prepared by a variety of techniques, including but not limited to, proteolytic digestion of intact antibodies, as described herein, as well as production in recombinant host cells, such as *E. coli* or phage.

Papain digestion of an intact antibody produces two identical antigen binding fragments, called "Fab" fragments, which contain the heavy and light chain variable domains and the constant domain of the light chain and the first constant domain (CH1) of the heavy chain. Therefore, as used herein, the term "Fab fragment" refers to a light chain fragment including the VL domain and the constant domain (CL) of the light chain, and an antibody fragment including the VH domain and the first constant domain (CH1) of the heavy chain. A Fab' fragment differs from a Fab fragment in that it adds a few residues to the carboxy terminus of the heavy chain CH1 domain, including one or more cysteines from the antibody hinge region. Fab'-SH is a Fab' fragment in which the cysteine residue(s) of the constant domain have a free thiol group. Pepsin treatment produces an F(ab')₂ fragment having two antigen binding sites (two Fab fragments) and part of the Fc region. As used herein, the "F(ab')₂ fragment" includes two light chains, and two heavy chains including a variable region, CH1 and a portion of a constant region between the CH1 and CH2 domains, as described above, thereby forming an intrachain disulfide bond between the two heavy chains. Accordingly, the F(ab')₂ fragment is composed of two Fab' fragments, and the two Fab' fragments are linked to each other by a disulfide bond therebetween.

The term "cross-Fab fragment" or "xFab fragment" or "crossover Fab fragment" refers to a Fab fragment in which the variable regions or the constant regions of the heavy and light chains are exchanged. Two different chain compositions of crossed Fab molecules are possible and are included in the bispecific antibodies of the invention: on one hand, the variable regions of the Fab heavy and light chains are exchanged, that is, the crossed Fab molecule includes a peptide chain composed of a light chain variable region (VL) and a heavy chain constant region (CH1), and a peptide chain composed of a heavy chain variable region (VH) and a light chain constant region (CL). Such a crossed Fab molecule is also called CrossFab (VLVH). On the other hand, when the constant regions of the Fab heavy and light chains are exchanged, the crossover Fab molecule includes a peptide chain composed of the heavy chain variable region (VH) and the light chain constant region (CL), and a peptide chain composed of the light chain variable region (VL) and the heavy chain constant region (CH1). Such a crossover Fab molecule is also called CrossFab (CLCH1).

A "single chain Fab fragment" or "scFab" is a polypeptide composed of an antibody heavy chain variable domain (VH), an antibody constant domain 1 (CH1), an antibody light chain variable domain (VL), an antibody light chain constant domain (CL) and a linker, wherein the antibody domains and the linker have one of the following sequences from N-terminus to C-terminus: a) VH-CH1-linker-VL-CL, b) VL-CL-linker-VH-CH1, c) VH-CL-linker-VL-CH1 or d) VL-CH1-linker-VH-CL; and wherein the linker is a polypeptide having at least 30 amino acids, and preferably 32 to 50 amino acids. The single chain Fab fragment is stabilized by a natural disulfide bond between the CL domain and the CH1 domain. In addition, these single-chain Fab molecules may be further stabilized by the creation of interchain disulfide bonds through the insertion of cysteine residues (e.g., at position 44 in the variable heavy chain and position 100 in the variable light chain according to Kabat numbering).

A "crossover single chain Fab fragment" or "x-scFab" is a polypeptide composed of an antibody heavy chain variable domain (VH), an antibody constant domain 1 (CH1), an antibody light chain variable domain (VL), an antibody light chain constant domain (CL) and a linker, wherein the antibody domains and the linker have one of the following sequences from N-terminus to C-terminus: (a) VH-CL-linker-VLCH1 and (b) VL-CH1-linker-VH-CL; wherein the VH and VL together form an antigen binding domain that specifically binds to an antigen, and wherein the linker is a polypeptide having at least 30 amino acids. In addition, these x-scFab molecules may be further stabilized by the formation of interchain disulfide bonds through the insertion of cysteine residues (e.g., at position 44 in the variable heavy chain and position 100 in the variable light chain according to the Kabat numbering).

An "Fv region" is an antibody that includes the variable regions of each of the heavy and light chains, but no constant regions. An scFv is an Fv linked by a flexible linker. An scFv-Fc is an Fc linked to an scFv. A diabody includes two molecules of scFv. A "single-chain variable fragment" or "scFv" refers to a fusion protein of the variable regions of the heavy (VH) and light (VL) chains of an immunoglobulin. In some embodiments, the regions are linked to a short linker peptide having about 10 to about 25 amino acids. The linker may be glycine-rich for flexibility, and serine or threonine-rich for solubility, and may link the N-terminus of the VH to the C-terminus of the VL, or vice versa. These proteins retain the specificity of the native immunoglobulin, despite the deletion of the constant region and the introduction of a linker. ScFv molecules are known in the corresponding technical field and are described, for example, in U.S. Patent No. 5,892,019.

A "short-chain antibody (scAb)" is a single polypeptide chain including one variable region of a heavy chain or a constant region of a light chain, wherein the heavy and light chain variable regions are linked by a flexible linker. Single-chain antibodies are described, for example, in U.S. Patent No. 5,260,203, which is incorporated herein by reference.

A "domain antibody (dAb)" is an immunologically functional immunoglobulin fragment including only the variable region of a heavy chain or only the variable region of a light chain. In one embodiment, two or more VH regions are covalently linked by a peptide linker to form a bivalent domain antibody. The two VH regions of such a bivalent domain antibody may target the same or different antigens.

The term "full length IgG" according to the present invention is defined as including essentially complete IgG, but it does not necessarily have all the functions of a complete IgG. For the avoidance of doubt, full length IgG contains two heavy chains and two light chains. Each chain contains a constant (C) and a variable (V) region, which can be divided into domains designated CH1, CH2, CH3, VH, CL, and VL. An IgG antibody binds to an antigen through the variable region domain contained in the Fab portion, and after binding, it may interact with cells and molecules of the immune system through the constant domain, mostly through the Fc portion. The terms 'variable region domain', 'variable region', 'variable domain', 'VH/VL pair', 'VH/VL', 'Fab portion', 'Fab arm', 'Fab' or 'arm' are used interchangeably herein. A full-length antibody according to the present invention includes an IgG molecule in which mutations which impart the desired characteristics may be present. Such mutations must not result in the deletion of a significant portion of any region. However, an IgG molecule in which one or more amino acid residues are deleted without substantially altering the binding properties of the resulting IgG molecule is encompassed by the term "full-length IgG". For example, such an IgG molecule may have one or more deletions of 1 to 10 amino acid residues, preferably in a non-CDR region, wherein the deletion of the amino acid is not essential for the binding specificity of the IgG.

As used herein, the term "antigen binding domain" or "antigen binding site" refers to a portion of an antibody or antibody fragment that specifically binds to an antigenic determinant. More specifically, the term "antigen binding domain" refers to a portion of an antibody that specifically binds to and is complementary to part or all of an antigen. In the case of a large antigen, the antibody or antibody fragment may bind only to a specific portion of the antigen, and this portion is referred to as an epitope. The antigen binding domain may be provided, for example, by one or more variable domains (also referred to as variable regions). Preferably, the antigen binding domain includes an antibody light chain variable region (VL) and an antibody heavy chain variable region (VH). In one embodiment, the antigen binding domain is capable of binding to its antigen and blocking or partially blocking its function. Antigen binding domains that specifically bind c-Kit and VEGF include antibodies and fragments thereof as further defined herein. In addition, the antigen binding domain may include a scaffold antigen binding protein, for example, a binding domain based on a designed repeat protein or a designed repeat domain (see, e.g., WO 2002/020565).

As used herein, the term "antigenic determinant" is synonymous with "antigen" and "epitope" and refers to a site on a polypeptide macromolecule (e.g., a contiguous stretch of amino acids or a conformational configuration included of different regions of non-contiguous amino acids) to which an antigen binding moiety binds to form an antigen binding moiety-antigen complex. Useful antigenic determinants may be found, for example, on the surface of tumor cells, the surface of virus-infected cells, the surface of other diseased cells, the surface of immune cells, in serum-free blood, and/or in the extracellular matrix (ECM). A protein useful as an antigen herein may be any native form of the protein from any vertebrate source, including mammals such as primates (e.g., humans) and rodents (e.g., mice and rats), unless otherwise specified. In one particular embodiment, the antigen is a human or mouse protein. When referring to a particular protein herein, such terms include the "full-length," unprocessed protein, as well as all forms of the protein that are processed and produced in the cell. Such terms also include naturally occurring variants of the protein, such as splice variants or allelic variants.

"Specific binding" means that binding is selective for the antigen and is distinguishable from unwanted or non-specific interactions. The ability of an antibody or antibody fragment to bind a specific antigen may be measured by enzyme-linked immunosorbent assay (ELISA) or other techniques famEylear to a person skilled in the art, such as surface plasmon resonance (SPR) technology (analyzed on a BIAcore instrument) (Liljeblad et al., Glyco J 17, 323-329 (2000)), and traditional binding assays (Heeley, Endocr Res 28, 217-229 (2002)).

"Affinity" or "binding affinity" refers to the strength of the sum of non-covalent interactions between a single binding site of a molecule (e.g., an antibody) and its binding partner (e.g., an antigen). As used herein, unless otherwise specified, "binding affinity" refers to the intrinsic binding affinity reflecting a 1:1 interaction between members of a binding pair (e.g., an antibody and an antigen). The affinity of a molecule X for its partner Y may generally be expressed in terms of the dissociation constant (Kd), which is the ratio of the dissociation rate constant and the association rate constant (koff and kon, respectively). Therefore, equivalent affinities may include different rate constants, as long as the ratio of the rate constants remains the same. Affinity may be measured by methods known in the art, including those described herein. A particular method for measuring affinity is surface plasmon resonance (SPR). According to one embodiment of the present invention, the bispecific antibody of the present invention has a dissociation constant (KD) of ≤1 µM, ≤100 nM, ≤10 nM, ≤1 nM, ≤0.1 nM, ≤0.01 nM or ≤0.001 nM (e.g., less than or equal to 10⁻⁷ M, e.g., from 10⁻⁷ M to 10⁻¹³ M, e.g., from 10⁻⁹ M to 10⁻¹³ M).

The term "high affinity" of an antibody refers to an antibody having a KD of 10⁻⁹ M or less, and more preferably 10⁻¹⁰ M or less, for its target antigen.

The terms "bispecific antibody including a first antigen binding site that specifically binds c-Kit and a second antigen binding site that specifically binds VEGF", "bispecific antibody that specifically binds c-Kit and VEGF", and "bispecific antigen binding molecule specific for c-Kit and VEGF" are used interchangeably herein and refer to a bispecific antibody capable of binding c-Kit and VEGF with sufficient affinity to render the antibody useful as a diagnostic and/or therapeutic agent in targeting c-Kit and VEGF.

The term "c-Kit" used in the present invention belongs to class III of receptor tyrosine kinase (RTK), and is also known as a receptor for SCF.

c-Kit, which is one of the targets of the angiogenesis inhibitors, belongs to class III of receptor tyrosine kinase (RTK), and is a receptor for SCF (Stem Cell Factor), which plays an important role in haematopoiesis.

The term "anti-c-Kit antibody" used in the present invention means an antibody that specifically binds to c-Kit. Specifically, the anti-c-Kit antibody specifically binds to domain II of c-Kit, and may thereby inhibit or neutralize the activity or activation of c-Kit.

"Anti-c-Kit antibody" and "antibody including an antigen binding site that binds c-Kit" refer to an antibody capable of binding c-Kit, particularly a c-Kit polypeptide, with sufficient affinity to render the antibody useful as a diagnostic and/or therapeutic agent in the targeting of c-Kit. In one embodiment, the extent of binding of the anti-c-Kit antibody to an unrelated, non-c-Kit protein is less than about 10% of the binding of the antibody to c-Kit, as measured, for example, by radioimmunoassay (RIA) or flow cytometry (FACS) or by surface plasmon resonance analysis using a biosensor system such as the Biacore^{®} system. By way of example, an antibody that binds to human c-Kit has a KD value for binding affinity for binding to human c-Kit of ≤1 µM, ≤100 nM, ≤10 nM, ≤1 nM, ≤0.1 nM, ≤0.01 nM, or ≤0.001 nM (e.g., less than or equal to 10⁻⁸ M, e.g., between 10⁻⁸ M and 10⁻¹³ M, e.g., between 10⁻⁹ M and 10⁻¹³ M).

As used herein, a "VEGF antagonist" means any molecule that blocks, reduces or interferes with the normal biological activity of vascular endothelial growth factor (VEGF) or a VEGF receptor. VEGF antagonists include molecules that interfere with the interaction between VEGF and a native VEGF receptor, for example, molecules that bind to VEGF or a VEGF receptor and prevent or otherwise interfere with the interaction between VEGF and the VEGF receptor. Specific examples of VEGF antagonists include anti-VEGF antibodies (e.g., bevacizumab [AVASTIN^{®}]), anti-VEGF receptor antibodies (e.g., anti-VEGFR1 antibodies, anti-VEGFR2 antibodies, etc.), and VEGF receptor-based chimeric molecules (also referred to herein as "VEGF-traps"). VEGF receptor-based chimeric molecules include a chimeric polypeptide including two or more immunoglobulin (Ig)-like domains of a VEGF receptor such as VEGFR1 (also represented as Flt1) and/or VEGFR2 (also represented as Flk1 or KDR) and may also contain a multimerization domain (e.g., an Fc domain that enables multimerization [e.g., dimerization] of two or more chimeric polypeptides). Exemplary VEGF receptor-based chimeric molecules are VEGFR1R2-FcΔC1(a) or Flt1D2.Flk1D3.FcΔC1 (also known as aflibercept (trade names: Eylea, Zaltrap)) comprising or consisting of an amino acid sequence of SEQ ID NO: 21.

"VEGF antagonist" and "antibody including an antigen binding site that binds VEGF" refer to an antibody capable of binding VEGF, particularly a VEGF polypeptide, with sufficient affinity to render the antagonist or antibody useful as a diagnostic and/or therapeutic agent in the targeting of VEGF. In one embodiment, the extent of binding of the VEGF antagonist or antibody to an unrelated, non-VEGF protein is less than about 10% of the binding of the antagonist or antibody to VEGF, as measured, for example, by radioimmunoassay (RIA) or flow cytometry (FACS) or by surface plasmon resonance analysis using a biosensor system such as the Biacore^{®} system. By way of example, an antibody that binds to human VEGF has a KD value for binding affinity for human VEGF of ≤1 µM, ≤100 nM, ≤10 nM, ≤1 nM, ≤0.1 nM, ≤0.01 nM, or ≤0.001 nM (e.g., less than or equal to 10⁻⁸ M, e.g., from 10⁻⁸ M to 10⁻¹³ M, e.g., from 10⁻⁹ M to 10⁻¹³ M).

The term "mouse" antibody is intended to include antibodies having variable regions in which both the framework and CDR regions are derived from mouse germline immunoglobulin sequences. Furthermore, if the antibody contains a constant region, the constant region is also derived from mouse germline immunoglobulin sequences. The mouse antibodies of the present disclosure may include amino acid residues that are not encoded by a mouse germline immunoglobulin sequence (e.g., mutations introduced by *in vitro* random or site-specific mutagenesis or by *in vivo* somatic mutations).

The term "chimeric" antibody refers to an antibody in which a portion of the heavy and/or light chain is derived from a particular source or species, and the remainder of the heavy and/or light chain is derived from a different source or species.

The "class" of an antibody refers to the type of constant domain or constant region that the heavy chain of the antibody possesses. There are five major classes of antibodies, IgA, IgD, IgE, IgG and IgM, and some of these may be further classified into subclasses (isotypes), such as IgG1, IgG2, IgG3, IgG4, IgA1, and IgA2. The heavy chain constant domains corresponding to various classes of immunoglobulins are called α, δ, ε, γ and µ, respectively.

A "humanized" antibody refers to a chimeric antibody including amino acid residues from non-human HVRs and amino acid residues from human FRs. In certain embodiments, a humanized antibody will include substantially all of at least one, and typically two, variable domains, wherein all or substantially all of the HVRs (e.g., CDRs) correspond to those of a non-human antibody, and all or substantially all of the FRs correspond to those of a human antibody.

A humanized antibody may optionally include at least a portion of an antibody constant region derived from a human antibody. A "humanized form" of an antibody, for example, a non-human antibody, refers to an antibody that has undergone humanization. Other forms of "humanized antibodies" included in the invention are those in which the constant region has been further modified or altered from the constant region of the original antibody to produce the properties according to the invention, particularly with respect to C1q binding and/or Fc receptor (FcR) binding.

A "human" antibody is an antibody that has an amino acid sequence that corresponds to the amino acid sequence of an antibody produced by a human or a human cell, or derived from a non-human source that utilizes a human antibody repertoire or other human antibody-coding sequences. This definition of a human antibody specifically excludes humanized antibodies that include non-human antigen binding residues.

The term "Fc domain" or "Fc region" is used herein to define a C-terminal region of an immunoglobulin heavy chain that contains at least a portion of the constant region. The term includes native sequence Fc regions and variant Fc regions.

The term "linker" refers to a peptide including one or more amino acids, typically about 2 to 20 amino acids. Any peptide linker known in the art may be used as the linker. The peptide linker separates the light chain variable domain and the heavy chain variable domain by a sufficient distance to allow each variable domain to fold into the appropriate secondary and tertiary structures. The sequence of a suitable peptide linker may be selected by considering the following factors: (a) the ability to have a flexible extended conformation; (b) the ability to not form a secondary structure that interacts with the epitope; and (c) the absence of hydrophobic residues or charged residues that can react with the epitope. Preferred peptide linkers include Gly, Glu, Asn, Lys, Ser and Pro residues. Other neutral amino acids, such as Thr and Ala, may also be included in the linker sequence. The linker sequence may consist of 1 to 50 amino acid residues, and preferably 10 to 20 amino acid residues. For example, a suitable non-immunogenic linker peptide may include, but is not limited to, 5 to 10 consecutive glycines (G).

"Percent (%) amino acid sequence identity" for a reference polypeptide sequence is defined as the percentage of amino acid residues in a candidate sequence that are equal to the percentage of amino acid residues in a reference polypeptide sequence, after aligning the sequences and, if necessary, introducing gaps to achieve the maximum percentage of sequence identity, without considering conservative substitutions as part of the sequence identity. Alignment for purposes of determining percent amino acid sequence identity may be accomplished in a variety of ways that are within the corresponding technical field, for example, using publicly available computer software such as BLAST, BLAST-2, Clustal W, Megalign (DNASTAR) software or the FASTA program package. A person skilled in the art may determine appropriate parameters for aligning the sequences, including any algorithm necessary to achieve maximum alignment over the entire length of the sequences being compared. However, for purposes herein, percent amino acid sequence identity values are generated by using the ggsearch program in the FASTA package, version 36.3.8c or later, in conjunction with a BLOSUM50 comparison matrix. The FASTA program package is described in W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227-258; and Pearson et. al. (1997) Genomics 46:24-36, and is publicly available at http:/fasta.bioch.virginia.edu/fasta_www2/fasta_down.shtml. Alternatively, one may compare sequences by using the public server accessible at http:/fasta.bioch.virginia.edu/fasta_www2/index.cgi, using the ggsearch(global protein:protein) program with default options (BLOSUM50; open: -10; ext: -2; Ktup=2) to perform a global rather than a local alignment. The percent amino acid identity is provided in the output alignment header.

As used herein, the term "polypeptide" is intended to encompass the singular "polypeptide" as well as the plural "polypeptides", and refers to a molecule including monomers (amino acids) that are linearly linked by amide bonds (also known as peptide bonds). The term "polypeptide" refers to any chain or chains of two or more amino acids, and does not refer to a particular length of the product. Therefore, peptide, dipeptide, tripeptide, oligopeptide, "protein", "amino acid chain", or any other term used to refer to a chain or chains of two or more amino acids are included within the definition of "polypeptide", and the term "polypeptide" may be used in place of, or interchangeably with, any of these terms. The term "polypeptide" is also intended to refer to the product of post-expression modification of a polypeptide, including but not limited to glycosylation, acetylation, phosphorylation, amidation, derivatization with known protecting/blocking groups, proteolytic cleavage, or modification with non-naturally occurring amino acids. The polypeptide may be derived from a natural biological source or produced by recombinant techniques, but is not necessarily translated from a designated nucleic acid sequence. It may be produced in any manner, including chemical synthesis. The term "polypeptide" also includes variants of a polypeptide and derivatives of a polypeptide. Furthermore, a "polypeptide fragment" means a polypeptide having a deletion of the amino-terminal amino acid sequence, a deletion of the carboxyl-terminal amino acid sequence and/or an internal deletion, as compared to the full-length protein. Such fragments may also include modified amino acids as compared to the full-length protein. In one embodiment, the fragment may be about 5 to 900 amino acids in length, for example, at least 5, 6, 8, 10, 14, 20, 50, 70, 100, 110, 150, 200, 250, 300, 350, 400, 450, 500, 550, 600, 650, 700, 750, 800, 850 or more amino acids in length. For the purposes of the present invention, useful polypeptide fragments include immunologically functional fragments of an antibody including an antigen binding domain. For antibodies that specifically bind to c-Kit and VEGF, such useful fragments include, but are not limited to, all or part of an antibody chain including one, two or three heavy or light chain CDR sequences, or a variable region or constant region of a heavy or light chain.

As used herein, a "variant" of a polypeptide, for example, an antigen binding fragment, protein or antibody, is a polypeptide having one or more amino acid residues inserted, deleted, added and/or substituted as compared to another polypeptide sequence, including a fusion polypeptide. In addition, protein variants include those that are modified by proteolytic cleavage, phosphorylation or other post-translational modification, but which retain the biological activity of an antibody disclosed herein, for example, specific binding to c-Kit and VEGF and biological activity. The variant may be about 99%, 98%, 97%, 96%, 95%, 94%, 93%, 92%, 91%, 90%, 89%, 88%, 87%, 86%, 85%, 84%, 83%, 82%, 81% or 80% identical to the sequence of an antibody or antigen binding fragment thereof disclosed herein.

The term "recombinant" as used herein with respect to a polypeptide or polynucleotide means a form of a polypeptide or polynucleotide that does not exist in nature, a non-limiting example of which may be formed by combining polynucleotides or polypeptides that do not normally exist together.

"Homology" or "identity" or "similarity" refers to sequence similarity between two peptides, or between two nucleic acid molecules. Homology may be determined by comparing positions in each sequence that can be aligned for comparison purposes. If a position in the compared sequences is occupied by the same base or amino acid, the molecules are homologous at the corresponding position. The degree of homology between sequences is a function of the number of positions shared by the sequences that are identical or homologous. An "unrelated" or "non-homologous" sequence shares less than 40% identity, and preferably less than 25% identity, with one of the sequences of the present disclosure.

A polynucleotide or polynucleotide region (or polypeptide or polypeptide region) having a predetermined percentage (e.g., 60%, 65%, 70%, 75%, 80%, 85%, 90%, 95%, 98% or 99%) of "sequence identity" to another sequence means that when two sequences are compared by aligning the same, the bases (or amino acids) are the same to the corresponding percentage.

The term "polynucleotide" refers to an isolated nucleic acid molecule or structure, such as messenger RNA (mRNA), virus-derived RNA or plasmid DNA (pDNA). A polynucleotide may include conventional phosphodiester bonds or nonconventional bonds (e.g., amide bonds, such as amide bonds found in peptide nucleic acids (PNAs)). The term "nucleic acid molecule" refers to any one or more nucleic acid segments, such as DNA or RNA fragments, present in a polynucleotide.

An "isolated" nucleic acid molecule or polynucleotide means a nucleic acid molecule, DNA or RNA, taken from its natural environment. For example, a recombinant polynucleotide encoding a polypeptide contained in a vector is considered to be isolated for the purposes of the present invention. Additional examples of isolated polynucleotides include recombinant polynucleotides maintained in a heterologous host cell or polynucleotides purified (partially or substantially) in solution. An isolated polynucleotide generally includes a polynucleotide molecule contained in a cell containing the polynucleotide molecule, but the polynucleotide molecule is present extrachromosomally or at a chromosomal location different from the natural chromosomal location. Isolated RNA molecules include *in vivo* or *in vitro* RNA transcripts of the present invention, as well as positive and negative strand forms, and double-stranded forms. Isolated polynucleotides or nucleic acids according to the present invention further include synthetically produced molecules. In addition, the polynucleotide or nucleic acid may be or include regulatory elements such as a promoter, a ribosome binding site, or a transcription terminator. The term "isolated" as used herein also refers to a nucleic acid or peptide in which cellular material, viral material or culture medium is substantially absent when produced by recombinant DNA technology, or in which chemical precursors or other chemicals are substantially absent when chemically synthesized. The term "isolated" is also used herein to refer to a cell or polypeptide being isolated from another cellular protein or tissue. Isolated polypeptides are meant to include both purified polypeptides and recombinant polypeptides.

The term "expression cassette" refers to a polynucleotide that is produced by recombination or synthetically, by using a series of specified nucleic acid elements that enable the transcription of a specific nucleic acid in a target cell. The recombinant expression cassette may be incorporated into a plasmid, a chromosome, mitochondrial DNA, plasmid DNA, a virus or a nucleic acid fragment. Typically, the recombinant expression cassette portion of an expression vector includes, *inter alia*, the nucleic acid sequence to be transcribed and a promoter. In certain embodiments, the expression cassette includes a polynucleotide sequence encoding a bispecific antibody of the present invention.

The term "vector" or "expression vector" refers to a DNA molecule used to introduce and express a specific gene that is operably linked to a cell. The term includes vectors as self-replicating nucleic acid structures as well as vectors introduced by incorporation into the genome of a host cell. The expression vectors of the present invention include an expression cassette. The expression vectors enable the transcription of large quantities of stable mRNA. When the expression vector is present inside a cell, the ribonucleic acid molecule or protein encoded by the gene is produced by the cellular transcription and/or translation machinery. In one embodiment, the expression vectors of the present invention include an expression cassette including a polynucleotide sequence encoding a bispecific antibody of the present invention.

The terms "host cell", "host cell line" and "host cell culture" are used interchangeably and refer to cells into which exogenous nucleic acid has been introduced (including the progeny of such cells). Host cells include "transformants" and "transformed cells", including the primary transformed cell and progeny derived therefrom, regardless of the number of passages. The progeny may not be completely identical in nucleic acid content to the parent cell, but may contain mutations. Mutant progeny that have the same function or biological activity as those sorted or selected for in the originally transformed cell are included herein. A host cell is any type of cell system that can be used to produce an antibody or bispecific antibody of the present invention. Host cells include cultured cells, for example mammalian cultured cells such as HEK cells, CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, including cells included within a transgenic animal, a transgenic plant or cultured plant or animal tissue.

A first aspect of the present invention relates to a bispecific antibody that specifically binds to c-Kit and VEGF.

The bispecific antibody that specifically binds to c-Kit and VEGF according to the present invention has the property of binding to c-Kit and VEGF with high affinity and may include (a) and (b) below:
(a) a first antigen binding site that specifically binds to c-Kit, including a heavy chain variable region including a heavy chain CDR1 including an amino acid sequence of SEQ ID NO: 2, a heavy chain CDR2 including an amino acid sequence of SEQ ID NO: 4, and a heavy chain CDR3 including an amino acid sequence of SEQ ID NO: 6; and a light chain variable region including a light chain CDR1 including an amino acid sequence of SEQ ID NO: 13, a light chain CDR2 including an amino acid sequence of SEQ ID NO: 15, and a light chain CDR3 including an amino acid sequence of SEQ ID NO: 17; and (b) a second antigen binding site that specifically binds to VEGF, wherein a soluble extracellular domain of VEGF and an Fc domain of IgG are fused.

The first antigen binding site of the bispecific antibody according to one embodiment of the present invention may include a light chain variable region including LCDR1, LCDR2 and LCDR3 consisting of each amino acid sequence described in (a) above and a heavy chain variable region including HCDR1, HCDR2 and HCDR3 consisting of each amino acid sequence described in (a) above.

In the present invention, the first antigen binding site may include a heavy chain variable region including an amino acid sequence having 90% or greater sequence homology to an amino acid sequence of SEQ ID NO: 27 and a light chain variable region including an amino acid sequence having 90% or greater sequence homology to an amino acid sequence of SEQ ID NO: 28. Specifically, the first antigen binding site according to one embodiment of the present invention may include a heavy chain variable region including an amino acid sequence having 90%, 91%, 92%, 93%, 94%, 95%, 96%, 97%, 98% or 99% or greater sequence homology to an amino acid sequence of SEQ ID NO: 27, and may include a light chain variable region including an amino acid sequence having 90%, 91%, 92%, 93%, 94%, 95%, 96%, 97%, 98% or 99% or greater sequence homology to an amino acid sequence of SEQ ID NO: 28.

The amino acid sequences of SEQ ID NOS: 27 and 28 above are as follows.

QVQLVESGGGVVQPGRSLRLSCAASGFTFSRYGMHWVRQAPGKGL
EWVAVIWYDGTNKDYTDSVRGRFTISRDNSKNTLYLQMNSLRAEDTAVYY
CAREDWAEAFDMWGQGTTVTVSS (SEQ ID NO: 27)

DIVMTQSPLSLPVTPGEPASISCRSSQSLLHSNGYNYLDWYLQKPGQS
PQLLIYLGSNRASGVPDRFSGSGSGTDFTLKISRVEAEDVGVYYCMQALQTIT
FGQGTRLEIK (SEQ ID NO: 28)

In the present invention, the soluble extracellular domain may include immunoglobulin-like domain 2 of a first VEGF receptor and immunoglobulin-like domain 3 of a second VEGF receptor. Preferably, the second antigen binding portion may be a VEGF antagonist known as aflibercept (trade name: Eylea, Zaltrap). According to a specific embodiment of the present invention, the VEGF antagonist may comprise or consist of an amino acid of SEQ ID NO: 21.

In the present invention, the second antigen binding site may be bound to a heavy chain C-terminus or N-terminus of the first antigen binding site by a peptide linker; or the second antigen binding site may be bound to a light chain C-terminus of the first antigen binding site by a peptide linker. According to a specific embodiment of the present invention, the antibody in which the second antigen binding site is bound to the heavy chain C-terminus of the first antigen binding site by a peptide linker is 'Candidate Antibody A', 'Candidate Antibody B', 'Candidate Antibody B-1' or 'Candidate Antibody C', the antibody in which the second antigen binding site is bound to the heavy chain N-terminus of the first antigen binding site by a peptide linker is 'Candidate Antibody E', and the antibody in which the second antigen binding site is bound to the light chain C-terminus of the first antigen binding site by a peptide linker is 'Candidate Antibody D'.

In the present invention, the peptide linker connecting the first antigen binding site and the second antigen binding site may have 5 to 10 glycines (Gly) connected in sequence, and is designated as SEQ ID NO: 20 (G5), SEQ ID NO: 67 (G6), SEQ ID NO: 68 (G7), SEQ ID NO: 23 (G8), SEQ ID NO: 69 (G9) or SEQ ID NO: 70 (G10), respectively.

The bispecific antibodies of the present invention may have their Fc domains modified to reduce Fc receptor binding and/or effector function.

According to one embodiment of the present invention, one or more amino acid modifications may be introduced into the Fc region of an antibody provided herein to produce an Fc region variant. The Fc region variant may include a human Fc region sequence (e.g., a human IgG1, IgG2, IgG3 or IgG4 Fc region) including an amino acid modification (e.g., a substitution) at one or more amino acid positions.

Specifically, the Fc domain of the bispecific antibody including a first antigen binding site that specifically binds c-Kit and a second antigen binding site that specifically binds VEGF may include one or more amino acid substitutions that reduce binding to an Fc receptor, and particularly an Fcγ receptor. For example, the Fc domain is of the human IgG1 subclass including an Fc variant including amino acid substitutions L234A and L235E; or an Fc variant including amino acid substitutions L234A, L235A I253A, H310A, P329G and H435A.

The Fc domain confers favorable pharmacodynamic properties to the bispecific antibody of the present invention, by including a long serum half-life, which contributes to good accumulation in target tissues and a favorable tissue-to-blood distribution ratio. However, at the same time, it may lead to undesirable targeting of the bispecific antibody of the present invention to cells expressing Fc receptors rather than to desired antigen-bearing cells. The Fc domain of the bispecific antibody according to one embodiment of the present invention exhibits a reduced binding affinity for Fc receptors and/or a reduced effector function compared to a native IgG Fc domain, particularly an IgG1 Fc domain. Preferably, the Fc domain may be a wild-type IgG1 Fc domain or a variant thereof.

In a specific embodiment of the present invention, the Fc domain exhibits less than 50%, preferably less than 20%, more preferably less than 10% and most preferably less than 5%, of the binding affinity for an Fc receptor relative to a native IgG1 Fc domain (or the bispecific antigen binding molecule of the present invention including a native IgG1 Fc domain), and/or less than 50%, preferably less than 20%, more preferably less than 10% and most preferably less than 5%, of the effector function relative to a native IgG1 Fc domain. In one embodiment, the Fc domain does not substantially bind to and/or induce effector function of an Fc receptor. In one embodiment, the Fc receptor is an Fcγ receptor. In another embodiment, the Fc receptor is a human Fc receptor. In still another embodiment, the Fc receptor is an activating Fc receptor. In another embodiment, the Fc receptor is an activating human Fcγ receptor, more specifically human FcγRIIIa, FcγRI or FcγRIIa, and preferably human FcγRIIIa. In one embodiment, the effector function is one or more of CDC, ADCC, ADCP and cytokine secretion. In another embodiment, the effector function is ADCC. In still another embodiment, the Fc domain exhibits substantially similar binding affinity to the neonatal Fc receptor (FcRn) as compared to a native IgG1 Fc domain.

In one embodiment of the present invention, the Fc domain may be engineered to have reduced binding affinity and/or reduced effector function for an Fc receptor, compared to a non-engineered Fc domain (wild-type Fc). In one embodiment, the Fc domain of the bispecific antibody of the present invention may include one or more amino acid mutations that reduce the binding affinity and/or effector function of the Fc domain for an Fc receptor. Typically, the same one or more amino acid mutations are present in each of the two subunits of the Fc domain. The amino acid mutations may reduce the binding affinity of the Fc domain for an Fc receptor. For example, the amino acid mutations may reduce the binding affinity of the Fc domain for an Fc receptor by at least 2-fold, at least 5-fold, or at least 10-fold, or may eliminate binding affinity such that the Fc domain does not bind to an Fc receptor at all. In a specific embodiment, the bispecific antibody of the present invention including an engineered Fc domain may exhibit a binding affinity for an Fc receptor of less than 20%, particularly less than 10%, and more particularly less than 5%, compared to a bispecific antibody of the present invention including a non-engineered Fc domain. In one embodiment, the Fc receptor is an Fcγ receptor. In another embodiment, the Fc receptor is a human Fc receptor. In still another embodiment, the Fc receptor is an activating human Fcγ receptor, more particularly human FcγRIIIa, FcγRI or FcγRIIa, and preferably human FcγRIIIa. In one embodiment, the binding affinity for complement components, particularly binding affinity for C1q, may also be reduced. In another embodiment, the binding affinity for neonatal Fc receptor (FcRn) may be reduced. Substantially similar binding to FcRn, that is, preservation of the binding affinity of the Fc domain for the receptor, is achieved when the Fc domain exhibits a binding affinity for FcRn that is greater than about 70% of the binding affinity of the non-engineered form of the Fc domain. The Fc domain, or a bispecific antibody of the invention including the Fc domain, may exhibit such affinities of greater than about 80% and even greater than about 90%. In some embodiments, the Fc domain of the bispecific antigen binding molecule of the present invention is engineered to have reduced effector function compared to the non-engineered Fc domain. The reduced effector function may include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling-induced apoptosis, reduced dendritic cell maturation, or reduced T cell priming.

Antibodies with reduced effector function may include those having substitutions in one or more of Fc region residues 234, 235, 237, 253, 310, 329 and 435. In a specific embodiment, the Fc domain may include amino acid substitutions L234A, L235A and P329G ("LALAPG"), L234A, L235A, P329G, I253A, H310A and H435A ("LALAPG-AAA"), L235A and G237A ("LAGA") or amino acid substitutions L234A and L235E ("LALE").

In the present invention, an exemplary bispecific antibody having a wild-type Fc domain may be, but is not limited to, Candidate Antibody A or Candidate Antibody D. The heavy chain of the first antigen binding portion of Candidate Antibody A or Candidate Antibody D having a wild-type Fc domain may comprise or consist of an amino acid sequence of SEQ ID NO: 29, and the light chain of the first antigen binding portion may comprise or consist of an amino acid sequence of SEQ ID NO: 30.

The amino acid sequences of SEQ ID NOS: 29 and 30 above are as follows.

QVQLVESGGGVVQPGRSLRLSCAASGFTFSRYGMHWVRQAPGKGL
EWVAVIWYDGTNKDYTDSVRGRFTISRDNSKNTLYLQMNSLRAEDTAVYY
CAREDWAEAFDMWGQGTTVTVSSASTKGPSVFPLAPSSKSTSGGTAALGCL
VKDYFPEPVTVSWNSGALTSGVHTFPAVLQSSGLYSLSSVVTVPSSSLGTQT
YICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYR

VVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK (SEQ ID NO:
29)

DIVMTQSPLSLPVTPGEPASISCRSSQSLLHSNGYNYLDWYLQKPGQS
PQLLIYLGSNRASGVPDRFSGSGSGTDFTLKISRVEAEDVGVYYCMQALQTIT
FGQGTRLEIKRTVAAPSVFIFPPSDEQLKSGTASVVCLLNNFYPREAKVQWK
VDNALQSGNSQESVTEQDSKDSTYSLSSTLTLSKADYEKHKVYACEVTHQ
GLSSPVTKSFNRGEC (SEQ ID NO: 30)

For reduced effector function, the Fc domain of the bispecific antibody according to the present invention may include amino acid substitutions as defined above. In the present invention, an exemplary bispecific antibody having LALAPG introduced into the Fc domain may be, but is not limited to, Candidate Antibody B or Candidate Antibody E. The heavy chain of Candidate Antibody B or Candidate Antibody E having LALAPG introduced into the Fc domain may comprise or consist of an amino acid sequence of SEQ ID NO: 31, and the light chain of the first antigen binding site may comprise or consist of an amino acid sequence of SEQ ID NO: 30.

The amino acid sequence of SEQ ID NO: 31 above is as follows.

QVQLVESGGGVVQPGRSLRLSCAASGFTFSRYGMHWVRQAPGKGL
EWVAVIWYDGTNKDYTDSVRGRFTISRDNSKNTLYLQMNSLRAEDTAVYY
CAREDWAEAFDMWGQGTTVTVSSASTKGPSVFPLAPSSKSTSGGTAALGCL
VKDYFPEPVTVSWNSGALTSGVHTFPAVLQSSGLYSLSSVVTVPSSSLGTQT
YICNVNHKPSNTKVDKKVEPKSCDKTHTCPPCPAPEAAGGPSVFLFPPKPKD
TLMISRTPEVTCVVVDVSHEDPEVKFNWYVDGVEVHNAKTKPREEQYNSTY
RVVSVLTVLHQDWLNGKEYKCKVSNKALGAPIEKTISKAKGQPREPQVYTL

PPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK (SEQ ID
NO: 31)

In the present invention, an exemplary bispecific antibody having LALE introduced into the Fc domain may be, but is not limited to, Candidate Antibody C. The heavy chain of candidate antibody C having LALE introduced into the Fc domain may comprise or consist of an amino acid sequence of SEQ ID NO: 32, and the light chain of the first antigen binding site may comprise or consist of an amino acid sequence of SEQ ID NO: 30.

The amino acid sequence of SEQ ID NO: 32 is as follows.

QVQLVESGGGVVQPGRSLRLSCAASGFTFSRYGMHWVRQAPGKGL
EWVAVIWYDGTNKDYTDSVRGRFTISRDNSKNTLYLQMNSLRAEDTAVYY
CAREDWAEAFDMWGQGTTVTVSSASTKGPSVFPLAPSSKSTSGGTAALGCL
VKDYFPEPVTVSWNSGALTSGVHTFPAVLQSSGLYSLSSVVTVPSSSLGTQT
YICNVNHKPSNTKVDKKVEPKSCDKTHTCPPCPAPEAEGGPSVFLFPPKPKD
TLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK (SEQ ID
NO: 32)

In the present invention, an exemplary bispecific antibody having LALAPG-AAA introduced into the Fc domain may be, but is not limited to, Candidate Antibody B-1. The heavy chain of Candidate Antibody B-1 having LALAPG-AAA introduced into the Fc domain may comprise or consist of an amino acid sequence of SEQ ID NO: 33, and the light chain of the first antigen binding site may comprise or consist of an amino acid sequence of SEQ ID NO: 30.

The amino acid sequence of SEQ ID NO: 33 is as follows.

QVQLVESGGGVVQPGRSLRLSCAASGFTFSRYGMHWVRQAPGKGL
EWVAVIWYDGTNKDYTDSVRGRFTISRDNSKNTLYLQMNSLRAEDTAVYY
CAREDWAEAFDMWGQGTTVTVSSASTKGPSVFPLAPSSKSTSGGTAALGCL
VKDYFPEPVTVSWNSGALTSGVHTFPAVLQSSGLYSLSSVVTVPSSSLGTQT
YICNVNHKPSNTKVDKKVEPKSCDKTHTCPPCPAPEAAGGPSVFLFPPKPKD
TLMASRTPEVTCVVVDVSHEDPEVKFNWYVDGVEVHNAKTKPREEQYNST
YRVVSVLTVLAQDWLNGKEYKCKVSNKALGAPIEKTISKAKGQPREPQVYT
LPPSRDELTKNQVSLTCLVKGFYPSDIAVEWESNGQPENNYKTTPPVLDSDG
SFFLYSKLTVDKSRWQQGNVFSCSVMHEALHNAYTQKSLSLSPGK (SEQ ID
NO: 33)

Binding to an Fc receptor may be easily measured by surface plasmon resonance (SPR) using standard equipment, such as, for example, an ELISA or a BIAcore instrument (GE Healthcare), or the Fc receptor itself may be obtained by recombinant expression. The binding affinity of an Fc domain, or of a cell-activating bispecific antibody including an Fc domain, for an Fc receptor may be assessed by using cell lines known to express particular Fc receptors, such as the human NK cell-expressed FcγIIIa receptor. The effector functions of the Fc domain, or of a bispecific antibody of the present invention including an Fc domain, may be measured by methods known in the art.

The bispecific antibody according to the present invention may be a tetravalent antibody having four binding sites. The bispecific antibody according to the present invention is a bispecific antibody having four binding sites. Preferably, the first antigen binding site of the bispecific antibody according to the present invention may be a full-length bivalent antibody, and the second antigen binding site may be composed of two VEGF antagonists in which a soluble extracellular domain of VEGF and an Fc domain of IgG are fused. Therefore, the bispecific antibody according to the present invention has a valency of 2+2.

Faricimab (brand name: Vabysmo), which is a commercially available treatment for macular degeneration, is a 2(1+1) antibody that binds Ang-2 and VEGF CrossMab. Accordingly, in one embodiment of the present invention, two types of 2(1+1)valent antibodies [Fab-VEGFR-Fc(K-H) and Fab-VEGFR-Fc(H-K)] were produced by linking the first antigen binding site and the second antigen binding site to anti-c-Kit antibody and VEGFR using the "knob-into-hole" technology, and one type of 2(1+1)valent antibody in which c-Kit scFv and VEGFR trap were linked with a peptide linker (G4S or G8) was produced, and their purity, stability (HMW and LMW production), thermostability and binding affinity to c-Kit and VEGF were evaluated. However, these antibodies showed endotoxin detection, low stability (HMW and LMW production), low thermostability and low antigen-antibody binding affinity, and thus were confirmed to be unsuitable as bispecific antibodies.

In a specific embodiment of the present invention, the antibody in the form of Fab-VEGFR-Fc(K-H) produced above was named a "comparative antibody" and was used for comparison of efficacy with the bispecific antibody according to the present invention.

The "knob-into-hole" technology is described, for example, in U.S. Patent No. 5,731,168; U.S. Patent No. 7,695,936; Ridgway et al., Prot Eng 9, 617-621 (1996) and Carter, J Immunol Meth 248, 7-15 (2001). In general, the method involves introducing a knob ("knob") into the contact surface of a first polypeptide and introducing a corresponding hole ("hole") into the contact surface of a second polypeptide such that the knob is positioned within the hole to promote heterodimer formation and prevent homodimer formation. The knob is constructed by replacing a small amino acid side chain from the contact surface of the first polypeptide with a larger side chain (e.g., tyrosine or tryptophan). A compensating hole of the same or similar size as the knob is formed on a contact surface of the second polypeptide by replacing the large amino acid side chain with a smaller one (e.g., alanine or threonine). The knobs and holes may be produced by altering the nucleic acid encoding the polypeptide, for example, by site-directed mutagenesis or by peptide synthesis.

In the present invention, the bispecific antibody may have a change in the amino acid sequence of the antibody in order to improve its binding affinity and/or other biological properties. Such modifications include, for example, deletion, insertion and/or substitution of amino acid sequence residues of the antibody. Such amino acid mutations are made based on the relative similarity of the amino acid side chain substituents, such as hydrophobicity, hydrophilicity, charge, size and the like Analysis of the size, shape and type of the amino acid side chain substituents reveals that arginine, lysine and histidine are all positively charged residues; alanine, glycine and serine have similar sizes; and phenylalanine, tryptophan and tyrosine have similar shapes. Therefore, based on these considerations, arginine, lysine and histidine; alanine, glycine and serine; and phenylalanine, tryptophan and tyrosine may be considered as biologically functional equivalents.

Amino acid exchanges in proteins that do not alter the overall activity of the molecule are well known in the art (H. Neurath, R.L.Hill, The Proteins, Academic Press, New York, 1979). The most common exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu and Asp/Gly.

Considering the mutations having the above-described biologically equivalent activity, the bispecific antibodies of the present invention and the nucleic acid molecules encoding the same are interpreted to also include sequences showing substantial identity with the sequences listed in the sequence listing. The substantial identity means a sequence showing at least 60% identity, more preferably 70% identity, even more preferably 80% identity, and most preferably at least 90% identity, when the sequences of the present invention and any other sequences are aligned to the greatest extent possible and the aligned sequences are analyzed by using an algorithm commonly used in the art.

According to one embodiment of the present invention, the bispecific antibody may include a peptide having at least about 90%, 91%, 92%, 93%, 94%, 95%, 96%, 97%, 98% or 99% sequence identity to the above defined amino acid sequence.

A second aspect of the present invention relates to a nucleic acid molecule encoding the above-described bispecific antibody or fragment thereof according to the present invention, a vector including the nucleic acid molecule and a host cell including the vector.

The nucleic acid molecule of the present invention is an isolated nucleic acid molecule. In one embodiment of the present invention, the nucleic acid molecule encoding the bispecific antibody or fragment thereof may comprise or consist of a nucleotide sequence encoding the amino acid sequences of SEQ ID NOs: 2, 4, 6, 13, 15, 17, 21, 27, 28, 29, 30, 31, 32 and 33.

According to a specific embodiment of the present invention, the nucleotide sequences encoding the amino acid sequences of SEQ ID NOs: 2, 4, 6, 13, 15 and 17, respectively, may comprise or consist of the nucleotide sequences of SEQ ID NOs: 35, 37, 39, 46, 48 and 50, in that order, but are not limited thereto.

According to a specific embodiment of the present invention, the nucleotide sequence encoding the amino acid sequence of SEQ ID NO: 21 may comprise or consist of, but is not limited to, a nucleotide sequence of SEQ ID NO: 54.

According to a specific embodiment of the present invention, the nucleotide sequences encoding the amino acid sequences of SEQ ID NOs: 27 and 28, respectively, may comprise or consist of the nucleotide sequences of SEQ ID NOs: 60 and 61, in that order, but are not limited thereto.

The nucleotide sequences of the above sequence numbers 60 and 61 are as follows.

CAGGTGCAGCTGGTGGAATCTGGTGGCGGAGTTGTGCAGCCTGGC
AGATCCCTGAGACTGTCTTGTGCCGCCTCCGGCTTCACCTTCTCAGATAC
GGAATGCACTGGGTCCGACAGGCCCCTGGCAAAGGATTGGAATGGGTCG
CCGTGATTTGGTACGACGGCACCAACAAGGACTACACCGACTCTGTGCG
GGGCAGATTCACCATCTCTCGGGACAACTCCAAGAACACCCTGTACCTGC
AGATGAACTCCCTGAGAGAGCCGAGGACACCGCCGTGTACTACTGTGCC
AGAGAGGATTGGGCCGAAGCCTTCGATATGTGGGGCCAGGGCACAACC
GTGACCGTGTCCTCT (SEQ ID NO: 60)

GACATCGTGATGACCCAGTCTCCACTGAGCCTGCCTGTGACACCT
GGCGAGCCTGCTTCCATCTCCTGCAGATCCTCTCAGTCCCTGCTGCACTCC
AACGGCTACAACTACCTGGACTGGTATCTGCAGAAGCCCGGCCAGTCTCC
TCAGCTGCTGATCTACCTGGGCTCCAACAGAGCTTCTGGCGTGCCCGATA
GATTCTCCGGCTCTGGCTCTGGCACCGACTTCACCCTGAAGATCTCCAGA

GTGGAAGCCGAGGACGTGGGCGTGTACTACTGTATGCAGGCCCTGCAGA
CCATCACCTTCGGCCAGGGAACCAGACTGGAAATCAAG (SEQ ID NO: 61)

According to a specific embodiment of the present invention, the nucleotide sequences encoding the amino acid sequences of SEQ ID NOs: 29, 30, 31, 32 and 33, respectively, may comprise or consist of the nucleotide sequences of SEQ ID NOs: 62, 63, 64, 65 and 66, in that order, but are not limited thereto.

The nucleotide sequences of SEQ ID NOs: 62, 63, 64, 65 and 66 are as follows.

CAGGTGCAGCTGGTGGAATCTGGTGGCGGAGTTGTGCAGCCTGGCAGATCCCTGAGACTGTCTTGTGCCGC
CTCCGGCTTCACCTTCTCCAGATACGGAATGCACTGGGTCCGACAGGCCCCTGGCAAAGGATTGGAATGGGTCGCCG
TGATTTGGTACGACGGCACCAACAAGGACTACACCGACTCTGTGCGGGGCAGATTCACCATCTCTCGGGACAACTCC
AAGAACACCCTGTACCTGCAGATGAACTCCCTGAGAGCCGAGGACACCGCCGTGTACTACTGTGCCAGAGAGGATTG
GGCCGAAGCCTTCGATATGTGGGGCCAGGGCACAACCGTGACCGTGTCCTCTGCTAGCACCAAGGGACCCAGCGTGT
TCCCTCTGGCTCCTTCCAGCAAGTCTACCTCTGGCGGAACAGCTGCTCTGGGCTGCCTGGTCAAGGATTACTTCCCT
GAGCCTGTGACAGTGTCCTGGAACTCTGGCGCTCTGACATCCGGCGTGCACACCTTTCCAGCTGTGCTGCAATCCTC
CGGCCTGTACTCTCTGTCCTCCGTCGTGACAGTGCCTTCCAGCTCTCTGGGAACCCAGACCTACATCTGCAATGTGA
ACCACAAGCCTTCCAACACCAAGGTGGACAAGAAGGTGGAACCCAAGTCCTGCGACAAGACCCACACCTGTCCACCA
TGTCCTGCTCCAGAACTGCTCGGCGGACCTTCCGTGTTCCTGTTTCCTCCAAAGCCTAAGGACACCCTGATGATCTC
TCGGACCCCTGAAGTGACCTGCGTGGTGGTGGATGTGTCTCACGAGGATCCCGAAGTGAAGTTCAATTGGTACGTGG
ACGGCGTGGAAGTGCACAACGCCAAGACCAAGCCTAGAGAGGAACAGTACAACTCCACCTACAGAGTGGTGTCCGTG
CTGACCGTGCTGCACCAGGATTGGCTGAACGGCAAAGAGTACAAGTGCAAGGTGTCCAACAAGGCCCTGCCTGCTCC
TATCGAAAAGACCATCTCCAAGGCCAAGGGCCAGCCTAGAGAACCCCAGGTTTACACCTTGCCTCCATCTCGGGACG
AGCTGACCAAGAACCAGGTGTCCCTGACCTGTCTCGTGAAGGGCTTCTACCCCTCCGACATCGCCGTGGAATGGGAG
TCTAATGGCCAGCCTGAGAACAACTACAAGACAACCCCTCCTGTGCTGGACTCCGACGGCTCATTCTTCCTGTACTC
CAAGCTGACAGTGGACAAGTCCAGATGGCAGCAGGGCAACGTTTTCTCCTGCTCCGTGATGCACGAGGCCCTGCACA
ATCACTACACCCAGAAGTCCCTGTCTCTGAGCCCCGGCAAA (SEQ ID NO: 62)

GACATCGTGATGACCCAGTCTCCACTGAGCCTGCCTGTGACACCTGGCGAGCCTGCTTCCATCTCCTGCAG
ATCCTCTCAGTCCCTGCTGCACTCCAACGGCTACAACTACCTGGACTGGTATCTGCAGAAGCCCGGCCAGTCTCCTC
AGCTGCTGATCTACCTGGGCTCCAACAGAGCTTCTGGCGTGCCCGATAGATTCTCCGGCTCTGGCTCTGGCACCGAC
TTCACCCTGAAGATCTCCAGAGTGGAAGCCGAGGACGTGGGCGTGTACTACTGTATGCAGGCCCTGCAGACCATCAC
CTTCGGCCAGGGAACCAGACTGGAAATCAAGCGTACGGTGGCCGCTCCTTCCGTGTTCATCTTCCCACCTTCCGACG
AGCAGCTTAAGTCCGGCACAGCTTCTGTCGTGTGCCTGCTGAACAACTTCTACCCTCGGGAAGCCAAGGTGCAGTGG
AAGGTGGACAATGCCCTGCAGTCCGGCAACTCCCAAGAGTCTGTGACCGAGCAGGACTCCAAGGACAGCACCTACAG
CCTGTCCTCCACACTGACCCTGTCCAAGGCCGACTACGAGAAGCACAAGGTGTACGCCTGCGAAGTGACCCATCAGG
GCCTGTCTAGCCCTGTGACCAAGTCTTTCAACCGGGGCGAGTGC (SEQ ID NO: 63)

CAGGTGCAGCTGGTGGAATCTGGTGGCGGAGTTGTGCAGCCTGGCAGATCCCTGAGACTGTCTTGTGCCGC
CTCCGGCTTCACCTTCTCCAGATACGGAATGCACTGGGTCCGACAGGCCCCTGGCAAAGGATTGGAATGGGTCGCCG
TGATTTGGTACGACGGCACCAACAAGGACTACACCGACTCTGTGCGGGGCAGATTCACCATCTCTCGGGACAACTCC
AAGAACACCCTGTACCTGCAGATGAACTCCCTGAGAGCCGAGGACACCGCCGTGTACTACTGTGCCAGAGAGGATTG
GGCCGAAGCCTTCGATATGTGGGGCCAGGGCACAACCGTGACCGTGTCCTCTGCTAGCACCAAGGGACCCAGCGTGT
TCCCTCTGGCTCCTTCCAGCAAGTCTACCTCTGGCGGAACAGCTGCTCTGGGCTGCCTGGTCAAGGATTACTTCCCT
GAGCCTGTGACAGTGTCCTGGAACTCTGGCGCTCTGACATCCGGCGTGCACACCTTTCCAGCTGTGCTGCAATCCTC
CGGCCTGTACTCTCTGTCCTCCGTCGTGACAGTGCCTTCCAGCTCTCTGGGAACCCAGACCTACATCTGCAATGTGA
ACCACAAGCCTTCCAACACCAAGGTGGACAAGAAGGTGGAACCCAAGTCCTGCGACAAGACCCACACCTGTCCACCA
TGTCCTGCTCCAGAAGCCGCCGGCGGACCTTCCGTGTTCCTGTTTCCTCCAAAGCCTAAGGACACCCTGATGATCTC
TCGGACCCCTGAAGTGACCTGCGTGGTGGTGGATGTGTCTCACGAGGATCCCGAAGTGAAGTTCAATTGGTACGTGG
ACGGCGTGGAAGTGCACAACGCCAAGACCAAGCCTAGAGAGGAACAGTACAACTCCACCTACAGAGTGGTGTCCGTG
CTGACCGTGCTGCACCAGGATTGGCTGAACGGCAAAGAGTACAAGTGCAAGGTGTCCAACAAGGCCCTGGGCGCTCC
TATCGAAAAGACCATCTCCAAGGCCAAGGGCCAGCCTAGAGAACCCCAGGTTTACACCTTGCCTCCATCTCGGGACG
AGCTGACCAAGAACCAGGTGTCCCTGACCTGTCTCGTGAAGGGCTTCTACCCCTCCGACATCGCCGTGGAATGGGAG
TCTAATGGCCAGCCTGAGAACAACTACAAGACAACCCCTCCTGTGCTGGACTCCGACGGCTCATTCTTCCTGTACTC
CAAGCTGACAGTGGACAAGTCCAGATGGCAGCAGGGCAACGTTTTCTCCTGCTCCGTGATGCACGAGGCCCTGCACA
ATCACTACACCCAGAAGTCCCTGTCTCTGAGCCCCGGCAAA (SEQ ID NO: 64)

CAGGTGCAGCTGGTGGAATCTGGTGGCGGAGTTGTGCAGCCTGGCAGATCCCTGAGACTGTCTTGTGCCGC
CTCCGGCTTCACCTTCTCCAGATACGGAATGCACTGGGTCCGACAGGCCCCTGGCAAAGGATTGGAATGGGTCGCCG
TGATTTGGTACGACGGCACCAACAAGGACTACACCGACTCTGTGCGGGGCAGATTCACCATCTCTCGGGACAACTCC
AAGAACACCCTGTACCTGCAGATGAACTCCCTGAGAGCCGAGGACACCGCCGTGTACTACTGTGCCAGAGAGGATTG
GGCCGAAGCCTTCGATATGTGGGGCCAGGGCACAACCGTGACCGTGTCCTCTGCTAGCACCAAGGGACCCAGCGTGT
TCCCTCTGGCTCCTTCCAGCAAGTCTACCTCTGGCGGAACAGCTGCTCTGGGCTGCCTGGTCAAGGATTACTTCCCT
GAGCCTGTGACAGTGTCCTGGAACTCTGGCGCTCTGACATCCGGCGTGCACACCTTTCCAGCTGTGCTGCAATCCTC
CGGCCTGTACTCTCTGTCCTCCGTCGTGACAGTGCCTTCCAGCTCTCTGGGAACCCAGACCTACATCTGCAATGTGA
ACCACAAGCCTTCCAACACCAAGGTGGACAAGAAGGTGGAACCCAAGTCCTGCGACAAGACCCACACCTGTCCACCA
TGTCCTGCTCCAGAAGCCGAAGGCGGACCTTCCGTGTTCCTGTTTCCTCCAAAGCCTAAGGACACCCTGATGATCTC
TCGGACCCCTGAAGTGACCTGCGTGGTGGTGGATGTGTCTCACGAGGATCCCGAAGTGAAGTTCAATTGGTACGTGG
ACGGCGTGGAAGTGCACAACGCCAAGACCAAGCCTAGAGAGGAACAGTACAACTCCACCTACAGAGTGGTGTCCGTG
CTGACCGTGCTGCACCAGGATTGGCTGAACGGCAAAGAGTACAAGTGCAAGGTGTCCAACAAGGCCCTGCCTGCTCC
TATCGAAAAGACCATCTCCAAGGCCAAGGGCCAGCCTAGAGAACCCCAGGTTTACACCTTGCCTCCATCTCGGGACG
AGCTGACCAAGAACCAGGTGTCCCTGACCTGTCTCGTGAAGGGCTTCTACCCCTCCGACATCGCCGTGGAATGGGAG
TCTAATGGCCAGCCTGAGAACAACTACAAGACAACCCCTCCTGTGCTGGACTCCGACGGCTCATTCTTCCTGTACTC
CAAGCTGACAGTGGACAAGTCCAGATGGCAGCAGGGCAACGTTTTCTCCTGCTCCGTGATGCACGAGGCCCTGCACA
ATCACTACACCCAGAAGTCCCTGTCTCTGAGCCCCGGCAAA (SEQ ID NO: 65)

CAGGTGCAGCTGGTGGAATCTGGTGGCGGAGTTGTGCAGCCTGGCAGATCCCTGAGACTGTCTTGTGCCGC
CTCCGGCTTCACCTTCTCCAGATACGGAATGCACTGGGTCCGACAGGCCCCTGGCAAAGGATTGGAATGGGTCGCCG
TGATTTGGTACGACGGCACCAACAAGGACTACACCGACTCTGTGCGGGGCAGATTCACCATCTCTCGGGACAACTCC
AAGAACACCCTGTACCTGCAGATGAACTCCCTGAGAGCCGAGGACACCGCCGTGTACTACTGTGCCAGAGAGGATTG
GGCCGAAGCCTTCGATATGTGGGGCCAGGGCACAACCGTGACCGTGTCCTCTGCTAGCACCAAGGGACCCAGCGTGT
TCCCTCTGGCTCCTTCCAGCAAGTCTACCTCTGGCGGAACAGCTGCTCTGGGCTGCCTGGTCAAGGATTACTTCCCT
GAGCCTGTGACAGTGTCCTGGAACTCTGGCGCTCTGACATCCGGCGTGCACACCTTTCCAGCTGTGCTGCAATCCTC
CGGCCTGTACTCTCTGTCCTCCGTCGTGACAGTGCCTTCCAGCTCTCTGGGAACCCAGACCTACATCTGCAATGTGA
ACCACAAGCCTTCCAACACCAAGGTGGACAAGAAGGTGGAACCCAAGTCCTGCGACAAGACCCACACCTGTCCACCA
TGTCCTGCTCCAGAAGCCGCCGGCGGACCTTCCGTGTTCCTGTTTCCTCCAAAGCCTAAGGACACCCTGATGGCTTC
TCGGACCCCTGAAGTGACCTGCGTGGTGGTGGATGTGTCTCACGAGGATCCCGAAGTGAAGTTCAATTGGTACGTGG
ACGGCGTGGAAGTGCACAACGCCAAGACCAAGCCTAGAGAGGAACAGTACAACTCCACCTACAGAGTGGTGTCCGTG
CTGACCGTGCTGGCCCAGGATTGGCTGAACGGCAAAGAGTACAAGTGCAAGGTGTCCAACAAGGCCCTGGGCGCTCC
TATCGAAAAGACCATCTCCAAGGCCAAGGGCCAGCCTAGAGAACCCCAGGTTTACACCTTGCCTCCATCTCGGGACG
AGCTGACCAAGAACCAGGTGTCCCTGACCTGTCTCGTGAAGGGCTTCTACCCCTCCGACATCGCCGTGGAATGGGAG
TCTAATGGCCAGCCTGAGAACAACTACAAGACAACCCCTCCTGTGCTGGACTCCGACGGCTCATTCTTCCTGTACTC
CAAGCTGACAGTGGACAAGTCCAGATGGCAGCAGGGCAACGTTTTCTCCTGCTCCGTGATGCACGAGGCCCTGCACA
ATGCTTACACCCAGAAGTCCCTGTCTCTGAGCCCCGGCAAA (SEQ ID NO: 66)

The nucleic acid molecule of the present invention is also interpreted to include a nucleotide sequence showing substantial identity to the nucleotide sequence described above. The substantial identity refers to a nucleotide sequence showing at least 80% identity, and more preferably at least 90% identity, when the nucleotide sequence of the present invention described above and any other sequence are arranged to correspond as much as possible and the arranged sequence is analyzed by using an algorithm commonly used in the art.

In one embodiment, the nucleic acid molecule may include a nucleic acid molecule having at least about 90%, 91%, 92%, 93%, 94%, 95%, 96%, 97%, 98% or 99% sequence identity to the nucleotide sequence defined above.

In the present invention, the vector includes a plasmid vector; a phagemid vector; a cosmid vector; and a viral vector such as a bacteriophage vector, an adenovirus vector, a retrovirus vector and an adeno-associated virus vector, and is preferably a plasmid vector.

The vector system of the present invention may be constructed by various methods known in the art, and specific methods thereof are disclosed in Sambrook et al. (2001), Molecular Cloning, A Laboratory Manual, Cold Spring Harbor Laboratory Press, which is incorporated herein by reference.

The vector of the present invention may typically be constructed as a vector for cloning or as a vector for expression. In addition, the vector of the present invention may be constructed by using a prokaryotic cell or a eukaryotic cell as a host.

When the vector of the present invention is an expression vector and uses a prokaryotic cell as a host, it generally includes a strong promoter capable of driving transcription (e.g., tac promoter, lac promoter, lacUV5 promoter, lpp promoter, pLλ promoter, pRλ promoter, rac5 promoter, amp promoter, recA promoter, SP6 promoter, trp promoter, T7 promoter, etc.), a ribosome binding site for the initiation of translation, and a transcription/translation termination sequence. When *E. coli* (e.g., BL21, HB101, DH5α, etc.) is used as a host cell, the promoter and operator regions of *the E. coli* tryptophan biosynthetic pathway (Yanofsky, C. (1984), J. Bacteriol., 158:1018-1024) and the left-hand promoter of phage λ (pLλ promoter, Herskowitz, I. and Hagen, D. (1980), Ann. Rev. Genet., 14:399-445) may be used as regulatory regions.

The vector that can be used in the present invention may be produced by manipulating plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series (pET28a, pET21a, etc.), and pUC19, etc.), phagemids (e.g., pComb3X), phages (e.g., λgt4·λB, λ-Charon, λΔz1, and M13, etc.) or viruses (e.g., SV40, etc.) that are frequently used in the art.

When the vector of the present invention is an expression vector and uses a eukaryotic cell as a host, a promoter derived from the genome of a mammalian cell (e.g., a metallothionein promoter) or a promoter derived from a mammalian virus (e.g., an adenovirus late promoter, a vaccinia virus 7.5K promoter, an SV40 promoter, a cytomegalovirus promoter, and a HSV tk promoter) may be used, and it generally has a polyadenylation sequence as a transcription termination sequence.

The vector of the present invention may be fused with other sequences as needed to facilitate purification of the amino-terminal protein of a protein expressed therefrom. Examples of the sequence to be fused include, but are not limited to, glutathione S-transferase (Pharmacia, USA), maltose binding protein (NEB, USA), FLAG (IBI, USA) and 6x His (hexahistidine; Quiagen, USA).

The expression vector of the present invention may include an antibiotic resistance gene commonly used in the art as a selectable marker, for example, a resistance gene for ampicillin, gentamicin, carbenicillin, chloramphenicol, streptomycin, kanamycin, geneticin, neomycin and tetracycline.

The vector expressing the bispecific antibody of the present invention may be a vector system in which the first antigen binding site and the second antigen binding site are expressed in a single vector in a form in which they are linked by a peptide linker, or a system in which the first antigen binding site and the second antigen binding site are expressed in separate vectors.

The host cell according to one embodiment of the present invention is a cell transformed with the vector described above. Any host cell known in the art that can stably and continuously clone and express the vector of the present invention may be used, and examples thereof include, but are not limited to, prokaryotic host cells such as *Bacillus* spp. such as *Escherichia coli, Bacillus subtilis* and *B. thuringensis, Streptomyces, Pseudomonas* (e.g., *Pseudomonas putida), Proteus mirabilis* or *Staphylococcus* (e.g., *Staphylococcus carnosus).*

Suitable eukaryotic host cells for the above vector include multicellular fungi such as *Aspergillus* spp. and *Neurospora crassa* belonging to the Phylum Ascomycota, unicellular fungi including yeasts such as *Pichia pastoris, Saccharomyces cerevisiae, and Schizosaccharomyces,* other lower eukaryotic cells, higher eukaryotic cells such as insect-derived cells, and cells derived from plants or mammals.

In the present invention, the term "transfection" refers to introducing a desired gene into a host cell using the recombinant vector of the present invention, and is used in the same meaning as "transformation". Therefore, "transfection" and/or "transformation" into a host cell includes any method for introducing a nucleic acid into an organism, cell, tissue or organ, and it may be performed by selecting a standard technique suitable for the host cell as known in the art. Such methods include, but are not limited to, electroporation, protoplast fusion, calcium phosphate (CaPO₄) precipitation, calcium chloride (CaCl₂) precipitation, stirring using silicon carbide fibers, Agrobacterium-mediated transformation, PEG, dextran sulfate, lipofectamine, and desiccation/inhibition-mediated transformation methods.

A third aspect of the present invention relates to a method for producing a bispecific antibody that specifically binds to c-Kit and VEGF, including steps (a) and (b) below: (a) culturing the transformant described above; and (b) recovering the bispecific antibody that specifically binds to c-Kit and VEGF from a culture.

The culture of the transformant for the antibody may be carried out according to the appropriate medium and culture conditions known in the art. This culture process may be easily adjusted and used by a person skilled in the art according to the selected strain. Cell culture is divided into suspension culture and attachment culture according to the cell growth method, and batch, fed-batch and continuous culture methods according to the culture method. The medium used for culture must appropriately satisfy the requirements of a specific strain.

The medium used for culturing animal cells include various carbon sources, nitrogen sources and trace element components. Examples of carbon sources that can be used include carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, and organic acids such as acetic acid. These carbon sources may be used alone or in combination. Examples of nitrogen sources that can be used include organic nitrogen sources such as peptone, yeast extract, meat juice, malt extract, corn steep liquor (CSL), and soybean meal, and inorganic nitrogen sources such as urea, ammonium sulfate, ammonium chloride, ammonium phosphate, ammonium carbonate, and ammonium nitrate. These nitrogen sources may be used alone or in combination. The medium may include, as a phosphorus source, potassium dihydrogen phosphate, potassium dihydrogen phosphate and the corresponding sodium-containing salt. In addition, it may include a metal salt such as magnesium sulfate or iron sulfate. In addition, it may include amino acids, vitamins, and suitable precursors and the like.

During the culture, compounds such as ammonium hydroxide, potassium hydroxide, ammonia, phosphoric acid and sulfuric acid may be added to the culture in an appropriate manner to adjust the pH of the culture. In addition, during the culture, an antifoaming agent such as fatty acid polyglycol ester may be used to suppress bubble formation. Additionally, in order to maintain the aerobic state of the culture, oxygen or an oxygen-containing gas (e.g., air) is injected into the culture. The temperature of the culture is usually 20°C to 45°C, and preferably 25°C to 40°C.

The antibody obtained by culturing the transformant may be used in an unpurified state, and may be further purified to a high purity using various conventional methods, such as dialysis, salt precipitation, and chromatography, and then used. Among these, the method using chromatography is the most commonly used, and the type and order of the column may be selected from ion exchange chromatography, size exclusion chromatography and affinity chromatography depending on the characteristics of the antibody, the culturing method and the like.

A fourth aspect of the present invention relates to a pharmaceutical composition for preventing or treating an angiogenic disease using the bispecific antibody described above.

Specifically, the present invention provides a c-Kit and VEGF bispecific antibody according to the first aspect for use in preventing or treating an angiogenic disease and a pharmaceutical composition for preventing or treating an angiogenic disease including the same as an active ingredient.

In addition, the present invention provides a method for preventing or treating an angiogenic disease, including administering a therapeutically effective amount of a c-Kit and VEGF bispecific antibody according to the first aspect to a subject in need thereof.

Furthermore, the present invention provides the use of a c-Kit and VEGF bispecific antibody according to the first aspect in the manufacture of a medicament for preventing or treating an angiogenic disease.

The angiogenic disease refers to a disease related to the occurrence or progression of angiogenesis. If it is a disease that can be treated with the bispecific antibody according to the present invention, it may be included in the scope of angiogenic disease. For example, the angiogenic disease may be selected from the group consisting of cancer, leukemia, ocular vascular disease, rheumatoid arthritis, psoriasis, chronic wound, chronic inflammation, hemangioma, angiofibroma, vascular malformation, arteriosclerosis, vascular adhesion, vasculitis, pyogenic granuloma, bullous disease, pulmonary hypertension, asthma, nasal polyp, infectious disease, inflammatory bowel disease, periodontal disease, peritoneal adhesion, endometrium, uterine bleeding, ovarian cyst, osteomyelitis, osteophyte, sepsis and autoimmune disease, but is not limited thereto.

In the present invention, the cancer may be selected from the group consisting of bone cancer, lung cancer, head and neck cancer, thyroid cancer, parathyroid cancer, non-small cell lung cancer, stomach cancer, liver cancer, pancreatic cancer, skin cancer, cutaneous or intraocular melanoma, rectal cancer, anal cancer, colon cancer, uterine cancer, breast cancer, ovarian cancer, fallopian tube carcinoma, endometrial carcinoma, cervical carcinoma, vaginal carcinoma, vulvar carcinoma, Hodgkin's disease, esophageal cancer, small intestine cancer, endocrine cancer, adrenal cancer, soft tissue sarcoma, urethral cancer, penile cancer, prostate cancer, bladder cancer, kidney cancer, ureteral cancer, renal cell carcinoma, renal pelvic carcinoma, central nervous system tumor, central nervous system lymphoma, spinal cord tumor, glioblastoma, brainstem glioma and pituitary adenoma, but is not limited thereto.

In the present invention, the ocular vascular disease may be selected from the group consisting of diabetic retinopathy, macular degeneration, age-related macular degeneration, glaucoma, glaucomatous retinitis pigmentosa, choroidal neovascularization, retinopathy of prematurity, corneal dystrophy and retinal detachment, but is not limited thereto.

The pharmaceutical composition according to the present invention may contain the bispecific antibody alone, or may additionally contain one or more pharmaceutically acceptable carriers, excipients or diluents.

The pharmaceutically acceptable carrier may further include, for example, a carrier for oral administration or a carrier for parenteral administration. The carrier for oral administration may include lactose, starch, cellulose derivatives, magnesium stearate, stearic acid and the like. In addition, the carrier for parenteral administration may include water, suitable oils, saline solution, aqueous glucose and glycol, and may further include a stabilizer and a preservative. Examples of stabilizers include antioxidants such as sodium bisulfite, sodium sulfite or ascorbic acid. Examples of preservatives include benzalkonium chloride, methyl- or propyl-paraben and chlorobutanol. Other pharmaceutically acceptable carriers known in the art may be used (Remington's Pharmaceutical Sciences, 19th ed, Mack Publishing Company, Easton, PA, 1995).

The pharmaceutical composition of the present invention may be administered to mammals, including humans, by any method. For example, it may be administered orally or parenterally. As a parenteral administration method, it may be intravenous, intramuscular, intraarterial, intramedullary, intrathecal, intracardiac, transdermal, subcutaneous, intraperitoneal, intranasal, enteral, topical, sublingual or rectal administration, but is not limited thereto. For example, the pharmaceutical composition of the present invention may be prepared in an injectable formulation and administered by lightly pricking the skin with a 30-gauge thin injection needle, or by directly applying the same to the skin.

The pharmaceutical composition of the present invention may be formulated as a preparation for oral administration or parenteral administration according to the administration route as described above. In the case of a preparation for oral administration, the composition of the present invention may be formulated as a powder, granule, tablet, pill, sugar-coated tablet, capsule, liquid, gel, syrup, slurry, suspension or the like by using a method known in the art. For example, an oral preparation may be obtained by mixing an active ingredient with a solid excipient, grinding the mixture, adding a suitable auxiliary agent, and then processing the same into a granule mixture to obtain a tablet or sugar-coated tablet. Examples of excipients may include sugars including lactose, dextrose, sucrose, sorbitol, mannitol, xylitol, erythritol and maltitol; starches including corn starch, wheat starch, rice starch and potato starch; cellulosics including cellulose, methyl cellulose, sodium carboxymethylcellulose and hydroxypropylmethylcellulose; and fillers such as gelatin and polyvinylpyrrolidone. In addition, cross-linked polyvinylpyrrolidone, agar, alginic acid, or sodium alginate may be added as a disintegrant, if desired. In addition, the pharmaceutical composition of the present invention may further include an anticoagulant, a lubricant, a wetting agent, a flavoring agent, an emulsifier and a preservative. In the case of parenteral administration preparations, they may be formulated in the form of injections, creams, lotions, ointments for external use, oils, moisturizers, gels, aerosols and nasal inhalers by methods known in the art. These formulations are described in a generally known reference in all pharmaceutical chemistry (Remington's Pharmaceutical Science, 15th Edition, 1975 Mack Publishing Company, Easton, Pennsylvania 18042, Chapter 87: Blaug, Seymour).

The total effective amount of the pharmaceutical composition of the present invention may be administered to a patient as a single dose, or may be administered by a fractionated treatment protocol in which multiple doses are administered for a long period of time. The pharmaceutical composition of the present invention may have different contents of the effective ingredient depending on the degree of symptoms of the disease. For example, the daily dosage of the pharmaceutical composition of the present invention may be 0.0001 to 100 mg/kg. However, for the dosage of the pharmaceutical composition of the present invention, an appropriate effective dosage for the patient may be determined by one of ordinary skill in the art in this field by taking into account various factors such as the administration route and the number of treatments as well as the patient's age, weight, health condition, gender, severity of the disease, diet and excretion rate. The pharmaceutical composition according to the present invention is not particularly limited in its formulation, administration route and administration method as long as it exhibits the effects of the present invention.

In addition, the pharmaceutical composition of the present invention may be administered as an individual therapeutic agent or in combination with other therapeutic agents. When administered in combination with other therapeutic agents, the composition of the present invention and other therapeutic agents may be administered simultaneously, separately or sequentially. The other therapeutic agent may be a substance already known to have a therapeutic or improving effect on angiogenic diseases, and includes all other anticancer therapies other than drug therapy, such as radiation therapy.

When the pharmaceutical composition of the present invention is administered in combination with another therapeutic agent, the bispecific antibody and the other therapeutic agent contained in the composition of the present invention may be formulated separately in separate containers or may be formulated together in the same container.

In the present invention, the term "subject" includes, but is not limited to, any animal (e.g., human, horse, pig, rabbit, dog, sheep, goat, non-human primate, cow, cat, guinea pig or rodent). This term does not indicate a particular age or gender. Therefore, it is intended to include female/female or male/male, adult/adult and newborn subjects, as well as fetuses. A patient refers to a subject suffering from a disease or disorder. The term patient includes human and veterinary subjects.

A fifth aspect of the present invention relates to a kit for detecting c-Kit and/or VEGF including the above-described bispecific antibody and a method for detecting c-Kit and/or VEGF using the above-described bispecific antibody.

The kit for detecting c-Kit and/or VEGF according to the present invention may be used for the purpose of diagnosing angiogenic diseases by applying the same to a biological sample. Accordingly, the present invention provides a kit for diagnosing an angiogenic disease including the above-described bispecific antibody and a method for diagnosing an angiogenic disease using the above-described bispecific antibody.

The kit of the present invention may be manufactured to be suitable for various immunoassays or immunostaining. The immunoassays or immunostaining include, but are not limited to, enzyme-linked immunosorbent assay (ELISA), immunofluorescence, Western blotting, immunohistochemistry staining, flow cytometry, immunocytochemistry, radioimmunoassay (RIA), immunoprecipitation assay, radioimmunoassay (RIA) and protein chips.

The kit of the present invention may additionally include tools or reagents known in the art for use in immunological analysis in addition to the bispecific antibody for c-Kit and VEGF.

Tools or reagents used in immunological analysis may include suitable carriers or supports, labels capable of generating detectable signals, solubilizers, detergents and stabilizers. Suitable carriers may include, but are not limited to, a substrate capable of measuring enzyme activity when the label is an enzyme, a suitable buffer solution, a secondary antibody labeled with a chromogenic enzyme or fluorescent substance, a chromogenic substrate and a reaction terminator.

The bispecific antibody against c-Kit and VEGF included in the kit of the present invention may preferably be immobilized on a suitable carrier or support by using various methods as disclosed in the literature, and examples of suitable carriers or supports include PBS, polystyrene, polyethylene, polypropylene, polyester, polyacrylonitrile, fluoropolymer, agarose, cellulose, nitrocellulose, dextran, sephadex, sepharose, liposome, carboxymethyl cellulose, polyacrylamide, polysterene, gabbro, filter paper, ion exchange resin, plastic film, plastic tube, polyamine-methyl vinylether-maleic acid copolymer, amino acid copolymer, ethylene-maleic acid copolymer, nylon, metal, glass, glass beads or magnetic particles. Other solid substrates include cell culture plates, ELISA plates, tubes and polymeric membranes. The support may have any possible shape, for example, spherical (bead), cylindrical (inside of a test tube or well) or planar (sheet, test strip).

Labels capable of generating a detectable signal enable qualitative or quantitative measurement of the formation of an antigen-antibody complex, and examples of such labels include enzymes, fluorescent substances, ligands, luminescent substances, microparticles, redox molecules and radioactive isotopes. Enzymes that can be used include β-glucuronidase, β-D-glucosidase, urease, peroxidase (such as horseradish peroxidase), alkaline phosphatase, acetylcholinesterase, glycose oxidase, hexokinase, malate dehydrogenase, glucose-6-phosphate dehydrogenase, invertase and luciferase. Fluorescent substances that can be used include fluorescein, isothiocyanate, rhodamine, phycoerythelin, phycocyanin, allophycocyanin and fluorsine isothiocyanate. Ligands include biotin derivatives and the like, and luminescent substances include acridinium ester, luciferin and the like. Microparticles include colloidal gold, colored latex and the like, and redox molecules include ferrocene, ruthenium complexes, viologen, quinone, Ti ion, Cs ion, diimide, 1,4-benzoquinone, hydroquinone and the like. Radioisotopes include ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, ¹⁸⁶Re and the like. However, in addition to those exemplified above, any one that can be used in an immunological analysis method may be used.

As an enzyme chromogenic substrate, for example, when horseradish peroxidase (HRP) is selected as an enzyme label, a solution including 3-amino-9-ethylcarbazole, 5-aminosalicylic acid, 4-chloro-1-naphthol, o-phenylenediamine, 2,2'-azino-bis(3-ethylbenzthiazoline-6-sulfonic acid), 3,3-diaminobenzidine, 3,3',5,5'-tetramethylbenzidine, o-dianisidine or 3,3-dimethoxybenzidine may be used as a substrate. In addition, when alkaline phosphatase is selected as an enzyme label, a solution including 5-bromo-4-chloro-3-indolyl phosphate, nitroblue tetrazolium or p-nitrophenyl phosphate may be used as a substrate. In addition, when β-D-galactosidase is selected as an enzyme label, a solution including o-nitrophenyl-β-D-galactoside or 5-bromo-4-chloro-3-indole-β-D-galactopyranoside may be used as a substrate. In addition, various enzymes and enzyme chromogenic substrates known in the art may be used.

A method for detecting c-Kit and/or VEGF according to one embodiment of the present invention may detect c-Kit and/or VEGF proteins in a biological sample or a mammal including a human. Specifically, the method for detecting c-Kit and/or VEGF according to the present invention may include detecting a c-Kit and/or VEGF antigen-antibody complex using the above-described bispecific antibody.

In the present invention, the sample is a biological sample, and may include, but is not limited to, tissue, cell, whole blood, serum, plasma, tissue autopsy samples (brain, skin, lymph node, spinal cord, etc.), cell culture supernatant, ruptured eukaryotic cells and bacterial expression systems. For example, the biological sample may be isolated from a mammal, including a human, having a disease in which c-Kit and/or VEGF is overexpressed or a disease related to c-Kit and/or VEGF, or may be isolated from an animal model that overexpresses c-Kit and/or VEGF protein or an animal model of a disease related to c-Kit and/or VEGF, but is not limited thereto. These biological samples may be reacted with the bispecific antibody of the present invention, with or without manipulation, to confirm the presence or absence of c-Kit and VEGF proteins.

The mammals include, but are not limited to, domestic animals (e.g., cattle, sheep, cats, dogs and horses), primates (e.g., humans, non-human primates such as monkeys), rabbits and rodents (e.g., mice and rats).

### MODES OF THE INVENTION

Hereinafter, the present invention will be described in more detail by the following examples. However, the following examples are only intended to illustrate the present invention, and the scope of the present invention is not limited thereto.

### [Production Example 1]

### Production of bispecific candidate antibody that specifically binds to c-KIT and VEGF

### 1-1. Production of vector expressing anti-c-Kit antibody

Based on the antibody sequence identified in Patent Document 1, a leader peptide sequence for secretion outside the cell was added, and gene synthesis (commissioned by Bioneer) was performed. The synthesized antibody gene was cloned into the PhiC31 vector (SBI, FC600A-1-SBI). For cloning, first of all, the synthesized antibody gene and PhiC31 vector were each mixed with restriction enzymes NotI (New England Biolabs, R3189S) and EcoRV (New England Biolabs, R3195S) and 10X buffer (10X Cutsmart Buffer) to a total volume of 50 µL, and the mixture was reacted in a 37°C incubator for 5 hours. After expanding on a 1% agarose gel and confirming that a band was generated at the expected size, the band was cut and separated by using a purification kit (MN, Macherey-Nagel). A 20 µL reaction mixture in which the two separated products and T4 ligase (New England Biolabs, M0202S) and 10X buffer (10X T4 DNA Ligase Buffer) were mixed was reacted at 25°C for 2 hours. The ligated vector was transformed into *E. coli* DH5a competent cells (RBC, RH718), spread on kanamycin LB agar plates, and reacted at 37°C for 16 hours. After obtaining a single colony, the colony was cultured, and DNA was obtained by using a Mini-prep (Qiagen, 27106) kit. The obtained DNA was cleaved with the same restriction enzyme, and the insertion of the gene into the vector was confirmed by the size of the cleaved band through electrophoresis, and then, sequencing (commissioned by Bioneer) was used for final analysis, and a vector expressing anti-c-Kit antibody was obtained.

### 1-2. Cloning and expression of bispecific candidate antibody that specifically binds to c-Kit and VEGF

Based on the vector expressing the above-produced anti-c-Kit antibody, gene synthesis (commissioned by Bioneer) was used to clone the heavy chain of the antibody, the peptide linker G5 (SEQ ID NO: 20) and the VEGFR sequence. For cloning, first of all, in order to remove the constant region of the existing vector, restriction enzymes NheI (New England Biolabs, R3131S) and EcoRV (New England Biolabs, R3195S) were mixed with 10X buffer (10X Cutsmart Buffer), and the total reaction volume was made 50 µL, and then, the reaction was performed in an incubator at 37°C for 5 hours. The cleaved band was confirmed through electrophoresis on a 1% agarose gel, and the cut vector was isolated by using a purification kit (MN, Macherey-Nagel). In the same way, the antibody heavy chain, linker and VEGFR genes synthesized by using gene synthesis were also cleaved with restriction enzymes NheI and EcoRV, isolated and purified to secure inserts. In order to link the cleaved vector and the insert, T4 ligase (New England Biolabs, M0202S) and 10X buffer (10X T4 DNA Ligase Buffer) were added to each gene to make a total of 20 µL, and the mixture was reacted at 25°C for 2 hours. The vectors combined into one were transformed by using *E. coli* DH5a competent cells (RBC, RH718), and the cells were spread on kanamycin LB agar plates and reacted at 37°C for 16 hours to confirm the formation of single colonies. DNA was secured by performing Mini-prep (Qiagen, 27106) using single colonies. The secured DNA was cleaved with a restriction enzyme, and the gene was inserted into the vector by confirming the size of the cut band through electrophoresis. Afterwards, it was verified again using sequencing (commissioned by Bioneer).

The vector thus prepared was cultured in 3 mL LB medium containing antibiotics for 8 hours, then increased in amount to 250 mL LB medium containing the same antibiotics and cultured for 16 hours. Plasmid DNA was extracted by using a Maxi-prep (MN, Macherey-Nagel, 740414.50) kit, filtered through a 0.22 µm PES filter, and then introduced into cells as follows.

The day before transfection, ExpiCHO (Gibco) cells were cultured in ExpiCHO Expression Medium (Gibco, A2910002) at a concentration of 3 x 10E6 to 4 x 10E6 viable cells/mL and then cultured at 8% CO₂, 37°C and 120 rpm for 1 day. On the day of DNA transfection, cells grown to 7 x 10E6 to 10 x 10⁶ viable cells/mL (viability rate of 95% or more) were diluted to 6 x 10⁶ viable cells/mL using fresh medium. ExpiFectamine CHO transfection kit (Gibco, A29129) and Optipro SFM (Gibco, 12309019) were used for transfection of the prepared ExpiCHO cells, and DNA transfection was performed at a concentration of 1 µg/mL. After transfection, supplements were added on day 1, and the culture conditions were changed to 5% CO₂, 32°C and 120 rpm. Supplements were then added again on day 5 and cultured for 10 to 12 days to complete production.

In order to secure the culture medium after production was completed, the culture medium was transferred to a centrifugation-only container, and centrifuged at 4°C and 8,000 rpm for 30 minutes, the supernatant was mixed with diatomaceous earth, and filtered through a 0.2 um PES filter to secure the culture medium with suspended matter removed, and the subsequent purification process was performed.

Purification was performed by using affinity chromatography (Protein A) resin, and the column was equilibrated with equilibration buffer (1X PBS). The recovered culture medium was injected into the column. After the injection was completed, the column was washed by using equilibration buffer, and an intermediate wash was performed by using 20 mM sodium citrate, 5% sorbitol and pH 5.0 buffer. After the washing was completed, the column was eluted by using 20 mM sodium citrate, 5% sorbitol and pH 3.5 buffer. The eluted solution was neutralized to pH 7.0 by adding 1 M Tris-HCl, pH 9.0.

If further purification was required, it was purified by using ion exchange chromatography (Cation Exchange) resin. After equilibration using equilibration buffer [20 mM potassium phosphate, pH 7.0], the neutralized eluate was loaded onto the column. After loading was completed, it was washed by using equilibration buffer and eluted by using 20 mM potassium phosphate, pH 8.0 buffer. Afterwards, the eluate was buffer-exchanged and concentrated with 20 mM L-histidine, 9% sucrose and pH 6.0, and its suitability for subsequent activity analysis was confirmed through purity analysis (SEC-HPLC) and endotoxin analysis. The finally confirmed antibody was stored at -80°C until use.

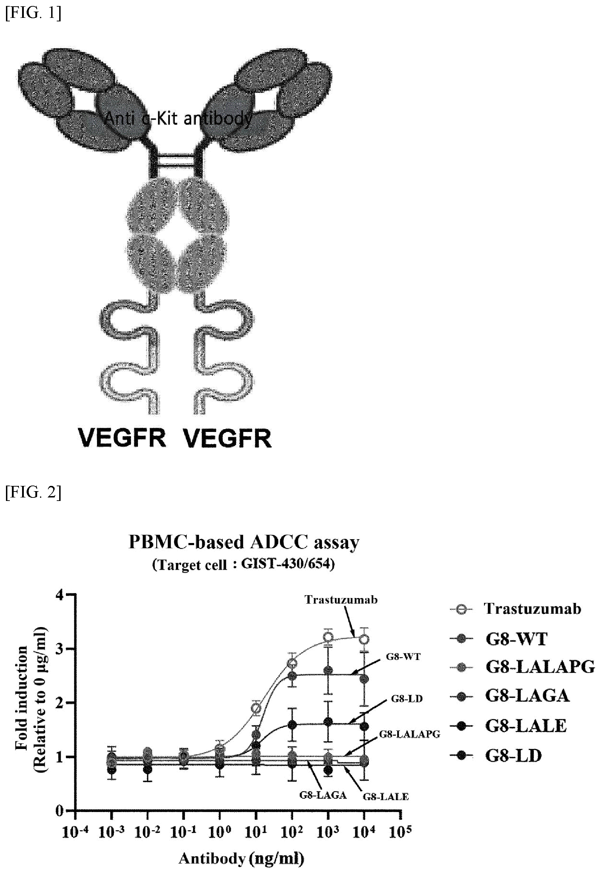

The obtained bispecific antibody is in the form in which VEGFR is linked to the heavy chain C-terminus of the IgG of the anti-c-Kit antibody by a G5 peptide linker. The structure of the produced bispecific antibody is shown in FIG. 1A, and it was named 'Candidate Antibody A'. The amino acid sequence of Candidate Antibody A and the nucleotide sequence information encoding the same are shown in Tables 2 and 3, respectively.

**[Table 2]**

| **Amino acid sequence information of Candidate Antibody A** | | | | |
|---|---|---|---|---|
| **Region** | | | **Amino acid sequence** | **SEQ ID NO** |
| | VH | FR1 | QVQLVESGGGVVQPGRSLRLSCAASGFTFS | 1 |
| | | CDR1 | RYGMH | 2 |
| | | FR2 | WVRQAPGKGLEWVA | 3 |
| | | CDR2 | VIWYDGTNKDYTDSVRG | 4 |
| | | FR3 | RFTISRDNSKNTLYLQMNSLRAEDTAVYYCAR | 5 |
| | | CDR3 | EDWAEAFDM | 6 |
| | | FR4 | WGQGTTVTVSS | 7 |
| | CH | CH1 | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKV | 8 |
| | | **Hinge** | ^{.}EPKSCDKTHTCPPCP | 9 |
| | | CH2 | APELLGGPSVFLFPPKPKDTLM IS RTPEVTCVVVDVSHE DPEVKFNWYVDGVEVHNAKTKPREEQYNSTYRVVSVLTV LHQDWLNGKEYKCKVSNKALPAPI EKT ISKAK | 10 |
| | | CH3 | GQPREPQVYT LPPSRDEL TKNQVS LTCLVKGFYPSDI AV EWESNGQPENNYKTT PPVLDSDGS FFLYSKLTVDKSRWQ QGNVFSCSVMHEA LHNHYTQKS LS LSPGK | 11 |
| | VL | FR1 | DIVMTQSPLSLPVTPGEPASISC | 12 |
| | | CDR1 | RSSQSLLHSNGYNYLD | 13 |
| | | FR2 | WYLQKPGQSPQLLIY | 14 |
| | | CDR2 | LGSNRAS | 15 |
| | | FR3 | GVPDRFSGSGSGTDFTLKISRVEAEDVGVYYC | 16 |
| | | CDR3 | MQALQTIT | 17 |
| | | FR4 | FGQTRLEIK | 18 |
| | CL | CL Kappa | | 19 |
| **G5 linker** | | | GGGGG | 20 |
| VEGFR | | | SDTGRPFVEMYSEIPEIIHMTEGRELVIPCRVTSPNITV TLKKFPLDTLIPDGKRIIWDSRKGFIISNATYKEIGLLT CEATVNGHLYKTNYLTHRQTNTIIDVVLSPSHGIELSVG EKLVLNCTARTELNVGIDFNWEYPSSKHQHKKLVNRDLK TQSGSEMKKFLSTLTIDGVTRSDQGLYTCAASSGLMTKK NSTFVRVHEK | 21 |

**[Table 3]**

| **Nucleotide sequence information of Candidate Antibody A** | | | | |
|---|---|---|---|---|
| **Region** | | | **Nucleotide sequence** | **SEQ ID NO** |
| c-Kit | VH | FR1 | CAGGTGCAGCTGGTGGAATCTGGTGGCGGAGTTGTGCAGCCTGGCAGATCCC TGAGACTGTCTTGTGCCGCCTCCGGCTTCACCTTCTCC | 34 |
| | | CDR1 | AGATACGGARTGCAC | 35 |
| | | FR2 | TGGGTCCGACAGGCCCCTGGCAAAGGATTGGAATGGGTCGCC | 36 |
| | | CDR2 | GTGATTTGGTACGACGGCACCAACAAGGACTACACCGACTCTGCGGGGC | 37 |
| | | FR3 | AGATTCACCATCTCTCGGGACAACTCCAAGAACACCCTGTACCTGCAGATGA ACTCCCTGAGAGCCGAGGACACCGCCGTGTACTACTGTGCCAGA | 38 |
| | | CDR3 | GAGGATTGGGCCGAAGCCTTCGATATG | 39 |
| | | FR4 | TGGGGCCAGGGCACAACCGTGACCGTGTCCTCT | 40 |
| | CH | CH1 | GCTAGCACCAAGGGACCCAGCGTGTTCCCTCTGGCTCCTTCCAGCAAGTCTA CCTCTGGCGGAACAGCTGCTCTGGGCTGCCTGGTCAAGGATTACTTCCCTGA GCCTGTGACAGTGTCCTGGAACTCTGGCGCTCTGACATCCGGCGTGCACACC TTTCCAGCTGTGCTGCAATCCTCCGGCCTGTACTCTCTGTCCTCCGTCGTGA CAGTGCCTTCCAGCTCTCTGGGAACCCAGACCTACATCTGCAATGTGAACCA CAAGCCTTCCAACACCAAGGTGGACAAGAAGGTG | 41 |
| | | **Hinge** | GAACCCAAGTCCTGCGACAAGACCCACACCTGTCCACCATGTCCT | 42 |
| | | CH2 | GCTCCAGAACTGCTCGGCGGACCTTCCGTGTTCCTGTTTCCTCCAAAGCCTA AGGACACCCTGATGATCTCTCGGACCCCTGAAGTGACCTGCGTGGTGGTGGA TGTGTCTCACGAGGATCCCGAAGTGAAGTTCAATTGGTACGTGGACGGCGTG GAAGTGCACAACGCCAAGACCAAGCCTAGAGAGGAACAGTACAACTCCACCT ACAGAGTGGTGTCCGTGCTGACCGTGCTGCACCAGGATTGGCTGAACGGCAA AGAGTACAAGTGCAAGGTGTCCAACAAGGCCCTGCCTGCTCCTATCGAAAAG ACCATCTCCAAGGCCAAG | 43 |
| | | CH3 | GGCCAGCCTAGAGAACCCCAGGTTTACACCTTGCCTCCATCTCGGGACGAGC TGACCAAGAACCAGGTGTCCCTGACCTGTCTCGTGAAGGGCTTCTACCCCTC CGACATCGCCGTGGAATGGGAGTCTAATGGCCAGCCTGAGAACAACTACAAG ACAACCCCTCCTGTGCTGGACTCCGACGGCTCATTCTTCCTGTACTCCAAGC TGACAGTGGACAAGTCCAGATGGCAGCAGGGCAACGTTTTCTCCTGCTCCGT GATGCACGAGGCCCTGCACAATCACTACACCCAGAAGTCCCTGTCTCTGAGC CCCGGCAAA | 44 |
| | VL | FBI | GACATCGTGATGACCCAGTCTCCACTGAGCCTGCCTGTGACACCTGGCGAGC CTGCTTCCATCTCCTGC | 45 |
| | | CDR1 | AGATCCTCTCAGTCCCTGCTGCACTCCAACGGCTACAACTACCTGGAC | 46 |
| | | FR2 | TGETATCTGCAGAAGCCOGGCCAGTCTCCTCAGETECTRATCTAC | 47 |
| | | CDR2 | CTGGGCTCCAACAGAGCTICT | 48 |
| | | FR3 | GGCGTGCCCGATAGATTCTCCGGCTCTGGCTCTGGCACCGACTTCACCCTGA AGATCTCCAGAGTGGAAGCCGAGGACGTGGGCGTGTACTACTGT | 49 |
| | | CDR3 | ATGCAGGCCCTGCAGACCATCACC | 50 |
| | | FR4 | TTCGGCCAGGGAACCAGACTGGAATCAAG | 51 |
| | CL | CL Kappa | CGTACGGTGGCCGCTCCTTCCGTGTTCATCTTCCCACCTTCCGACGAGCAGC TTAAGTCCGGCACAGCTTCTGTCGTGTGCCTGCTGAACAACTTCTACCCTCG GGAAGCCAAGGTGCAGTGGAAGGTGGACAATGCCCTGCAGTCCGGCAACTCC CAAGAGTCTGTGACCGAGCAGGACTCCAAGGACAGCACCTACAGCCTGTCCT CCACACTGACCCTGTCCAAGGCCGACTACGAGAAGCACAAGGTGTACGCCTG CGAAGTGACCCATCAGGGCCTGTCTAGCCCTGTGACCAAGTCTTTCAACCGG GGCGAGTGC | 52 |
| **G5 linker** | | | ggaggcggcggaggc | 53 |
| VEGFR | | | tctgataccggcagacccttcgtggaaatgtacagcgagatccccgagatca tccacatgaccgagggcagagagctggtcatccccctgcagagtgacctctcc taacatcaccgtgactctgaagaagttccctctggacacactgatccccgac ggcaagagaatcatctgggactcccggaagggcttcatcatctccaacgcca cctacaaagagatcggcctgctgacctgcgaggccaccgttaatggccacct gtacaagaccaactatctgacccacagacagaccaacaccatcatcgacgtg gtgctgagccccctctcatggcatcgagctgtccgtgggagaaaagctggtgc tgaactgcaccgccagaaccgagctgaacgtgggcatcgacttcaactggga gtacccctccagcaagcaccagcacaagaagctggtcaaccgggacctgaaa acccagtccggctccgagatgaagaaattcctgagcaccctgaccatcgacg gcgtgaccagatctgaccagggcctgtatacctgcgccgcttcttctggcct gatgaccaagaaaaactccaccttcgtgcgggtgcacgagaag | 54 |

### [Example 1]

### Fc Engineering

### 1-1. Introduction of various Fc mutations

Instead of the G5 peptide linker used in the above production example, a G8 peptide linker was used as the peptide linker for connecting the anti-c-Kit antibody and VEGFR, and mutations were introduced into the Fc to reduce Fc receptor binding and/or effector function. The types of Fc mutations that reduce the binding affinity for FcγRIIIa (CD16a) to less than 10% are shown in Table 4. Bispecific antibodies were produced by inserting the above 4 types of Fc mutations into the Fc region, and for comparison, bispecific antibodies having a G8 peptide linker and a wild-type Fc were also produced. The method for producing these bispecific antibodies is the same as in Production Example 1.

**[Table 4]**

| **Type of Fc mutations** | **LALAPG** | **LAGA** | **LALE** | **LD** |
|---|---|---|---|---|
| FcγIII binding affinity compared to wild type | 3% | 8% | 8% | 1.5% |

### 1-2. Confirmation of binding affinity for Fc receptors

For the 5 types of bispecific antibodies produced (i.e., bispecific antibodies having a G8 peptide linker and a wild-type Fc, LALAPG Fc variant, LAGA Fc variant, LALE Fc variant or LD Fc variant), SPR was performed as follows to select the Fc variant most optimized for reducing the immune effector function.

SPR was performed to determine the binding affinity of the 5 bispecific antibodies in Table 8 to the Fc receptor. A running buffer was prepared by diluting 1X from 20X HBS-EP+ buffer with deionized water, and a regeneration solution (5 mM NaOH) was prepared by diluting 10 N NaOH to 5 mM with deionized water. Immobilization and binding assays were performed at 25°C by using 1X HBS-EP+ buffer as the running buffer. The ligand and analyte were set as Fc receptor and antibody, respectively.

Amine coupling was used as a ligand immobilization method. The EDC/NHS mixture was injected into the sample and reference flow cells for 420 seconds to activate the sensor chip surface. The diluted Fc receptor protein in 10 mM sodium acetate solution was injected only into the sample flow cell to covalently bind the protein to the sensor chip surface. The reference flow cell served as a control to compensate for nonspecific signals, and the protein immobilization process was omitted. When an appropriate immobilization level was achieved, ethanolamine was injected into the sample and reference flow cells for 420 seconds to inactivate the remaining activation sites.

The general settings and running configuration are shown in Table 5.

**[Table 5]**

| Running configurations in human c-Kit analysis | |
|---|---|
| Sensor chip | Series S CM5 |
| Immobilization method | Covalent bond immobilization using amine coupling |
| Activation | EDC/NHS mixture, for 420 seconds |
| Deactivation | Ethanolamine, for 420 seconds |
| Immobilization solution | 10 mM sodium acetate, pH 5.5 |
| Flow rate | 10 uL/min |
| Concentration | 1 ug/mL |
| Running buffer | HBS-EP+ |

A binding analysis was performed to collect data to be used for affinity analysis. The binding and dissociation signals were measured in real time while flowing analytes at various concentrations through the sample and reference flow cells. The injection order was from low to high concentration, and each concentration was injected as an independent cycle. One cycle consists of a baseline section in which only the running buffer flowed before analyte injection, a binding section in which the analyte is injected and binds to the ligand, a dissociation section in which the analyte injection is stopped, only the running buffer flows, and the ligand-analyte complex dissociates, and a regeneration section in which a regeneration solution is injected to return the ligand to its original state.

The analyte concentrations and running configurations for each ligand are shown in Tables 6 and 7.

**[Table 6]**

| Concentration of analytes | |
|---|---|
| **Ligand** | **Concentration range of analytes** |
| Human FcγRI | 1.5625, 3.125, 6.25, 12.5, 25, 50, 100, 200 nM |
| Human FcγRIIa (167 His) | 15.625, 31.25, 62.5, 125, 250, 500, 1000, 2000 nM |
| Human FcγRIIIa (176 Val) | 15.625, 31.25, 62.5, 125, 250, 500, 1000, 2000 nM |
| Human FcγRIIIb (NA1) | 62.5, 125, 250, 500, 1000, 2000 nM |
| Human FcγRIIIb (NA2) | 62.5, 125, 250, 500, 1000, 2000 nM |
| Human FcRn (at pH 6.0) | 62.5, 125, 250, 500, 1000, 2000 nM |
| Human FcRn (at pH 7.4) | 62.5, 125, 250, 500, 1000, 2000 nM |

**[Table 7]**

| Running configurations | |
|---|---|
| Binding time | 120 seconds |
| Dissociation time | 120 seconds, 300 seconds for FcγRI analysis |
| Flow rate | 30 uL/min |
| Regeneration solution and injection time | 5 mM NaOH, 10 seconds |

Biacore Insight Evaluation Software was used to evaluate the collected data.

The steady-state affinity method was used to calculate the binding affinity (KD). In the case of FcγRI, the steady-state affinity method is not suitable, and thus, the binding rate constant (*ka*) and the dissociation rate constant (*kd*) were calculated, and the binding affinity (KD) was calculated as the ratio of the dissociation rate and the association rate. If the binding reaction signal was too low or non-specific binding occurred, the binding affinity (KD) could not be accurately determined, and thus, only the binding was determined.

As confirmed in Table 8, when LALAPG, LAGA and LALE Fc mutations were introduced, the binding affinity between Fc fragments expressed on immune cells and Fcγ receptors decreased. However, contrary to expectations in Table 4, when LD Fc mutations were introduced, the binding affinity to FcγIII increased compared to wild-type Fc, indicating that it is not a suitable Fc mutant.

**[Table 8]**

| | | **Wild type** | **LALAPG** | **LAGA** | **LALE** | **LD** |
|---|---|---|---|---|---|---|
| **KD (M)** | FcγR1a | 1.48x10⁻⁸ | NB | NB | NB | NB |
| | FcγR2a_167H | 1.26x10⁻⁶ (NSB) | NB | NB | NB | 5.78x10⁻⁶ (NSB) |
| | FcγR3a_167V | 4.18x10⁻⁷ | NB | NB | NB | 2.36x10⁻⁶(NSB) |
| | FcγR2b | NB | NB | NB | NB | NB |
| | FcγR3b_NA1 | 7.18x10⁻⁶ | NB | NB | NB | 1.09x10⁻¹(NSB) |
| | FcγR3b_NA2 | 5.44x10⁻⁶ | NB | NB | NB | 1.58x10⁻²(NSB) |
| | FcRn(pH 6.0) | 2.06x10⁻⁶ | 9.21x10⁻⁷ | 1.21x10⁻⁶ | 1.09x10⁻⁶ | 8.11x10⁻⁷ |
| | FcRn(pH 7.4) | 1.56x10⁻² (weak) | 2.45x10⁻² (weak) | 1.28x10⁻⁵ | 4.27x10⁻⁴ | 1.38x10⁻² (wewak) |

Next, antibody-dependent cellular cytotoxicity (ADCC) was tested in peripheral blood mononuclear cells, which include immune cells in human blood, to confirm whether the mutations do not actually induce immune effectors.

In order to measure antibody-dependent cellular cytotoxicity (ADCC), experiments were performed by using 96-well cell culture plates. Target cells, GIST-430/654 cells, were seeded per well at 6 × 10³ in a final volume of 100 µL and cultured overnight. Before starting the ADCC assay, the supernatant was removed, and effector cells, human peripheral blood mononuclear cells (hPBMC; Lonza_CC-2704), were added per well at 1.5 × 10⁵ in a total volume of 50 µL to adjust the effector : target ratio to 25 : 1. Each bispecific antibody was diluted by using PBS to 20 µg/mL (2-fold treatment concentration) and serially diluted 10-fold in the dilution plate. 50 µL of antibody was transferred to the cell culture plate containing target cells and effector cells and reacted at 37°C for 6 hours. After 6 hours of reaction, 100 µL of HiBiT extracellular reagent (Promega, N2421) was added to each well and reacted for about 10 minutes at room temperature. Afterwards, luminescence was measured by using the GloMax^{®} Navigator system. The measured results were graphed and compared with the negative control group (untreated group).

As shown in FIG. 2, cytotoxicity was confirmed to occur in the group treated with the wild-type Fc, and no cytotoxicity was observed in the groups treated with the mutants LALAPG, LAGA and LALE, respectively. On the other hand, although cytotoxicity was reduced compared to the wild type, the LD Fc mutant was confirmed to still have toxicity. Trastuzumab was used as a positive control. Therefore, it was confirmed that the LALAPG, LAGA and LALE Fc mutants significantly reduced ADCC compared to the wild-type Fc, and these three Fc mutants were the optimal mutants.

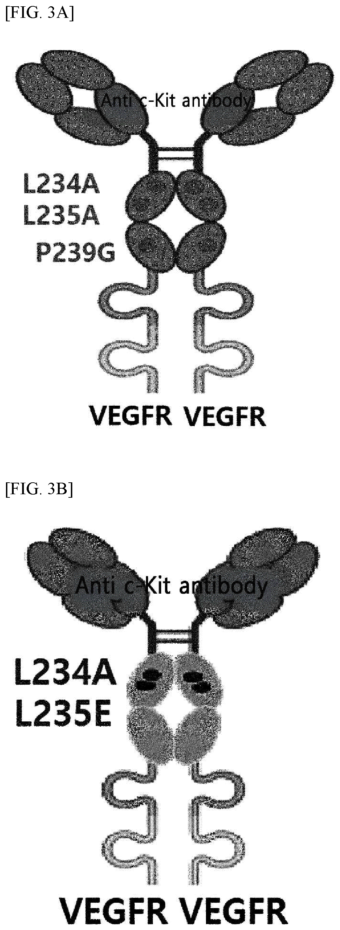

According to the above results, the bispecific antibodies linked by the G8 peptide linker and each introducing LALAPG and LALE Fc mutations were named 'Candidate Antibody B' (LALAPG) and 'Candidate Antibody C' (LALE), respectively, and the structure and sequence information of these bispecific antibodies are shown in FIGS. 3A to 3B and Tables 9 to 12, respectively.

**[Table 9]**

| **Amino acid sequence information of Candidate Antibody B** | | | | |
|---|---|---|---|---|
| **Region** | | | **Amino acid sequence** | **SEQ ID NO** |
| c-Kit | VH | FR1 | QVQLVESGGGVVQPGRSLRLSCAASGFTFS | 1 |
| | | CDR1 | RYGMH | 2 |
| | | FR2 | WVRQAPGKGLEWA | 3 |
| | | CDR2 | VIWYDGTNKDYTDSVRG | 4 |
| | | FR3 | RFTI SRDNSKNTLYLQMNSLRAEDTAVYYCAR | 5 |
| | | CDR3 | EDWAEAFDM | 6 |
| | | FR4 | WGQGTTVTVSS | 7 |
| | CH | CH1 | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKV | 8 |
| | | **Hinge** | EPKSCDKTHTCPPCP | 9 |
| | | **CH2 (LALAPG)** | APEAAGGPSVFLFPPKPKDTLMISRTPEVTCVVVDVSHE DPEVKFNWYVDGVEVHNAKTKPREEQYNSTYRVVSVLTV LHQDWLNGKEYKCKVSNKALGAPIEKTISKAK | 22 |
| | | CH3 | GQPREPQVYTLPPSRDELTENQVSLTCLVKGFYPSDIAV EWESNGQPENNYKTTPPVLDSDGSFFLYSKLTVDKSRWQ QGNVFSCSVMHEALHNHYTQKSLSLSPGK | 11 |
| | VL | FR1 | DIVMTQSPLSLPVTPGEPASISC | 12 |
| | | CDR1 | RSSQSLLHSNGYNYLD | 13 |
| | | FR2 | WYLQKPGQSPQLLIY | 14 |
| | | CDR2 | LGSNRAS | 15 |
| | | FR3 | GVPDRFSGSGSGTDFTLKISRVEAEDVGVYYC | 16 |
| | | CDR3 | MQALQTIT | 17 |
| | | FR4 | FGQGTRLEIK | 18 |
| | CL | CL Kappa | | 19 |
| **G8 linker** | | | GGGGGGGG | 23 |
| VEGFR | | | SDTGRPFVEMYSEIPEIIHMTEGRELVIPCRVTSPNITV TLKKFPLDTLIPDGKRIIWDSRKGFIISNATYKEIGLLT CEATVNGHLYKTNYLTHRQTNTIIDVVLSPSHGIELSVG EKLVLNCTARTELNVGIDFNWEYPSSKHQHKKLVNRDLK TQSGSEMKKFLSTLTIDGVTRSDQGLYTCAASSGLMTKK NSTFVRVHEK | 21 |

**[Table 10]**

| **Nucleotide sequence information of Candidate Antibody B** | | | | |
|---|---|---|---|---|
| **Region** | | | **Nucleotide sequence** | **SEQ ID NO** |
| c-Kit | VH | FR1 | CAGGTGCAGCTGGTGGAATCTGGTGGCGGAGTTGTGCAGCCTGGCAGATCCCTGAG ACTGTCTTGTGCCGCCTCCGGCTTCACCTTCTCC | 34 |
| | | CDR1 | AGATACGGAATGCAC | 35 |
| | | FR2 | TGGGTCCGACAGGCCCCTGGCAAAGGATTGGAATGGGTCGCC | 36 |
| | | CDR2 | GTGATTTGGTACGACGGCACCAACAAGGACTACACCGACTCTGTGCGGGGC | 37 |
| | | FR3 | AGATTCACCATCTCTCGGGACAACTCCAAGAACACCCTGTACCTGCAGATGAACTC CCTGAGAGCCGAGGACACCGCCGTGTACTACTGTGCCAGA | 38 |
| | | CDR3 | GAGGATTGGGCCGAAGDCTTCGATATG | 39 |
| | | FR4 | TGGGGCCAGGGCACAACCGTGACCGTGTCCTCT | 40 |
| | CH | CH1 | GCTAGCACCAAGGGACCCAGCGTGTTCCCTCTGGCTCCTTCCAGCAAGTCTACCTC TGGCGGAACAGCTGCTCTGGGCTGCCTGGTCAAGGATTACTTCCCTGAGCCTGTGA CAGTGTCCTGGAACTCTGGCGCTCTGACATCCGGCGTGCACACCTTTCCAGCTGTG CTGCAATCCTCCGGCCTGTACTCTCTGTCCTCCGTCGTGACAGTGCCTTCCAGCTC TCTGGGAACCCAGACCTACATCTGCAATGTGAACCACAAGCCTTCCAACACCAAGG TGGACAAGAAGGTG | 41 |
| | | **Hinge** | GAACCCAAGTCCTGCGACAAGACCCACACCTGTCCATGTCCT | 42 |
| | | **CH2 (LALAPG)** | GCTCCAGAAGCCGCCGGCGGACCTTCCGTGTTCCTGTTTCCTCCAAAGCCTAAGGA CACCCTGATGATCTCTCGGACCCCTGAAGTGACCTGCGTGGTGGTGGATGTGTCTC ACGAGGATCCCGAAGTGAAGTTCAATTGGTACGTGGACGGCGTGGAAGTGCACAAC GCCAAGACCAAGCCTAGAGAGGAACAGTACAACTCCACCTACAGAGTGGTGTCCGT GCTGACCGTGCTGCACCAGGATTGGCTGAACGGCAAAGAGTACAAGTGCAAGGTGT CCAACAAGGCCCTGGGCGCTCCTATCGAAAAGACCATCTCCAAGGCCAAG | 55 |
| | | CH3 | GGCCAGCCTAGAGAACCCCAGGTTTACACCTTGCCTCCATCTCGGGACGAGCTGAC CAAGAACCAGGTGTCCCTGACCTGTCTCGTGAAGGGCTTCTACCCCTCCGACATCG CCGTGGAATGGGAGTCTAATGGCCAGCCTGAGAACAACTACAAGACAACCCCTCCT GTGCTGGACTCCGACGGCTCATTCTTCCTGTACTCCAAGCTGACAGTGGACAAGTC CAGATGGCAGCAGGGCAACGTTTTCTCCTGCTCCGTGATGCACGAGGCCCTGCACA ATCACTACACCCAGAAGTCCCTGTCTCTGAGCCCCGGCAAA | 44 |
| | VL | FR1 | GACATCGTGATGACCCAGTCTCCACTGAGCCTGCCTGTGACACCTGGCGAGCCTGC TTCCATCTCCTGC | 45 |
| | | CDR1 | AGATCCTCTCAGTCCCTGCTGCACTCCAACGGCTACAACTACCTGGAC | 46 |
| | | FR2 | TGGTATCTGCAGAAGCCCGGCCAGTCTCCTCAGCTGCTGATCTAC | 47 |
| | | CDR2 | CTGGGCTCCAACAGAGCTTCT | 48 |
| | | FR3 | GGCGTGCCCGATAGATTCTCCGGCTCTGGCTCTGGCACCGACTTCACCCTGAAGAT CTCCAGAGTGGAAGCCGAGGACGTGGGCGTGTACTACTGT | 49 |
| | | CDR3 | ATGCAGGCCCTGCAGACTATCACC | 50 |
| | | FR4 | TTCGGCCAGGGAACCAGACTGGAAATCAAG | 51 |
| | CL | CL Kappa | CGTACGGTGGCCGCTCCTTCCGTGTTCATCTTCCCACCTTCCGACGAGCAGCTTAA GTCCGGCACAGCTTCTGTCGTGTGCCTGCTGAACAACTTCTACCCTCGGGAAGCCA AGGTGCAGTGGAAGGTGGACAATGCCCTGCAGTCCGGCAACTCCCAAGAGTCTGTG ACCGAGCAGGACTCCAAGGACAGCACCTACAGCCTGTCCTCCACACTGACCCTGTC CAAGGCCGACTACGAGAAGCACAAGGTGTACGCCTGCGAAGTGACCCATCAGGGCC TGTCTAGCCCTGTGACCAAGTCTTTCAACCGGGGCGAGTGC | 52 |
| **G8 linker** | | | ggtggaggcggtGGCGGAGGTGGA | 56 |
| VEGFR | | | tctgataccggcagacccttcgtggaaatgtacagcgagatccccgagatcatcca catgaccgagggcagagagctggtcatcccctgcagagtgacctctcctaacatca ccgtgactctgaagaagttccctctggacacactgatccccgacggcaagagaatc atctgggactcccggaagggcttcatcatctccaacgccacctacaaagagatcgg cctgctgacctgcgaggccaccgttaatggccacctgtacaagaccaactatctga cccacagacagaccaacaccatcatcgacgtggtgctgagcccctctcatggcatc gagctgtccgtgggagaaaagctggtgctgaactgcaccgccagaaccgagctgaa cgtgggcatcgacttcaactgggagtacccctccagcaagcaccagcacaagaagc tggtcaaccgggacctgaaaacccagtccggctccgagatgaagaaattcctgagc accctgaccatcgacggcgtgaccagatctgaccagggcctgTACacctgcgccgc ttcttctggcctgatgaccaagaaaaactccaccttcgtgcgggtgcacgagaag | 54 |

**[Table 11]**

| **Amino acid sequence information of Candidate Antibody C** | | | | |
|---|---|---|---|---|
| **Region** | | | **Amino acid sequence** | **SEQ ID NO** |
| c-Kit | VH | FB1 | QVQLVESGGGVVQPGRSLRLSCAASGFTFS | 1 |
| | | CDR1 | RYGMH | 2 |
| | | FR2 | WYRQAPGKGLEWVA | 3 |
| | | CDR2 | VIWYDGTNEDYTDSVRG | 4 |
| | | FR3 | RFTISRDNSKNTLYLQMNSLRAEDTAVYYCAR | 5 |
| | | CDR3 | EDWAEAFDN | 6 |
| | | FR4 | WGQGTTVTVSS | 7 |
| | CH | CH1 | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKV | 8 |
| | | **Hinge** | EPKSCDETHTCPPCP | 9 |
| | | **CH2 (LALE)** | APEAEGGPSVFLFPPKPKDTLMISRTPEVTCVVVDVSHE DPEVKFNWYVDGVEVHNAKTKPREEQYNSTYRVVSVLTV LHQDWLNGKEYKCKVSNKALPAPIEKTISKAK | 24 |
| | | CH3 | GQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 11 |
| | VL | FR1 | DIVMTQSPLSLPVTPGEPASISC | 12 |
| | | CDR1 | RSSQSLLHSNGYNYLD | 13 |
| | | FR2 | WYLQEPGQSPQLLIY | 14 |
| | | CDR2 | LGSNRAS | 15 |
| | | FB3 | GVPDRFSGSGTDFTLKISHVEAEDVGVYYC | 16 |
| | | CDR3 | MQALQTIT | 17 |
| | | FR4 | FGQGTRLEIK | 18 |
| | CL | CL Kappa | | 19 |
| **G8 linker** | | | GGGGGGGG | 23 |
| VEGFR | | | SDTGRPFVEMYSEIPEIIHMTEGRELVIPCRVTSPNITV TLKKFPLDTLIPDGKRIIWDSRKGFIISNATYKEIGLLT CEATVNGHLYKTNYLTHRQTNTIIDVVLSPSHGIELSVG EKLVLNCTARTELNVGIDFNWEYPSSKHQHKKLVNRDLK TQSGSEMKKFLSTLTIDGVTRSDQGLYTCAASSGLMTKK NSTFVRVHEK | 21 |

**[Table 12]**

| **Nucleotide sequence information of Candidate Antibody C** | | | | |
|---|---|---|---|---|
| **Region** | | | **Nucleotide sequence** | **SEQ ID NO** |
| c-Kit | VH | FR1 | CAGGTGCAGCTGGTGGAATCTGGTGGCGGAGTTGTGCAGCCTGGCAGATC CCTGAGACTGTCTTGTGCCGCCTCCGGCTTCACCTTCTCC | 34 |
| | | CDR1 | AGATACGGAATGCAC | 35 |
| | | FR2 | TGGGTCCGACAGGCCCCTGGCAAAGGATTGGAATGGGTCGCC | 36 |
| | | CDR2 | GTGATTTGGTACGACGGCACCAACAAGGACTACACCGACTCTGTGCGGGG C | 37 |
| | | FR3 | AGATTCACCATCTCTCGGGACAACTCCAAGAACACCCTGTACCTGCAGAT GAACTCCCTGAGAGCCGAGGACACCGCCGTGTACTACTGTGCCAGA | 38 |
| | | CDR3 | GAGGATTGGGCCGAAGCCTICGATATG | 39 |
| | | FR4 | TGGSGCCAGSGCACAACCGTGACCGISTCCTCT | 40 |
| | CH | CH1 | GCTAGCACCAAGGGACCCAGCGTGTTCCCTCTGGCTCCTTCCAGCAAGTC TACCTCTGGCGGAACAGCTGCTCTGGGCTGCCTGGTCAAGGATTACTTCC CTGAGCCTGTGACAGTGTCCTGGAACTCTGGCGCTCTGACATCCGGCGTG CACACCTTTCCAGCTGTGCTGCAATCCTCCGGCCTGTACTCTCTGTCCTC CGTCGTGACAGTGCCTTCCAGCTCTCTGGGAACCCAGACCTACATCTGCA ATGTGAACCACAAGCCTTCCAACACCAAGGTGGACAAGAAGGTG | 41 |
| | | **Hinge** | GAACCCAAGTCCTGCGACAAGACCCACACCTGTCCACCATGTCCT | 42 |
| | | **CH2 (LALE)** | GCTCCAGAAGCCGAAGGCGGACCTTCCGTGTTCCTGTTTCCTCCAAAGCC TAAGGACACCCTGATGATCTCTCGGACCCCTGAAGTGACCTGCGTGGTGG TGGATGTGTCTCACGAGGATCCCGAAGTGAAGTTCAATTGGTACGTGGAC GGCGTGGAAGTGCACAACGCCAAGACCAAGCCTAGAGAGGAACAGTACAA CTCCACCTACAGAGTGGTGTCCGTGCTGACCGTGCTGCACCAGGATTGGC TGAACGGCAAAGAGTACAAGTGCAAGGTGTCCAACAAGGCCCTGCCTGCT CCTATCGAAAAGACCATCTCCAAGGCCAAG | 57 |
| | | CH3 | GGCCAGCCTAGAGAACCCCAGGTTTACACCTTGCCTCCATCTCGGGACGA GCTGACCAAGAACCAGGTGTCCCTGACCTGTCTCGTGAAGGGCTTCTACC CCTCCGACATCGCCGTGGAATGGGAGTCTAATGGCCAGCCTGAGAACAAC TACAAGACAACCCCTCCTGTGCTGGACTCCGACGGCTCATTCTTCCTGTA CTCCAAGCTGACAGTGGACAAGTCCAGATGGCAGCAGGGCAACGTTTTCT CCTGCTCCGTGATGCACGAGGCCCTGCACAATCACTACACCCAGAAGTCC CTGTCTCTGAGCCCCGGCAAA | 44 |
| | VL | FR1 | GACATCGTGATGACCCAGTCTCCACTGAGCCTGCCTGTGACACCTGGCGA GCCTGCTTCCATCTCCTGC | 45 |
| | | CDR1 | AGATCCTCTCAGTCCCTRCTRCACTCCACGGCTACAACTACCTGGAC | 46 |
| | | FR2 | TGGTATCTGCAGAAGCCCGGCCAGTCTCCTCAGCTGCGATCTAC | 47 |
| | | CDR2 | CTGGGCTCCAACAGAGCTTCT | 48 |
| | | FR3 | GGCGTGCCCGATAGATTCTCCGGCTCTGGCTCTGGCACCGACTTCACCCT GAAGATCTCCAGAGTGGAAGCCGAGGACGTGGGCGTGTACTACTGT | 49 |
| | | CDR3 | ATGCAGGCCCTGCAGACCATCACC | 50 |
| | | FR4 | TTCGGCCAGGGAACCADACTGGAAATCAAG | 51 |
| | CL | CL Kappa | CGTACGGTGGCCGCTCCTTCCGTGTTCATCTTCCCACCTTCCGACGAGCA GCTTAAGTCCGGCACAGCTTCTGTCGTGTGCCTGCTGAACAACTTCTACC CTCGGGAAGCCAAGGTGCAGTGGAAGGTGGACAATGCCCTGCAGTCCGGC AACTCCCAAGAGTCTGTGACCGAGCAGGACTCCAAGGACAGCACCTACAG CCTGTCCTCCACACTGACCCTGTCCAAGGCCGACTACGAGAAGCACAAGG TGTACGCCTGCGAAGTGACCCATCAGGGCCTGTCTAGCCCTGTGACCAAG TCTTTCAACCGGGGCGAGTGC | 52 |
| **G8 linker** | | | ggtggaggcggtGGCGGAGGTGGA | 56 |
| VEGFR | | | tctgataccggcagaccccttcgtggaaatgtacagcgagatccccgagat catccacatgaccgagggcagagagctggtcatcccctgcagagtgacct ctcctaacatcaccgtgactctgaagaagttccctctggacacactgatc cccgacggcaagagaatcatctgggactcccggaagggcttcatcatctc caacgccacctacaaagagatcggcctgctgacctgcgaggccaccgtta atggccacctgtacaagaccaactatctgacccacagacagaccaacacc atcatcgacgtggtgctgagccccctctcatggcatcgagctgtccgtggg agaaaagctggtgctgaactgcaccgccagaaccgagctgaacgtgggca tcgacttcaactgggagtacccctccagcaagcaccagcacaagaagctg gtcaaccgggacctgaaaacccagtccggctccgagatgaagaaattcct gagcaccctgaccatcgacggcgtgaccagatctgaccagggcctgTACa cctgcgccgcttcttctggcctgatgaccaagaaaaactccaccttcgtg cgggtgcacgagaag | 54 |

### 1-3. Additional introduction of I253A, H310A and H435A mutations

A bispecific antibody was produced by additionally introducing I253A, H310A and H435A mutations into Candidate Antibody B above, and this was named 'Candidate Antibody B-1'. The binding affinity of Candidate Antibody B-1 to human FcRn (neonatal Fc receptor) was evaluated by SPR analysis. As confirmed in Table 13, no significant interaction between antibody B-1 and human FcRn was observed at pH 6.0, and no KD value was measured at pH 7.4.

**[Table 13]**

| **Ligand** | **pH** | Analyte | **KD (M)** | **Rmax (RU)** |
|---|---|---|---|---|
| Human FCGRT & B2M heterodimeric protein | 6.0 | Antibody B | 8.66E-07 | 89.9 |
| | | Antibody B-1 | N.D* | |
| | | Anti-c-Kit antibody | 1.99E-06 | 46.1 |
| | 7.4 | Antibody B | N.D* | |
| | | Antibody B-1 | N.D* | |
| | | Anti-c-Kit antibody | N.D* | |

| | | | | |
|---|---|---|---|---|
| N.D.*: Not measured in low response units (RU). | | | | |

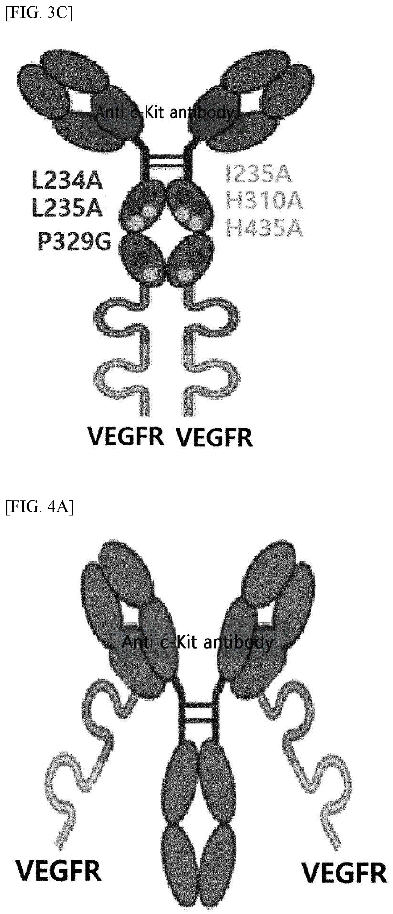

Since the absence of binding to FcRn results in a short half-life in serum, the results of this example suggest that additional introduction of the I253A, H310A and H435A mutations is expected to reduce systemic side effects induced by simultaneously targeting c-Kit and VEGF in the blood. The structure and sequence information of candidate antibody B-1 are shown in FIG. 3C and Tables 14 to 15, respectively.

**[Table 14]**

| **Amino acid sequence information of Candidate Antibody B-1** | | | | |
|---|---|---|---|---|
| **Region** | | | **Amino acid sequence** | **SEQ ID NO** |
| c-Kit | VH | FR1 | QVQLVESGGGVVQPGRSLRLSCAASGFTFS | 1 |
| | | CDR1 | RYGMH | 2 |
| | | FR2 | WVRQAPGKGLEWVA | 3 |
| | | CDR2 | VIWYDGTNKDYTDSVRG | 4 |
| | | FR3 | RFTISRDNSKNTLYLQMNSLRAEDTAVYYCAR | 5 |
| | | CDR3 | EIWAEAFIM | 6 |
| | | FR4 | WGQGTTVTYSS | 7 |
| | CH | CH1 | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKV | 8 |
| | | **Hinge** | EPKSCDKTHTCPPCP | 9 |
| | | **CH2 (LALAPG-AA)** | APEAAGGPSVFLFPPKPKDTLMI SRTPEVTCVVVDVSHE DPEVKFNWYVDGVEVHNAKTKPREEQYNSTYRVVSVLTV LHQDWLNGKEYKCKVSNKALGAPI EKTI SKAK | 25 |
| | | **CH3 (A)** | GQPREPQVYTLPPSRDELTKNQVSLTCLVKGFYPSDIAV EWESNGQPENNYKTTPPVLDSDGSFFLYSKLTVDKSRWQ QGNVFSCSVMHEALHNAYTQKSLSLSPGK | 26 |
| | VL | FR1 | DIVMTQSPLSLPVTPGEPASISC | 12 |
| | | CDR1 | RSSQSLLHSNGYNYLD | 13 |
| | | FR2 | WYLQKPGQSPQLLIY | 14 |
| | | CDR2 | LGSNRAS | 15 |
| | | FR3 | GVPDRFSGSGSGTDFTLKISRVEAELIVGVYYC | 16 |
| | | CDR3 | MQALQTIT | 17 |
| | | FR4 | FGQTRLEIK | 18 |
| | CL | CL Kappa | | 19 |
| **G8 linker** | | | GGGGGGGG | 23 |
| VEGFR | | | SDTGRPFVEMYSEIPEIIHMTEGRELVIPCRVTSPNITV TLKKFPLDTLIPDGKRIIWDSRKGFIISNATYKEIGLLT CEATVNGHLYKTNYLTHRQTNTIIDVVLSPSHGIELSVG EKLVLNCTARTELNVGIDFNWEYPSSKHQHKKLVNRDLK TQSGSEMKKFLSTLTIDGVTRSDQGLYTCAASSGLMTKK NSTFVRVHEK | 21 |

**[Table 15]**

| **Nucleotide sequence information of Candidate Antibody B-1** | | | | |
|---|---|---|---|---|
| **Region** | | | **Nucleotide sequence** | **SEQ ID NO** |
| c-Kit | VII | FR1 | CAGGTGCAGCTGGTGGAATCTGGTGGCGGAGTTGTGCAGCCTGGCAGATC CCTGAGACTGTCTTGTGCCGCCTCCGGCTTCACCTTCTCC | 34 |
| | | CDR1 | AGATICGGAATGCAC | 35 |
| | | FR2 | TGGGTCCGACAGGCCCCTGGCAAAGGATTGGAATGGGTCGCC | 36 |
| | | CDR2 | GTGATTTGGTACGACGGCACCAACAAGGACTACACCGACTCTGTGCGGGG C | 37 |
| | | FR3 | AGATTCACCATCTCTCGGGACAACTCCAAGAACACCCTGTACCTGCAGAT GAACTCCCTGAGAGCCGAGGACACCGCCGTGTACTACTGTGCCAGA | 38 |
| | | CDR3 | GAGGATTGGGCCGAAGCCTTCGATATG | 39 |
| | | FR4 | TGGGGCCAGGGCACAACCGIGACCGTGTCCTCT | 40 |
| | CH | CH1 | GCTAGCACCAAGGGACCCAGCGTGTTCCCTCTGGCTCCTTCCAGCAAGTC TACCTCTGGCGGAACAGCTGCTCTGGGCTGCCTGGTCAAGGATTACTTCC CTGAGCCTGTGACAGTGTCCTGGAACTCTGGCGCTCTGACATCCGGCGTG CACACCTTTCCAGCTGTGCTGCAATCCTCCGGCCTGTACTCTCTGTCCTC CGTCGTGACAGTGCCTTCCAGCTCTCTGGGAACCCAGACCTACATCTGCA ATGTGAACCACAAGCCTTCCAACACCAAGGTGGACAAGAAGGTG | 41 |
| | | **Hinge** | GAACCCAASTCCTGCSACAAGACCCACACCTGTCCACCATGTCCT | 42 |
| | | **CH2 (LALAPG-AA)** | GCTCCAGAAGCCGCCGGCGGACCTTCCGTGTTCCTGTTTCCTCCAAAGCC TAAGGACACCCTGATGGCTTCTCGGACCCCTGAAGTGACCTGCGTGGTGG TGGATGTGTCTCACGAGGATCCCGAAGTGAAGTTCAATTGGTACGTGGAC GGCGTGGAAGTGCACAACGCCAAGACCAAGCCTAGAGAGGAACAGTACAA CTCCACCTACAGAGTGGTGTCCGTGCTGACCGTGCTGGCCCAGGATTGGC TGAACGGCAAAGAGTACAAGTGCAAGGTGTCCAACAAGGCCCTGGGCGCT CCTATCGAAAAGACCATCTCCAAGGCCAAG | 58 |
| | | **CH3 (A)** | GGCCAGCCTAGAGAACCCCAGGTTTACACCTTGCCTCCATCTCGGGACGA GCTGACCAAGAACCAGGTGTCCCTGACCTGTCTCGTGAAGGGCTTCTACC CCTCCGACATCGCCGTGGAATGGGAGTCTAATGGCCAGCCTGAGAACAAC TACAAGACAACCCCTCCTGTGCTGGACTCCGACGGCTCATTCTTCCTGTA CTCCAAGCTGACAGTGGACAAGTCCAGATGGCAGCAGGGCAACGTTTTCT CCTGCTCCGTGATGCACGAGGCCCTGCACAATGCTTACACCCAGAAGTCC CTGTCTCTGAGCCCCGGCAAA | 59 |
| | VL | FR1 | GACATCGTGATGACCCAGTCTCCACTGAGCCTGCCTGTGACACCTGGCGA GCCTGCTTCCATCTCCTGC | 45 |
| | | CDR1 | AGATCCTCTCAGTCCCTGCTRCACTCCAACGGCTACAACTACCTGGAC | 46 |
| | | FR2 | TGGTATCTGCAGAAGCCCGGCCAGTCTCCTCAGCTGCTGATCTAC | 47 |
| | | CDR2 | CTGGEGCTCCARCAGAGCTTCT | 48 |
| | | FR3 | GGCGTGCCCGATAGATTCTCCGGCTCTGGCTCTGGCACCGACTTCACCCT GAAGATCTCCAGAGTGGAAGCCGAGGACGTGGGCGTGTACTACTGT | 49 |
| | | CDR3 | ATGCAGGCCCTGCAGACCATCACC | 50 |
| | | FR4 | TTCGGCCAGGGAACCAGACTGGAAATCAAG | 51 |
| | CL | CL Kappa | CGTACGGTGGCCGCTCCTTCCGTGTTCATCTTCCCACCTTCCGACGAGCA GCTTAAGTCCGGCACAGCTTCTGTCGTGTGCCTGCTGAACAACTTCTACC CTCGGGAAGCCAAGGTGCAGTGGAAGGTGGACAATGCCCTGCAGTCCGGC AACTCCCAAGAGTCTGTGACCGAGCAGGACTCCAAGGACAGCACCTACAG CCTGTCCTCCACACTGACCCTGTCCAAGGCCGACTACGAGAAGCACAAGG TGTACGCCTGCGAAGTGACCCATCAGGGCCTGTCTAGCCCTGTGACCAAG TCTTTCAACCGGGGCGAGTGC | 52 |
| **G8 linker** | | | ggtggaggcggtGGCGGAGGTGGA | 56 |
| VEGFR | | | tctgataccggcagacccttcgtggaaatgtacagcgagatccccgagat catccacatgaccgagggcagagagctggtcatccccctgcagagtgacct ctcctaacatcaccgtgactctgaagaagttccctctggacacactgatc cccgacggcaagagaatcatctgggactcccggaagggcttcatcatctc caacgccacctacaaagagatcggcctgctgacctgcgaggccaccgtta atggccacctgtacaagaccaactatctgacccacagacagaccaacacc atcatcgacgtggtgctgagccccctctcatggcatcgagctgtccgtggg agagaaagctggtgctgaactgcaccgccagaaccgagctgaacgtgggca tcgacttcaactgggagtacccctccagcaagcaccagcacaagaagctg gtcaaccgggacctgaaaacccagtccggctccgagatgaagaaattcct gagcaccctgaccatcgacggcgtgaccagatctgaccagggcctgTACa cctgcgccgcttcttctggcctgatgaccaagaaaaactccaccttcgtg cgggtgcacgagaag | 54 |

### [Example 2]

### Production of various types of bispecific antibodies according to the binding site of VEGFR

In order to produce a bispecific antibody in which VEGFR is linked to the light chain C-terminus of c-Kit antibody, cloning was performed to link the light chain of the antibody, linker and VEGFR sequences by using gene synthesis (commissioned by Bioneer) based on the vector expressing the c-Kit antibody produced in Production Example 1-1. Thereafter, cloning and expression of the antibody were performed in the same manner as in Production Example 1-2, and a purification process was performed. The structure of the obtained antibody is shown in FIG. 4A, and it was named 'Candidate Antibody D.' The sequence information of Candidate Antibody D is identical to that of Candidate Antibody A.

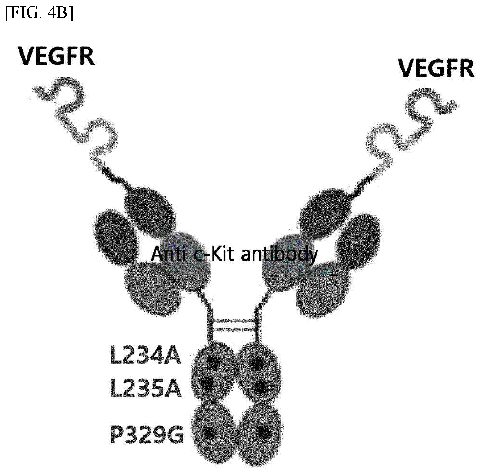

Next, in order to produce a bispecific antibody in which VEGFR is linked to the heavy chain N-terminus of c-Kit antibody via a G8 peptide linker and a LALAPG mutation is introduced into Fc, cloning was performed by using gene synthesis (commissioned by Bioneer) based on the vector expressing the c-Kit antibody produced in Production Example 1-1 to link the heavy chain of the antibody including the mutation, the linker and the VEGFR sequence. Thereafter, the cloning and expression of the antibody were performed in the same manner as in Production Example 1-2, and the purification process was performed. The structure of the obtained antibody is shown in FIG. 4B, and it was named 'Candidate Antibody E.' The sequence information of Candidate Antibody E is the same as that of Candidate Antibody B.

Information on the 6 candidate antibodies produced through the above results is shown in Table 16.

**[Table 16]**

| | **Candidate Antibody A** | **Candidate Antibody B** | **Candidate Antibody B-1** | **Candidate Antibody C** | **Candidate Antibody D** | **Candidate Antibody E** |
|---|---|---|---|---|---|---|
| Molecular form of bispecific antibodies | IgG(H)-VEGFR | IgG(H)-VEGFR | IgG(H)-VEGFR | IgG(H)-VEGFR | VEGFR-IgG(L) | VEGFR-IgG(H) |
| Binding value | 2+2 | 2+2 | 2+2 | 2+2 | 2+2 | 2+2 |
| Fc engineering | Wild type | LALAPG | LALAPG-AAA | LALE | Wild type | LALAPG |
| Type of linker | G5 | G8 | G8 | G8 | G5 | G8 |

### [Example 3]

### Confirmation of binding ability to c-Kit and VEGF

ELISA was performed to confirm the target binding ability of the 6 bispecific antibodies in Table 16 above. The amino acid sequence information of the c-Kit antigen (Sino Biological, 11996-H08H) and VEGF antigen (Sino Biological, 11066-HNAH) used in the experiment are as shown in SEQ ID NOs: 71 and 72, respectively.
**c-Kit antigen (P10721-2, Met1-Thr516)** 
MRGARGAWDFLCVLLLLLRVQTGSSQPSVSPGEPSPPSIHPGKSDLIVRVGDEIRLLCTDPGFVKWTFEIL
DETNENKQNEWITEKAEATNTGKYTCTNKHGLSNSIYVFVRDPAKLFLVDRSLYGKEDNDTLVRCPLTDPEVTNYSL
KGCQGKPLPKDLRFIPDPKAGIMIKSVKRAYHRLCLHCSVDQEGKSVLSEKFILKVRPAFKAVPVVSVSKASYLLRE
GEEFTVTCTIKDVSSSVYSTWKRENSQTKLQEKYNSWHHGDFNYERQATLTISSARVNDSGVFMCYANNTFGSANVT
TTLEVVDKGFINIFPMINTTVFVNDGENVDLIVEYEAFPKPEHQQWIYMNRTFTDKWEDYPKSENESNIRYVSELHL
TRLKGTEGGTYTFLVSNSDVNAAIAFNVYVNTKPEILTYDRLVNGMLQCVAAGFPEPTIDWYFCPGTEQRCSASVLP
VDVQTLNSSGPPFGKLVVQSSIDSSAFKHNGTVECKAYNDVGKTSAYFNFAFKEQIHPHT (SEQ ID NO: 71)
**VEGF antigen (P15692-4, Met1-Arg191)**

MNFLLSWVHWSLALLLYLHHAKWSQAAPMAEGGGQNHHEVVKFMDVYQRSYCHPIETLVDIFQEYPDEIEY
IFKPSCVPLMRCGGCCNDEGLECVPTEESNITMQIMRIKPHQGQHIGEMSFLQHNKCECRPKKDRARQENPCGPCSE
RRKHLFVQDPQTCKCSCKNTDSRCKARQLELNERTCRCDKPRR (SEQ ID NO: 72)

In order to coat the assay plate with c-Kit/VEGF working solution, 100 µL of working solution (0.5 µg/mL) was added per well, the plate was sealed with film, and incubated overnight at 2 to 8°C. A 5% BSA solution diluted in PBS was prepared as a blocking solution, which was filtered through a 0.22 µm filter. The coating solution was removed, and the plate was washed three times with 300 µL/well of 0.05% PBST buffer, and 300 µL of blocking solution was added per well of the assay plate. The plate was covered with a plate sealer and incubated at 25 ± 2°C for 1 hour without shaking.

Each antibody was diluted to 1 µg/mL in DPBS and prepared as a two-fold serial dilution in the dilution plate. After blocking, the solution was removed from the assay plate, and the plate was washed three times with 300 µL/well of 0.05% PBST buffer. By using a multichannel pipette, 100 µL of antibody was transferred from the dilution plate to the assay plate, the plate was sealed with a film, and incubated for 1 hour at 25 ± 2°C without shaking. After 1 hour, the plate was washed three times with 300 µL/well of 0.05% PBST buffer, and 100 µL of the diluted secondary antibody solution (80 ng/mL; final secondary antibody) was added to each well using a multichannel pipette. The plate was sealed with a film and incubated for 1 hour at 25 ± 2°C without shaking. After incubation with secondary antibodies, the solution was removed from the assay plate. The plates were washed three times with 300 µL/well of 0.05% PBST buffer, and 100 µL of TMB substrate reagent solution was added to each well using a multichannel pipette. The plates were covered with a film and incubated at 25 ± 2°C for 5 minutes. The plates were read within 10 minutes after adding 100 µL of 1 N sulfuric acid to the wells. Plates were read at absorbance at 450 nm using a SPECTROstar NaNo.

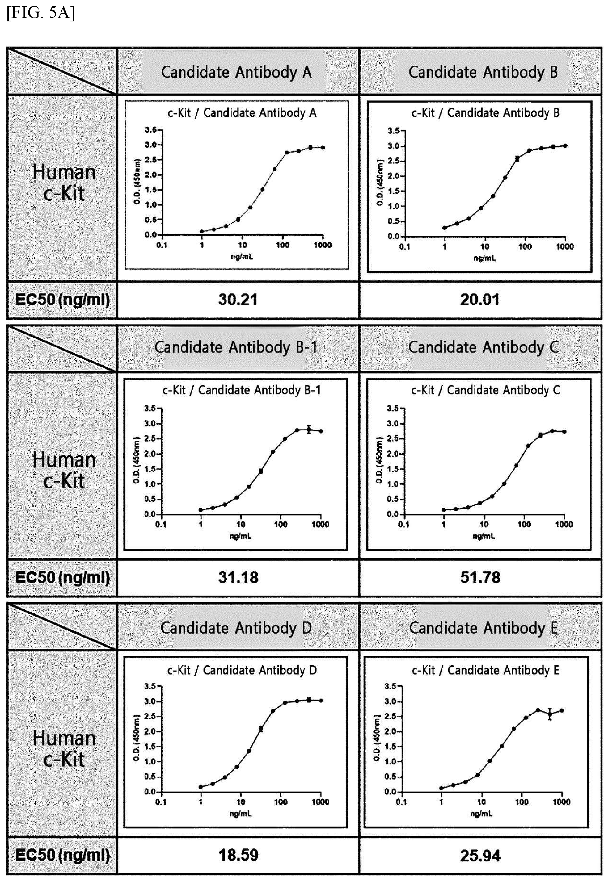

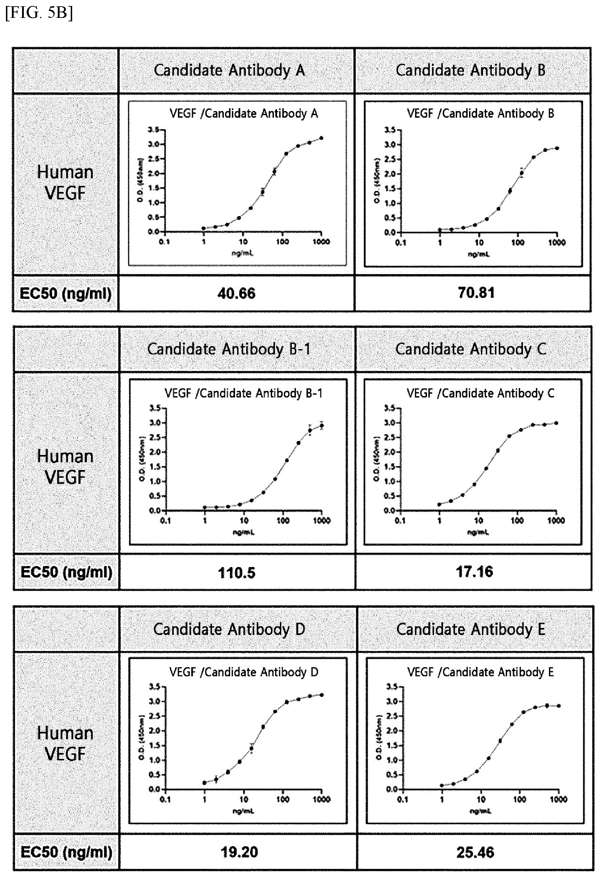

As confirmed in FIGS. 5A and 5B, all 6 antibodies bound to c-Kit and VEGF in a concentration-dependent manner, and showed excellent binding ability to c-Kit and VEGF, respectively.

### [Example 4]

### Confirmation of binding affinity for c-Kit and VEGF

In order to confirm the binding affinity of the 6 bispecific antibodies in Table 9 above to c-Kit and VEGF, SPR was performed. A running buffer was prepared by diluting 1X from 20X HBS-EP+ buffer with deionized water, and a regeneration solution (10 mM NaOH) was prepared by diluting 10 N NaOH to 10 mM with deionized water. Immobilization and binding assays were performed at 25°C by using 1X HBS-EP+ buffer as a running buffer.

For human c-Kit analysis, c-Kit protein was directly immobilized on the sensor chip surface via primary amine coupling. The sensor chip surfaces of the sample and reference flow cells were activated with freshly mixed EDC/NHS mixture for 420 seconds. Afterwards, c-Kit protein was diluted in 10 mM sodium acetate solution and injected into the sample flow cell to achieve an appropriate immobilization level, and the reference flow cell was set as blank. After the amine coupling reaction, the remaining active coupling sites were blocked with ethanolamine for 420 seconds.

For human VEGF165 analysis, the capture molecule, protein A, was directly immobilized on the sensor chip surface via primary amine coupling. The amine coupling process was the same as for c-Kit analysis, except that protein A was immobilized on both channels. Then, diluted antibodies or Eylea were injected only into the sample flow cell, and captured by pre-immobilized Protein A using affinity for the Fc region.

General settings and running configurations are shown in Tables 17 to 19.

**[Table 17]**

| General settings | |
|---|---|
| Sensor chip | Series S CM5 |
| Running buffer | HBS-EP+ |
| Flow rate | 10 L/min |

**[Table 18]**

| Running configurations in human c-Kit analysis | |
|---|---|
| Ligand | Human c-Kit |
| Attachment method | Covalent bond immobilization using amine coupling |
| Fluid cell used | Fc 2 (Fc 1 is a reference cell) |
| Immobilization solution | 10 mM sodium acetate, pH 5.5 |
| Concentration | 2 ug/mL |
| Activation | EDC/NHS mixture, for 420 seconds |
| Deactivation | Ethanolamine, for 420 seconds |

**[Table 19]**

| Running configurations in human VEGF165 analysis | | |
|---|---|---|
| Type | Capture molecule | Ligand |
| Name | Protein A | Antibody |
| Attachment method | Covalent bond immobilization using amine coupling | Capture using affinity for Protein A |
| Fluid cell used | Fc 1, 2 | Fc 2 |
| Immobilization solution | 10 mM sodium acetate, pH 4.5 | N/A |
| Concentration | 30 ug/mL | c-Kit antibody: 4 nM |
| | | Eylea 6 nM |
| Activation | EDC/NHS mixture, for 420 seconds | N/A |
| Deactivation | Ethanolamine, for 420 seconds | N/A |

Binding assays were performed at a flow rate of 30 uL/min. All analytes were serially diluted in running buffer, and all samples were prepared on ice. Analyte samples were injected during the binding step, followed by running buffer for separation. The sensor chip surface was regenerated at each cycle by injecting a regeneration solution. For the c-Kit assay, 10 mM NaOH solution was used as the regeneration solution, and for the VEGF assay, 10 mM glycine, pH 1.5 solution was used. The analyte concentrations and running configurations are shown in Tables 20 and 21.

**[Table 20]**

| Concentration of analytes | | |
|---|---|---|
| Ligand | Analyte | Concentration range of analytes |
| Human c-Kit | Antibody | 0.078, 0.156, 0.313, 0.625, 1.25, 2.5, 5, 10 nM |
| Human VEGF165 | Antibody | 0.020, 0.039, 0.078, 0.156, 0.313, 0.625, 1.25, 2.5, 5, 10 nM |

**[Table 21]**

| Running configurations | |
|---|---|
| Type of assay | Multicycle reaction rates |
| Start cycle | 2 runs, using running buffer |
| Blank cycle | 2 cycles per analyte, after concentration series |
| Binding time | c-Kit analysis: 240 seconds, VEGF165 analysis: 120 seconds |
| Dissociation time | c-Kit analysis: 480 seconds, VEGF165 analysis: 240 seconds |
| Flow rate | 30 uL/min |

Evaluations were performed by using Biacore Insight Evaluation Software. The 1:1 binding model was used to globally fit the sensorgram curves and calculate the kinetic parameters (association rate constant [ka] and dissociation rate constant [kd]). The KD value (equilibrium dissociation constant) was calculated as the ratio of Kd to k.

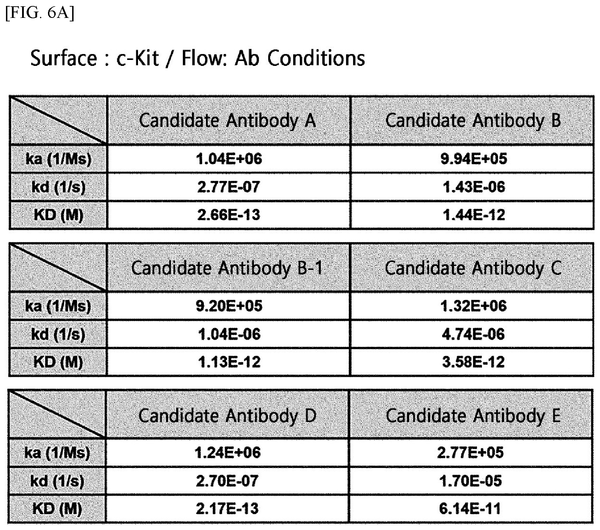

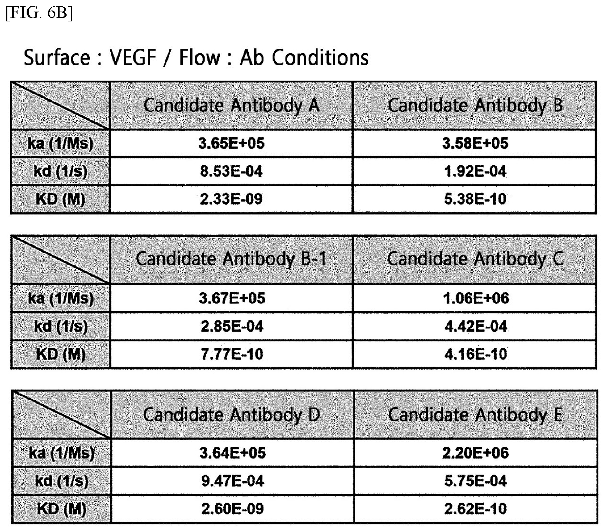

As confirmed in FIGS. 6A and 6B, all 6 antibodies showed excellent binding affinity to c-Kit and VEGF.

### [Example 5]

### Stability check

### 5-1. Thermal stability

In order to evaluate the thermal stability of the 6 bispecific antibodies in Table 16 above, DSF analysis was performed. PBS (1X) buffer to be used as a negative control was prepared by diluting PBS (20X) buffer, and the stock solution of each test sample was diluted to a concentration of 1 mg/mL using PBS (1X) buffer such that the total amount of test sample for each reaction was 5 ug. Protein Thermal Shift^{™} Dye (1000X) was freshly diluted to 8X by using PBS (1X) buffer. The reaction components described in Table 22 below were added to each well of the MicroAmp^{®} Optical 8-tube strip.

**[Table 22]**

| Reaction components | |
|---|---|
| **Component** | **Volume** |
| Protein Thermal Shift^{™} Buffer | 5.0 uL |
| Diluted Protein Thermal Shift^{™} Dye (8X) | 2.5 uL |
| Diluted test sample (1 mg/mL) | 5.0 uL |
| PBS (1X) | 7.5 uL |
| **Total volume for each reaction** | 20 uL |

The tubes were sealed with MicroAmp^{®} Optical 8-Cap Strips. Samples were mixed by using a vortex mixer and then centrifuged. Each reaction was prepared in quadruplicate. Fluorescence readings were monitored by using a real-time PCR instrument, StepOnePlus^{®} Real-Time PCR System, and control software. Protein melting reactions were performed by setting the experimental characteristics as described in Table 23 below.

**[Table 23]**

| Experimental characteristics | | |
|---|---|---|
| **Field** | | **Item** |
| Experimental characteristics | Type of experiment | Melting curve |
| | Type of reagent | Others |
| | Ramp rate | Fast |
| Target characteristics | Reporter | ROX |
| | Quencher | None |
| Plate layout | Manual reference | None |
| Performance method | Reaction volume per well | 20 uL |
| | Ramp mode | Continuous |
| | Heat profile | Step 1. Temperature: 25.0°C, Time: 2 min |
| | | Step 2. Temperature: 99.0°C, Time: 2 min |
| | Ramp rate | Step 1. 100% |
| | | Step 2. 1% |

Melting temperature (Tm) analysis was performed by using Protein Thermal Shift^{™} software. The analysis mode was set as shown below and in Table 24.

**[Table 24]**

| Analysis mode | |
|---|---|
| **Field** | Item |
| Analysis method | Boltzmann fitting |
| ROA (analysis area) | Automatic |
| Automatic analysis option | Single Tm |

Results were statistically analyzed through one-way ANOVA and Tukey's multiple comparisons using GraphPad Prism 10 software.

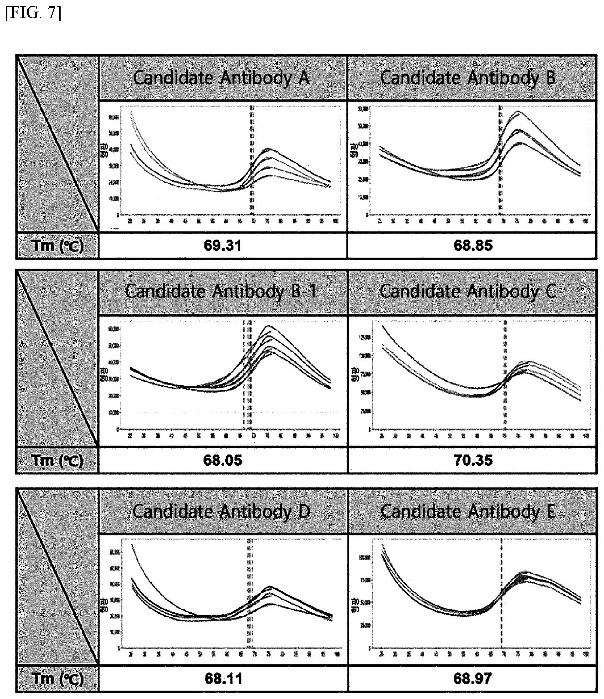

Each of the 6 antibodies was exposed to temperature changes from 25°C to 99°C, and the fluorescence signals generated when the protein unfolds were calculated and shown in FIG. 7. As confirmed in FIG. 7, all of the 6 antibodies produced exhibited excellent thermal stability.

### 5-2. Protein stability after repeated freeze/thaw cycles

The 6 bispecific antibodies in Table 16 above were frozen at -80°C for 16 hours and then thawed at 4°C for 2 hours. This was performed as 1 cycle, and the cycle was repeated 5 times. Afterwards, the antibody stock solution was diluted to 1 mg/mL using the formulation buffer, and SEC-HPLC analysis was performed under the conditions of Table 25, and the results are shown in Tables 26 to 31.

**[Table 25]**

| **Parameter** | Conditions | |
|---|---|---|
| **Column** | TOSOH TSKgel Super SW3000, 4 µm, 4.6 mm x 30 cm | |
| **Temperatures of column and autosampler** | 30°C and 4°C | |
| **Mobile phase** | 50 mM potassium phosphate buffer, 150 mM potassium chloride; pH 6.8 | |
| **Isosolvent method** | **Hour** | **% mobile phase** |
| | 0 | 100 |
| **Flow rate** | 0.35 mL/min | |
| **Injection volume** | 10 µL | |
| **Wavelength** | 280 nm/Bw: 4 nm Ref 360 nm/ Bw: 100 nm284 nm/Bw: 4 nm Ref 360 nm/ Bw: 100 nm | |

**[Table 26]**

| Candidate Antibody A | | | |
|---|---|---|---|
| **Cycle 1** | **RT** | **Area (%)** | |
| | 7.176 | 95.69 | Monomer |
| | 7.767 | 4.18 | LMW |
| | 9.620 | 0.13 | |
| **Cycle 2** | **RT** | **Area (%)** | |

| | 7.174 | 96.02 | Monomer |
|---|---|---|---|
| | 7.733 | 3.86 | LMW |
| | 9.632 | 0.12 | |

| **Cycle 3** | **RT** | **Area (%)** | |
|---|---|---|---|
| | 7.173 | 95.45 | Monomer |
| | 7.733 | 4.42 | LMW |
| | 9.621 | 0.13 | |

| **Cycle 4** | **RT** | **% area** | |
|---|---|---|---|
| | 7.170 | 95.60 | Monomer |
| | 7.817 | 4.27 | LMW |
| | 9.623 | 0.13 | |

**[Table 27]**

| Candidate Antibody B | | | |
|---|---|---|---|
| **Cycle 1** | **RT(min)** | **Area (%)** | |
| | 6.583 | 1.41 | HMW |
| | 7.128 | 97.55 | Monomer |
| | 8.926 | 0.76 | LMW |
| | 9.549 | 0.28 | |

| **Cycle 2** | **RT(min)** | **Area (%)** | |
|---|---|---|---|
| | 6.550 | 1.35 | HMW |
| | 7.110 | 97.71 | Monomer |
| | 8.926 | 0.69 | LMW |
| | 9.540 | 0.25 | |

| **Cycle 3** | **RT(min)** | **Area (%)** | |
|---|---|---|---|
| | 6.533 | 1.37 | HMW |
| | 7.118 | 97.70 | Monomer |
| | 8.941 | 0.69 | LMW |
| | 9.552 | 0.25 | |

| **Cycle 4** | **RT(min)** | **Area (%)** | |
|---|---|---|---|
| | 6.517 | 1.27 | HMW |
| | 7.113 | 97.82 | Monomer |
| | 8.945 | 0.67 | LMW |
| | 9.549 | 0.25 | |

**[Table 28]**

| Candidate Antibody B-1 | | | |
|---|---|---|---|
| **Cycle 1** | **RT(min)** | **Area (%)** | |
| | 6.497 | 0.20 | HMW |
| | 7.224 | 99.80 | Monomer |

| **Cycle 2** | **RT(min)** | **Area (%)** | |
|---|---|---|---|
| | 6.488 | 0.25 | HMW |
| | 7.199 | 99.75 | Monomer |

| **Cycle 3** | **RT(min)** | **Area (%)** | |
|---|---|---|---|
| | 6.493 | 0.27 | HMW |
| | 7.205 | 99.73 | Monomer |

| **Cycle 4** | **RT(min)** | **Area (%)** | |
|---|---|---|---|
| | 6.479 | 0.25 | HMW |
| | 7.202 | 99.75 | Monomer |

**[Table 29]**

| Candidate Antibody C | | | | | | |
|---|---|---|---|---|---|---|
| | | | | **Monomer** | **HMW** | **LMW** |
| **Cycle** | **RT** | **Area (%)** | **RT** | **Area (%)** | **RT** | **Area (%)** |
| 0 | 7.206 | 99.84 | 6.557 | 0.16 | N.D | N.D |
| 1 | 6.949 | 99.86 | 6.309 | 0.14 | N.D | N.D |
| 3 | 6.946 | 99.83 | 6.296 | 0.17 | N.D | N.D |
| 5 | 6.951 | 99.83 | 6.290 | 0.17 | N.D | N.D |

**[Table 30]**

| Candidate Antibody D | | | |
|---|---|---|---|
| **Cycle 0** | **RT(min)** | 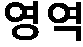 **(%)** | |
| | 7.235 | 100 | Monomer |

| **Cycle 1** | **RT(min)** | **Area(%)** | |
|---|---|---|---|
| | 6.878 | 100 | Monomer |

| **Cycle 3** | **RT(min)** | **Area(%)** | |
|---|---|---|---|
| | 6.890 | 100 | Monomer |

| **Cycle 5** | **RT(min)** | **Area(%)** | |
|---|---|---|---|
| | 6.880 | 100 | Monomer |

**[Table 31]**

| Candidate Antibody E | | | |
|---|---|---|---|
| **Cycle 0** | **RT(min)** | **Area(%)** | |
| | 7.849 | 1.44 | HMW |
| | 8.555 | 98.56 | Monomer |

| **Cycle 1** | **RT(min)** | **Area(%)** | |
|---|---|---|---|
| | 7.350 | 1.73 | HMW |
| | 7.948 | 98.27 | Monomer |

| **Cycle 3** | **RT(min)** | **Area(%)** | |
|---|---|---|---|
| | 7.350 | 1.79 | HMW |
| | 7.941 | 98.21 | Monomer |

| **Cycle 5** | **RT(min)** | **Area(%)** | |
|---|---|---|---|
| | 7.350 | 1.63 | HMW |
| | 7.943 | 98.37 | Monomer |

All 5 antibodies, except Candidate Antibody A, were confirmed to be relatively stable even under rapid temperature changes, with monomers remaining greater than 97.5% even after repeated freeze/thaw cycles.

### [Example 6]

### In vitro confirmation of efficacy of bispecific antibodies

### 6-1. Vascular permeability

Human umbilical vein endothelial cells (HUVEC; Lonza_C2519A) cultured in EBM medium including 0.5% FBS were pretreated with the indicated concentrations of each constructed bispecific antibody for 2 hours. The cells were additionally exposed to 100 mM CoCl₂ (hypoxia) or 100 ng/mL LPS overnight. Endothelial permeability was determined by measuring the passage of FITC-dextran on HUVEC monolayers. In order to measure fluorescein isothiocyanate-dextran permeability, excitation was performed at 480 nm, and fluorescence was detected at 520 nm.

The vascular permeability of Candidate Antibodies B and C was compared with that of Eylea alone, anti-c-Kit antibody alone or faricimab-treated groups, and the vascular permeability of Candidate Antibodies C and E was compared with that of anti-c-Kit antibody alone or comparator antibody-treated groups. The comparative antibody is a 2(1+1) antibody in the form of Fab-VEGFR-Fc(K-H) that links anti-c-Kit antibody IgG (knob) and Eylea (VEGFR trap (hole)) using knob-into-hole technology. The dexamethasone-treated group was used as a positive control.

Statistical significance was determined by using the unpaired t test [***P<0.001, ****P<0.0001 vs. vehicle; #P<0.05, ###P<0.001, and ####P<0.0001 vs. 1% O₂ or LPS; ns, not significant. (n=3)].

As confirmed in FIG. 8A, Candidate Antibody B showed a significantly superior vascular permeability inhibition effect compared to the combination treatment group of Eylea and anti-c-Kit antibodies and the faricimab treatment group, and as confirmed in FIG. 8B, Candidate Antibodies C and E showed a significantly superior vascular permeability inhibition effect compared to the comparative antibody. In addition, as confirmed in FIG. 8C, Candidate Antibody C showed a significantly superior vascular permeability inhibition effect compared to the combination treatment group of Eylea and anti-c-Kit antibodies, and showed a vascular permeability inhibition effect similar to the faricimab treatment group.

In summary, Candidate Antibodies B, C and E were observed to significantly inhibit the effect on paracellular permeability of HUVECs *in vitro*, suggesting that the bispecific candidate antibodies according to the present invention inhibit hypoxia- or LPS-induced venous endothelial barrier disruption.

### 6-2. Blocking signal transmission

Human Retinal Microvascular Endothelial Cells (HRMEC; Cell Systems_ ACBRI-181) or human mast cell line LAD2 (obtained from Dr. Kirshenbaum; National Institute of Health) were pretreated with each bispecific antibody for 30 minutes, and after treatment was completed, the cells were stimulated with 100 ng/mL SCF for 5 minutes. Western blot was performed as follows to analyze the expression levels of cell signaling-related proteins in whole cell lysates.

Cells were lysed with RIPA buffer including protease and phosphatase inhibitor cocktail. Protein was quantified by using the BCA protein assay kit. Western blot samples including appropriate amounts of protein, NuPAGE LDS sample buffer containing reducing agent and RIPA buffer were prepared and boiled at 70°C for 10 minutes. 30 µg of protein was loaded onto each well of an 8% SDS-PAGE gel. Gel electrophoresis was performed at 60 V for 30 minutes until it passed through the separating gel. The voltage was then increased to 120 V for 1 hour. Transfer was performed at 320 mA for 120 minutes [Running buffer: 1X Tris-glycine buffer with SDS, Transfer buffer: 1X Tris-glycine buffer (without SDS) including 20% methanol]. Membranes were blocked with SuperBlock blocking buffer in TBST for 1 hour. Membranes were incubated overnight at 4°C with the following primary antibodies: pho-cKit (1:1,000), pho-AKT (1:1,000), pho-Erk (1:3000), cKit (1:1,000), AKT (1:1,000), Erk (1:3,000) and β-actin (1:10,000). Each of the primary antibodies was diluted in SuperBlock blocking buffer dissolved in TBST. The membranes were washed with TBST for 10 minutes, and it was repeated three times. The membranes were incubated for 1 hour at room temperature with the following secondary antibodies: mouse-anti-goat IgG-HRP (1:5,000), mouse anti-rabbit IgG-HRP (1:5,000) and goat anti-mouse IgG-HRP (1:5,000). The above secondary antibodies were used by diluting each with 5% skim milk dissolved in TBST. The membrane was washed with TBST for 10 minutes, and it was repeated three times. The membrane was detected by using ECL buffer.

The signal transduction inhibitory effects of Candidate Antibodies B and B-1 were compared with the anti-c-Kit antibody treatment group, and the signal transduction inhibitory effects of Candidate Antibodies C and E were compared with the comparative antibody treatment group. The comparative antibody was the same as that used in Example 6-1.

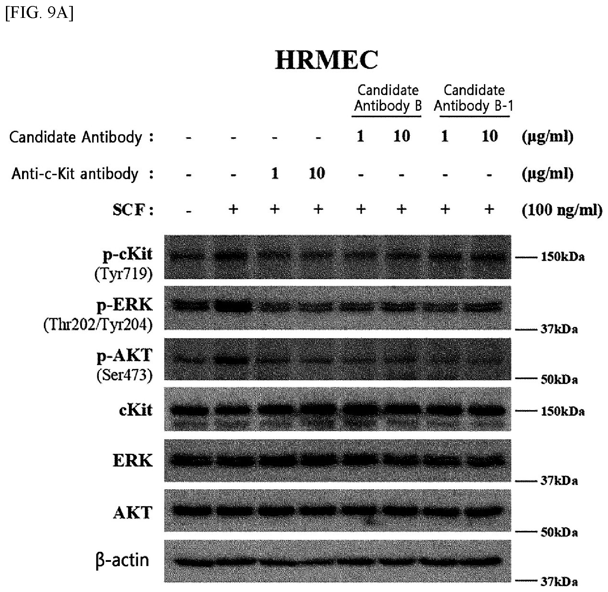

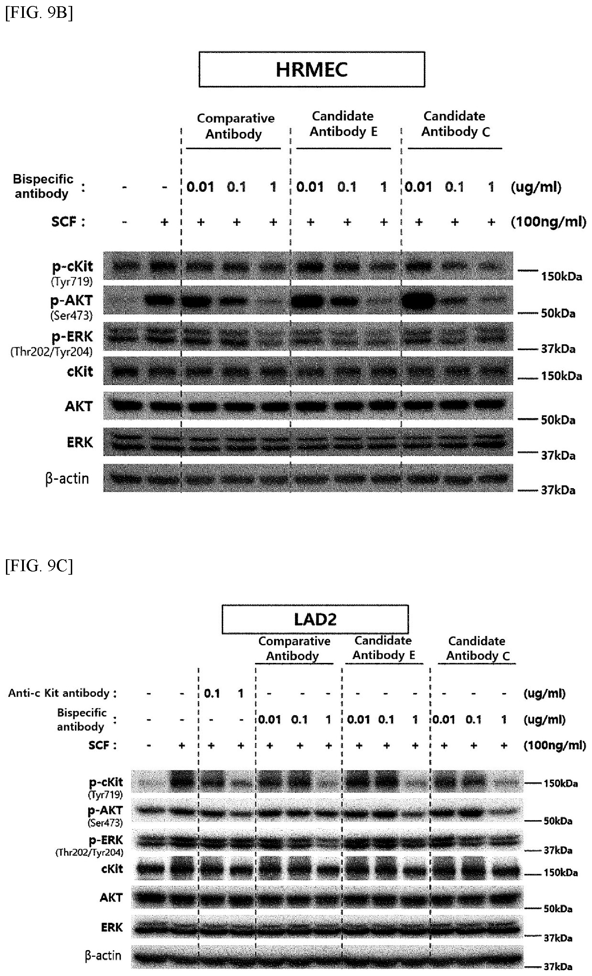

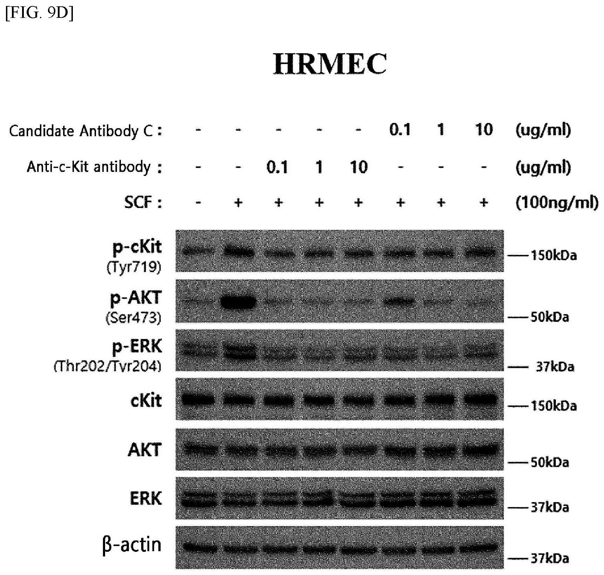

As confirmed in FIGS. 9A to 9D, Candidate Antibodies B, B-1, C and E were observed to effectively inhibit SCF-dependent c-Kit signaling in HRMEC or LAD2.

### 6-3. Analysis of cell cytokines

HRMEC or ARPE-19 (ATCC: CRL-2302) cells cultured in medium including 0.5% FBS were pretreated with each bispecific antibody for 6 hours. The cells were additionally exposed to 1% O₂ hypoxia or LPS for 12 hours. Cell supernatants were harvested and quantitative ELISA for VEGF, Ang-2, IL-6, IL-8 and MCP-1 was performed.

The cytokine inhibitory effect of Candidate Antibody A was compared with the Eylea treatment group, the cytokine inhibitory effects of candidate antibodies B and C were compared with the Eylea treatment group, anti-c-Kit antibody treatment group and faricimab treatment group (dexamethasone treatment group was used as a positive control group), and the cytokine inhibitory effects of Candidate Antibodies C and E were compared with the comparative antibody treatment group. The comparative antibody is the same as that used in Example 6-1.

Statistical significance was determined by using an unpaired t test (***P<0.001, ****P<0.0001 vs. vehicle; #P<0.05, ##P<0.005, ###P<0.001, and ####P<0.0001 vs. 1% O₂ or LPS; ns, not significant).

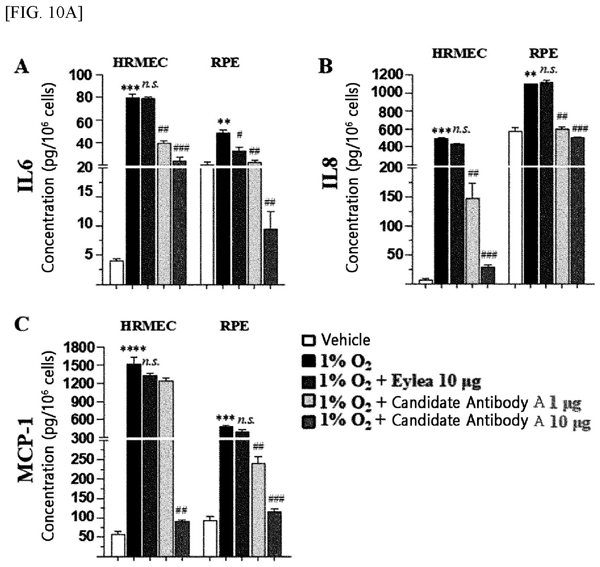

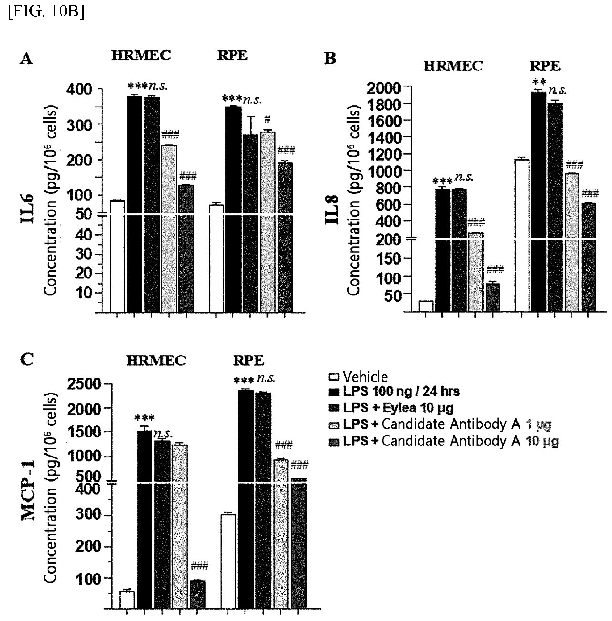

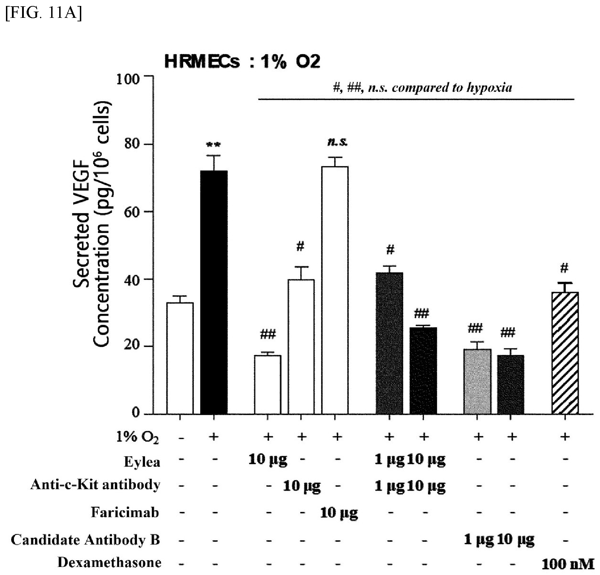

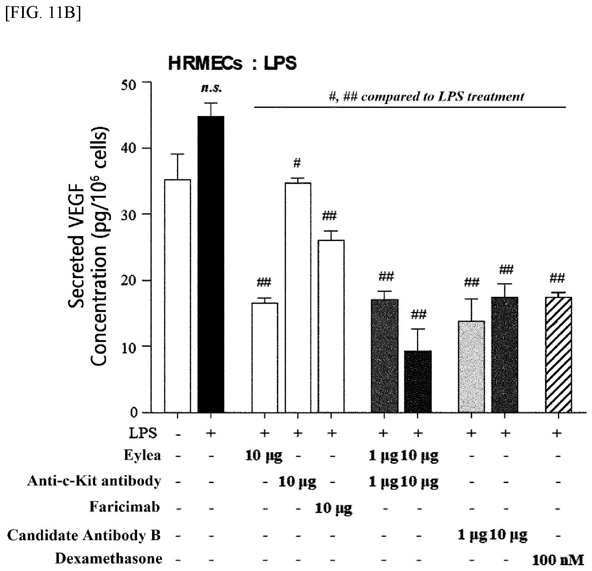

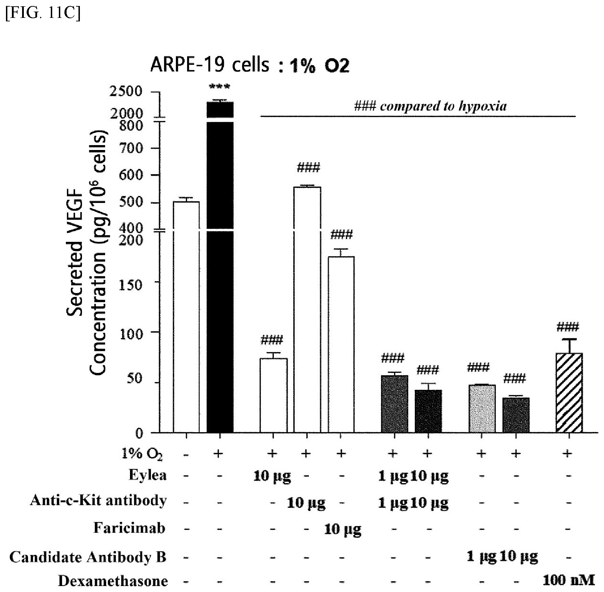

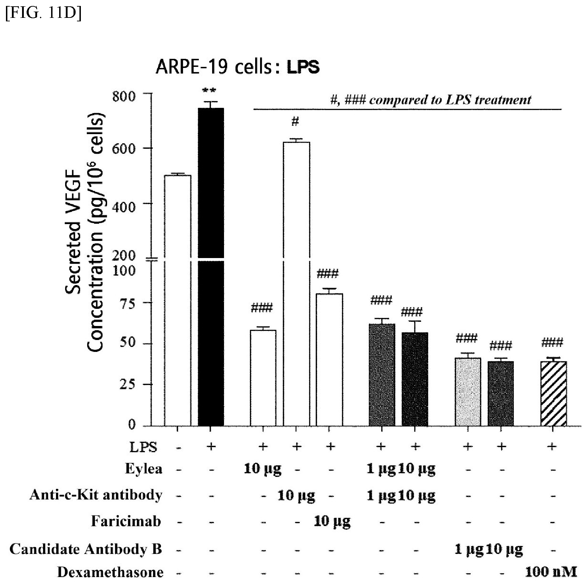

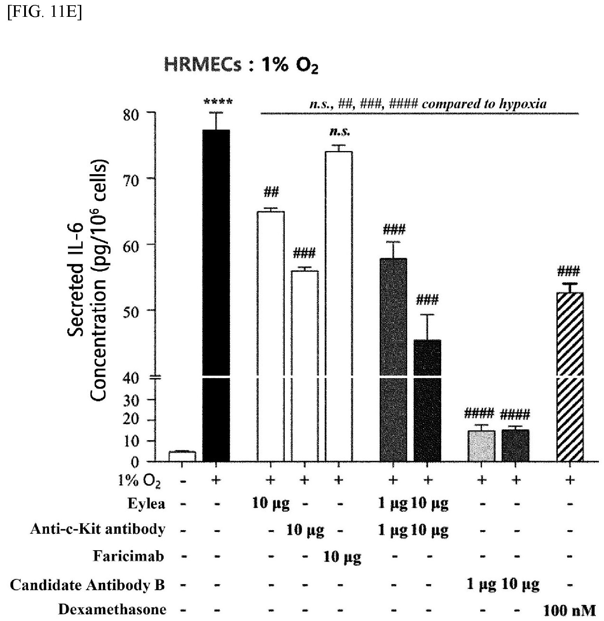

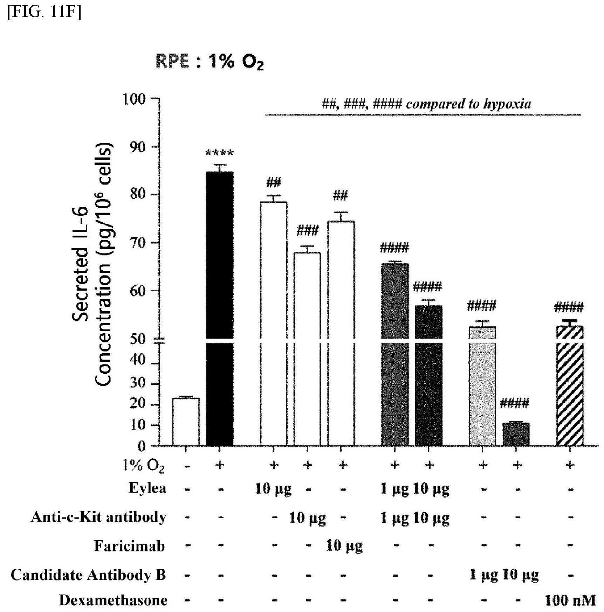

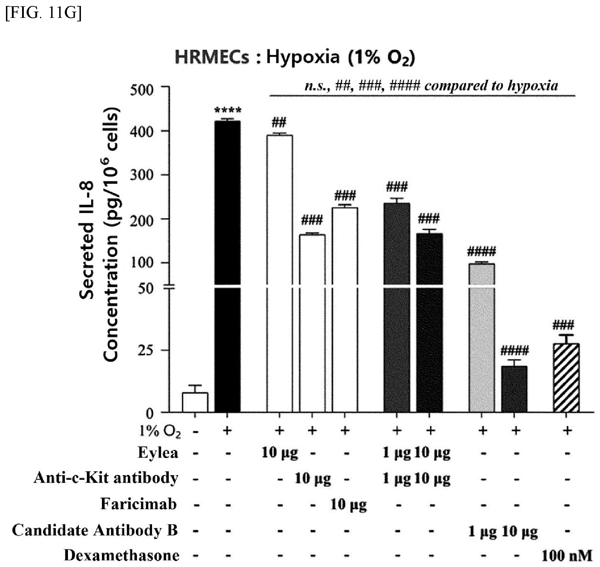

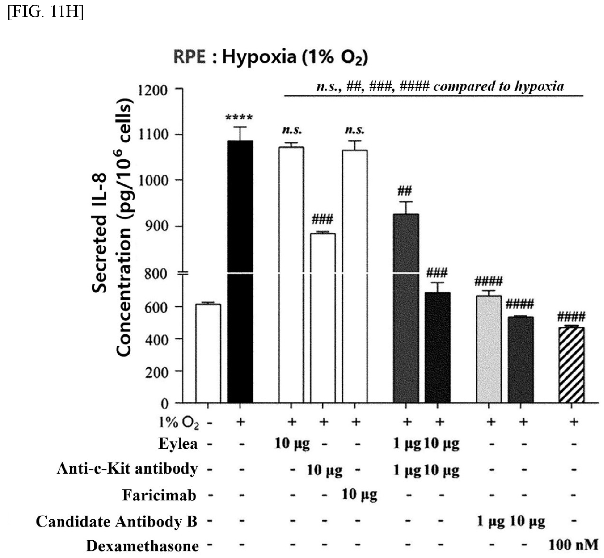

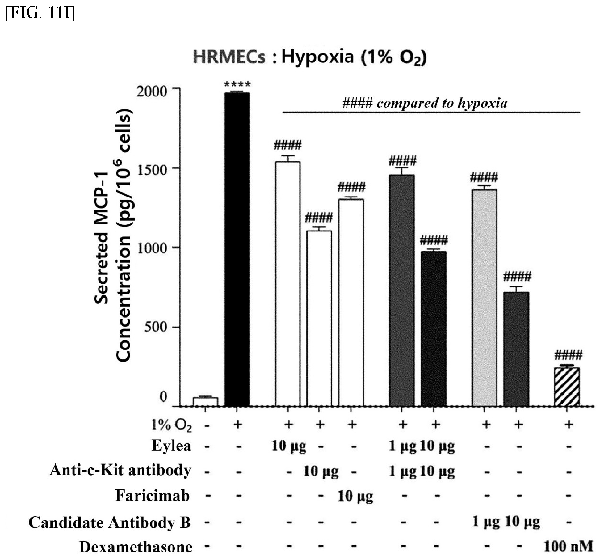

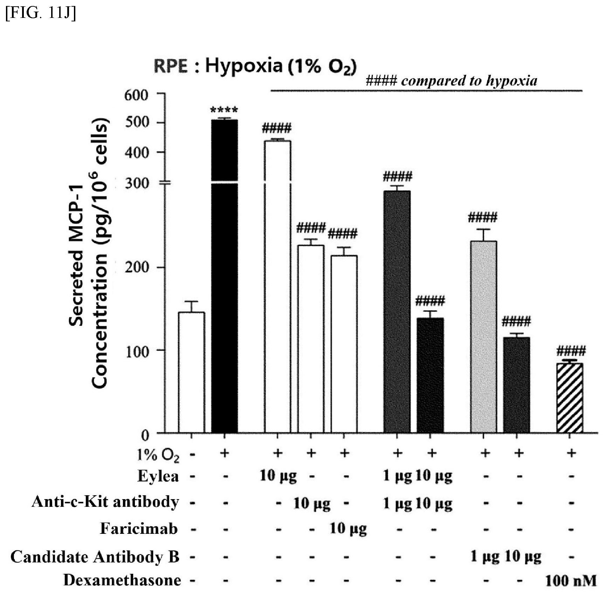

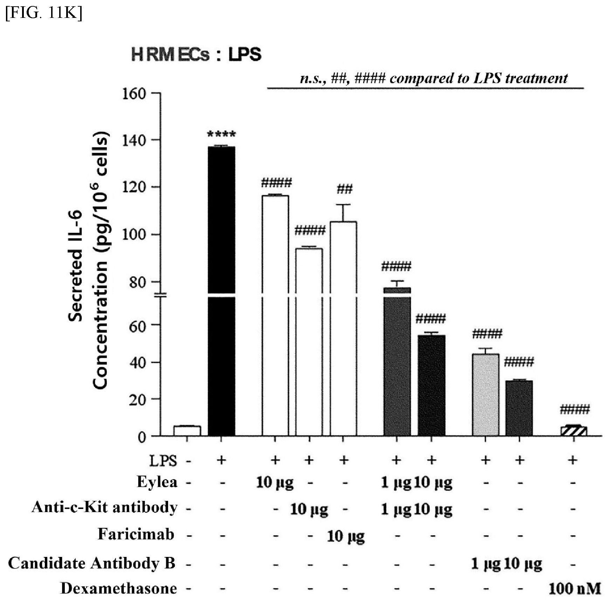

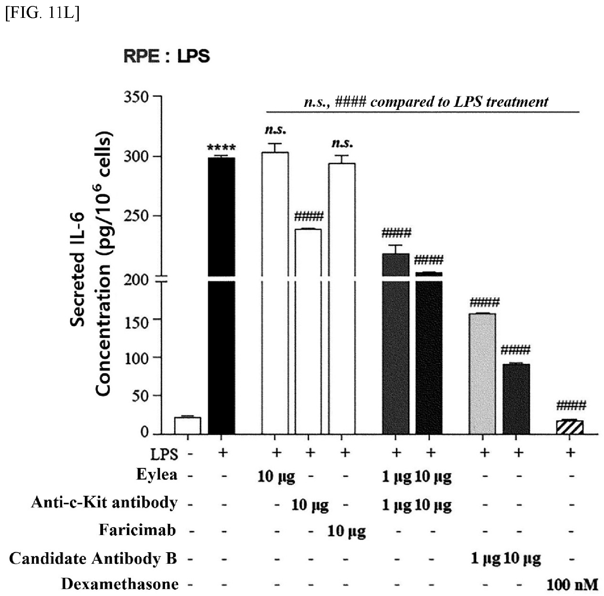

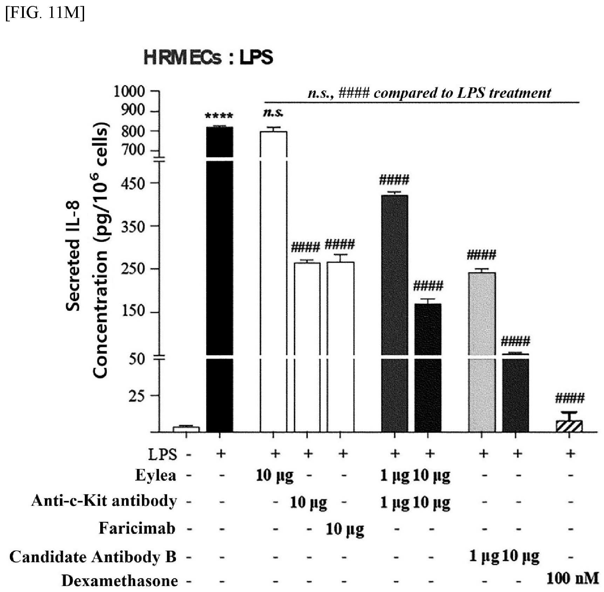

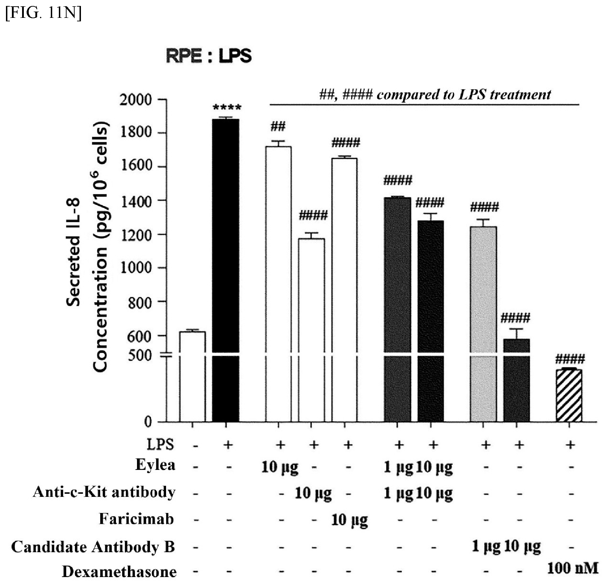

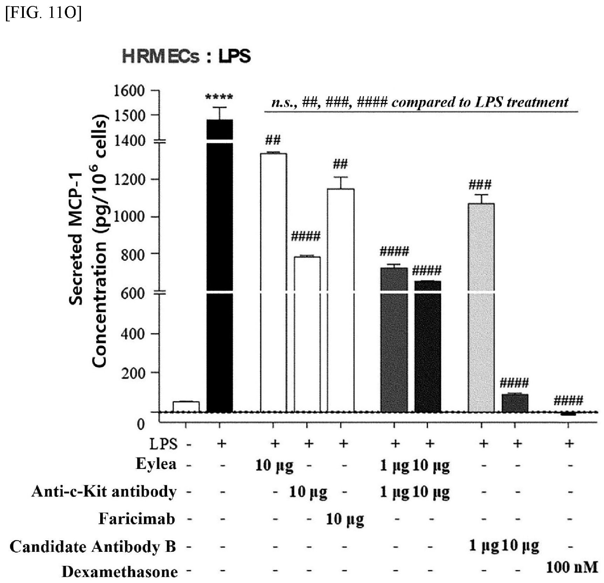

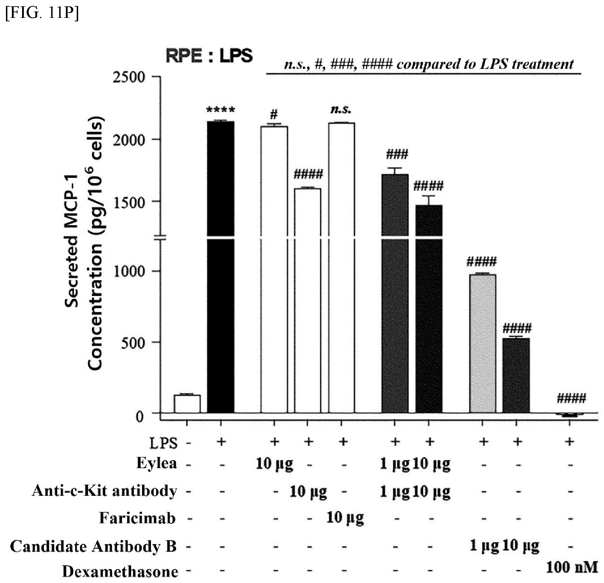

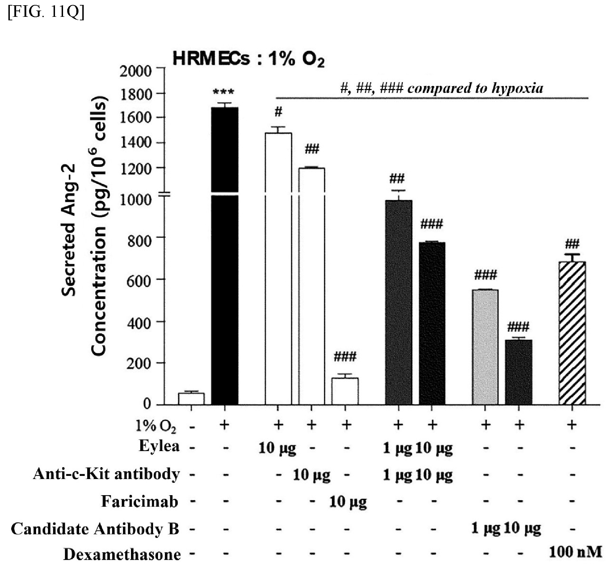

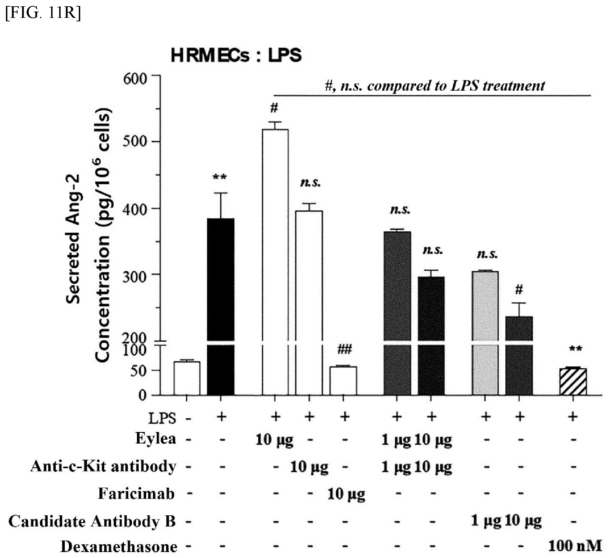

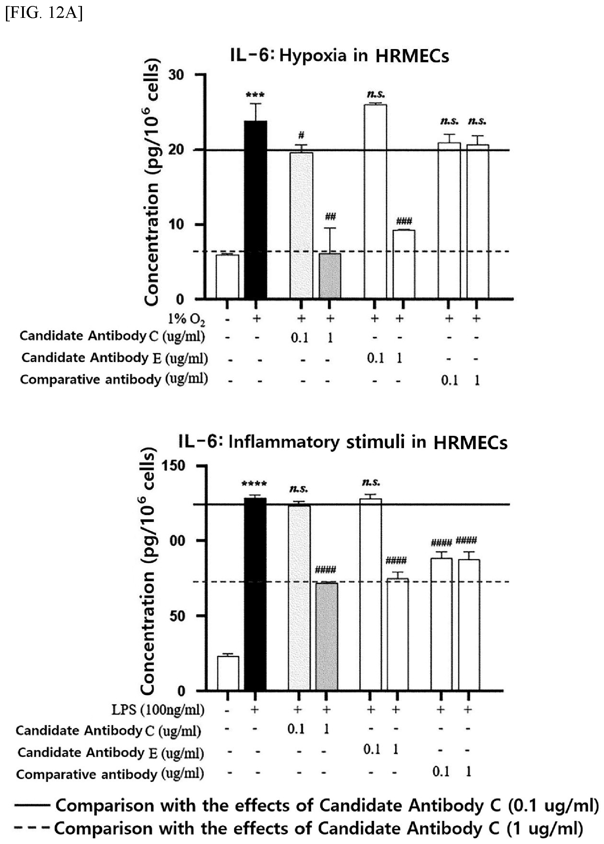

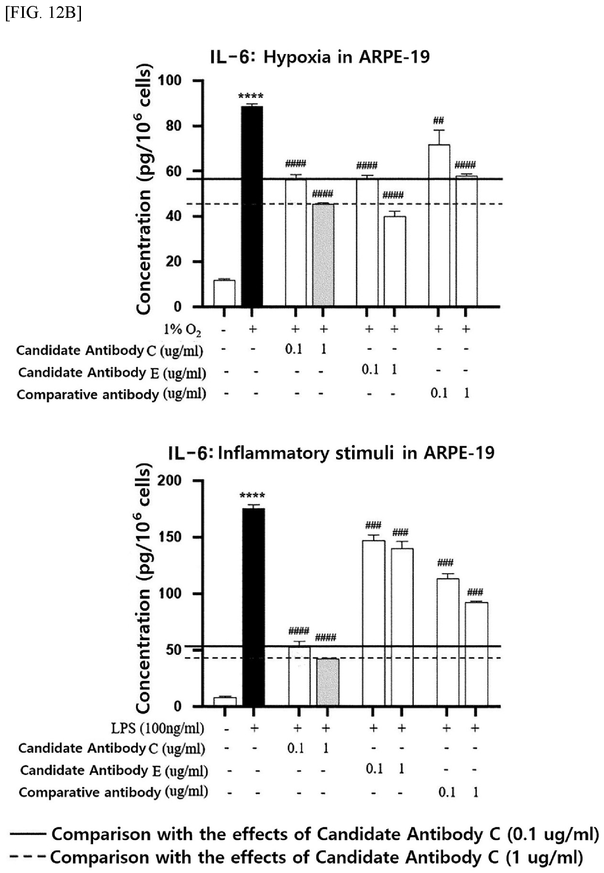

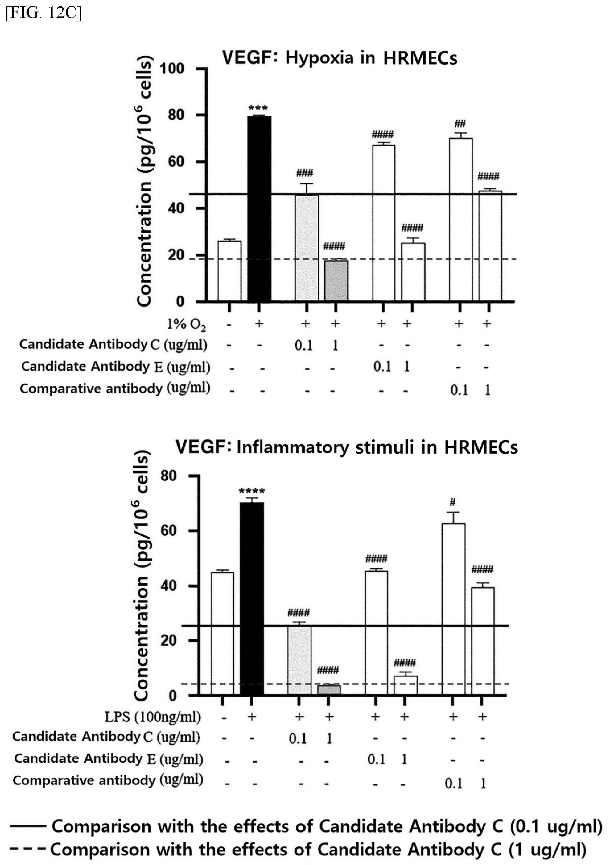

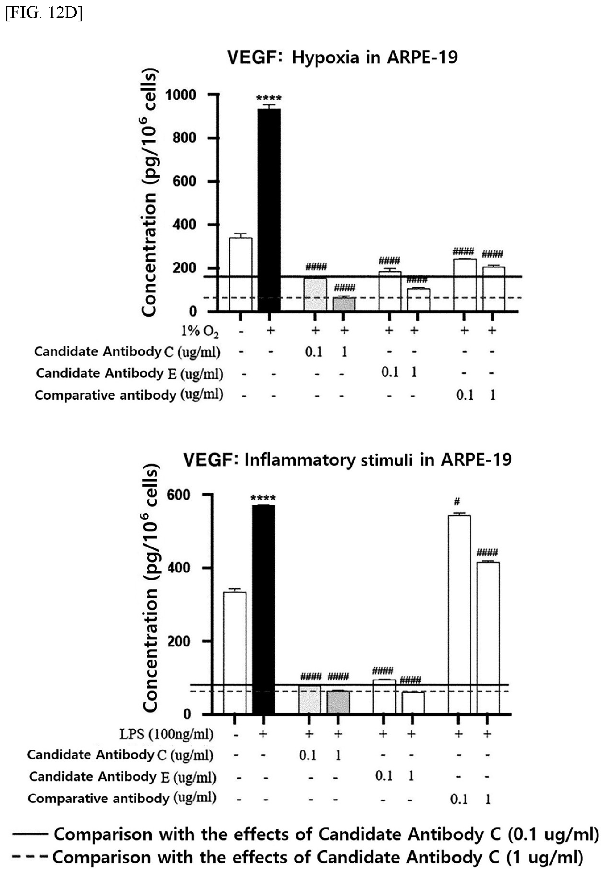

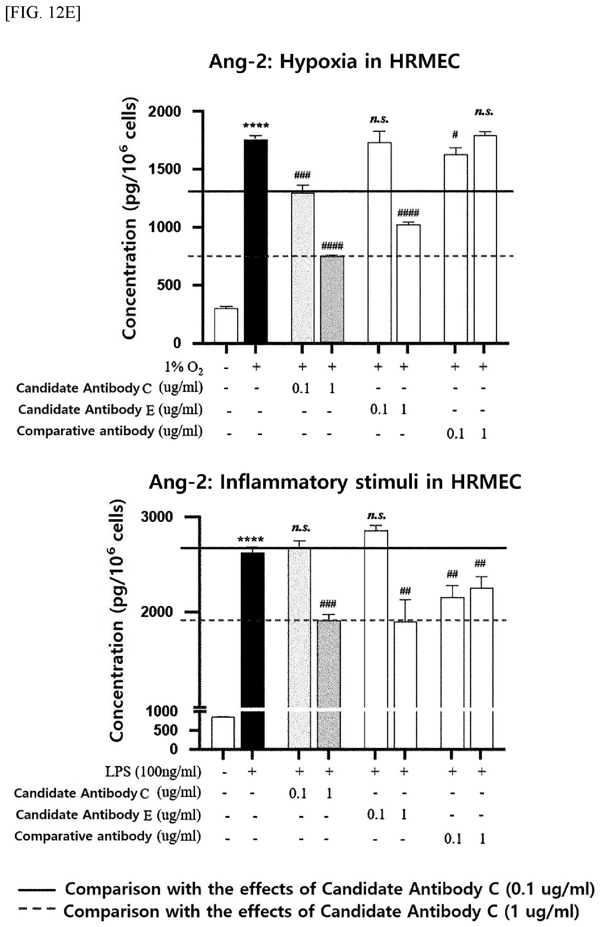

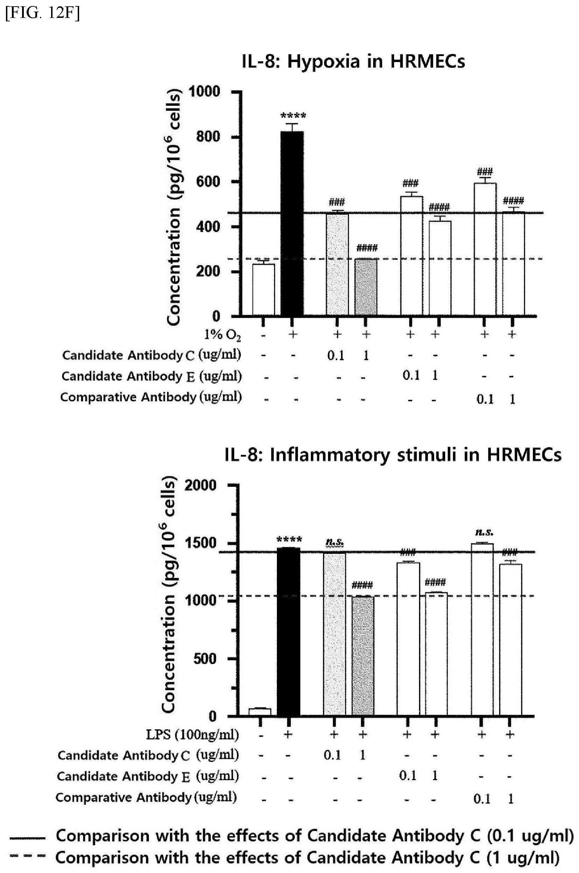

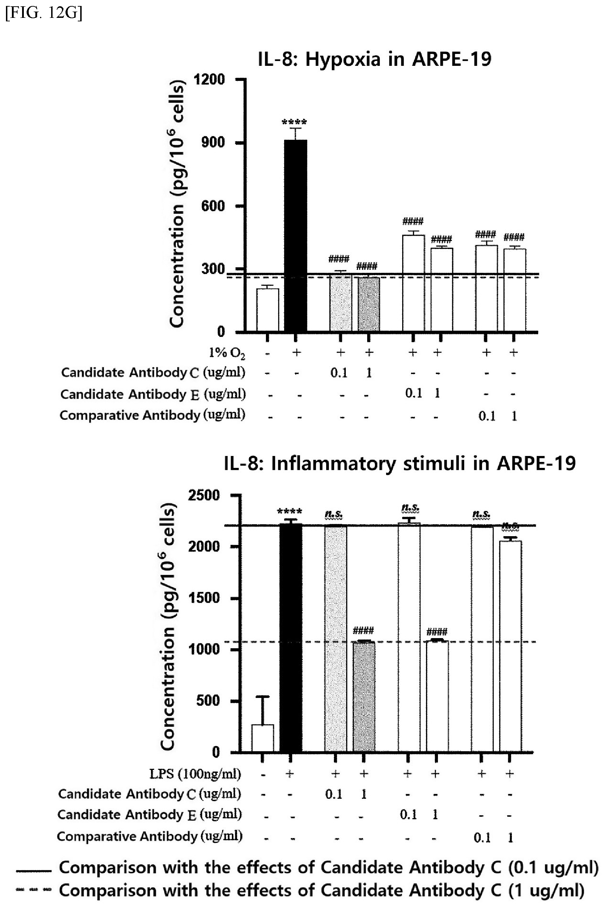

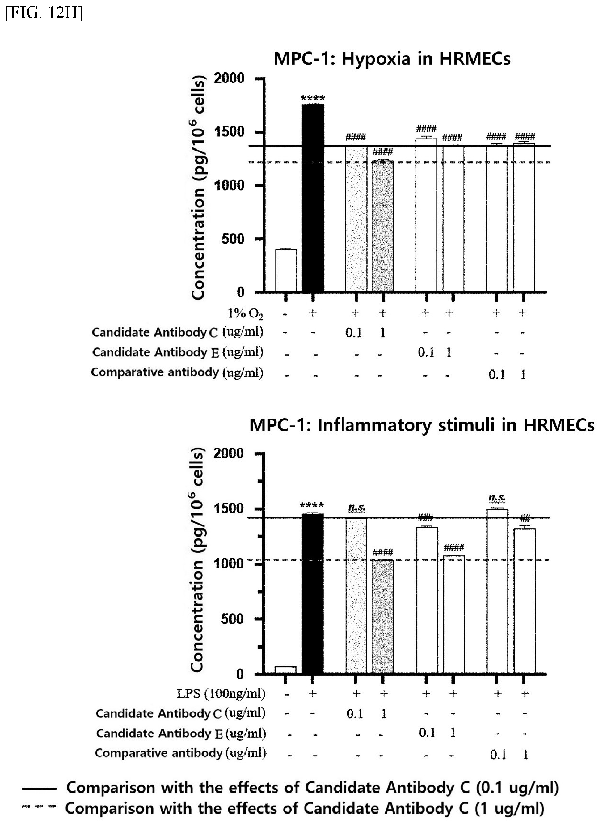

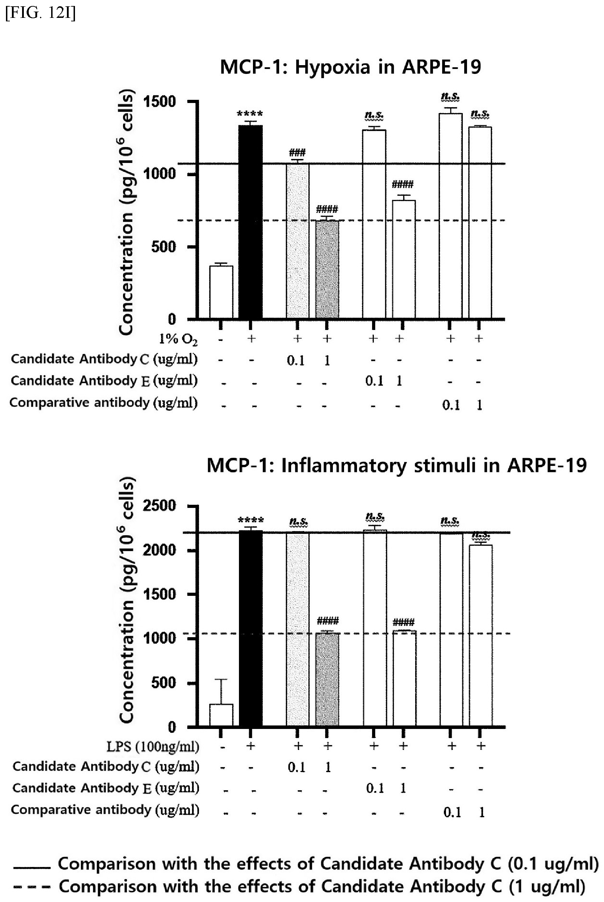

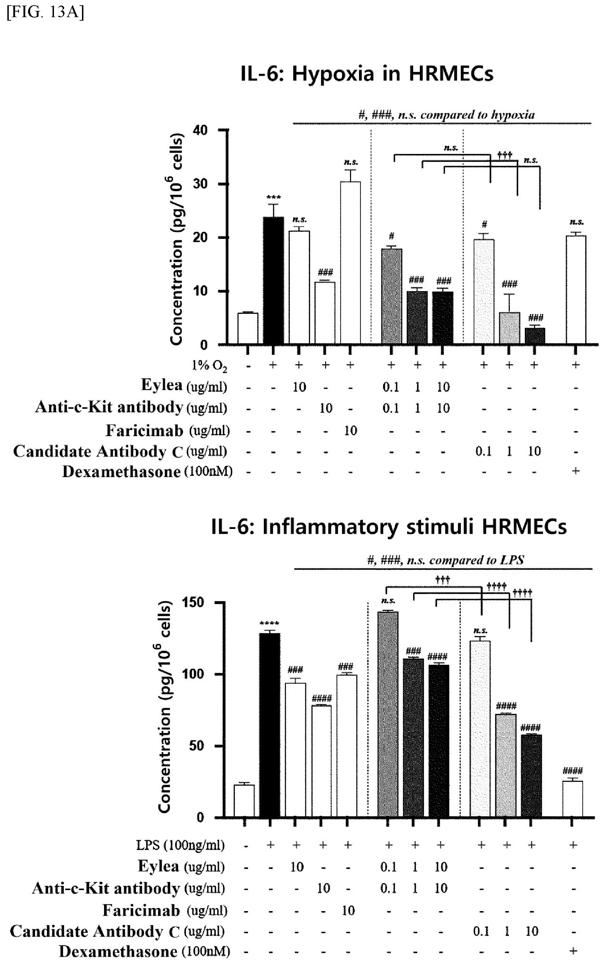

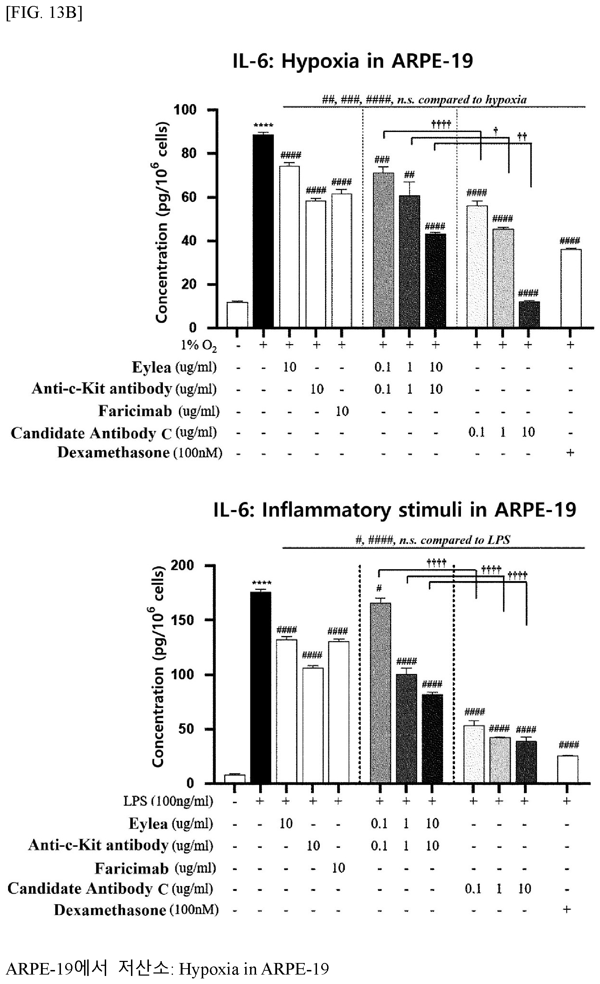

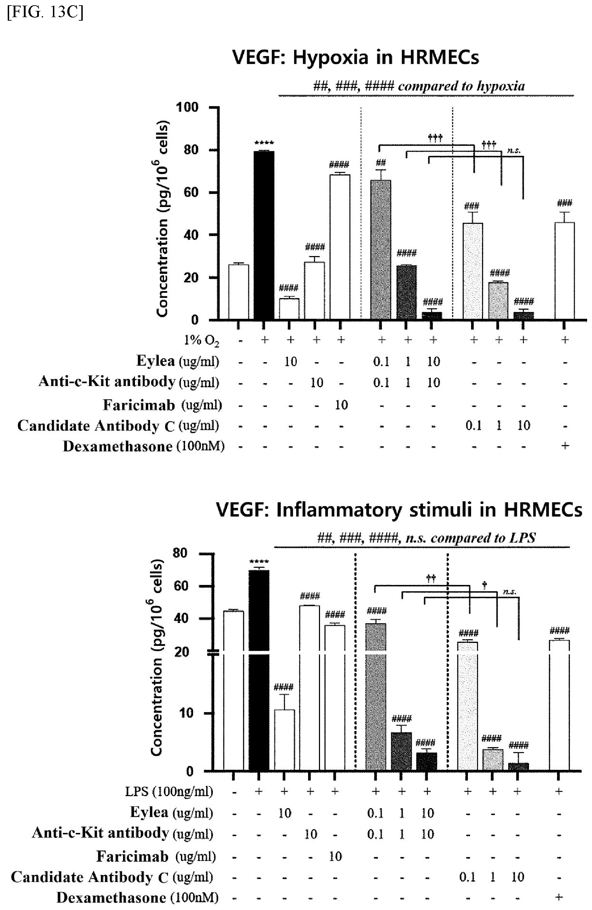

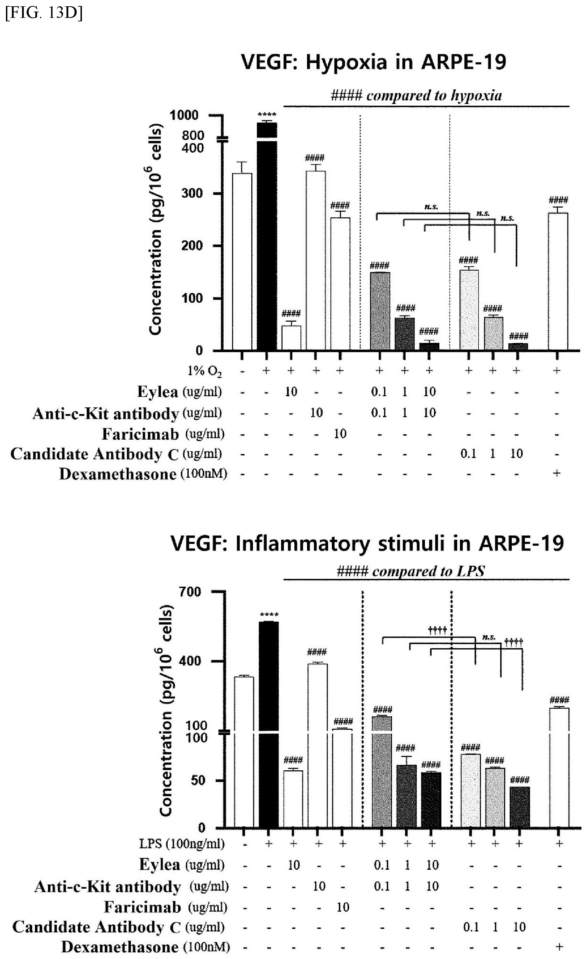

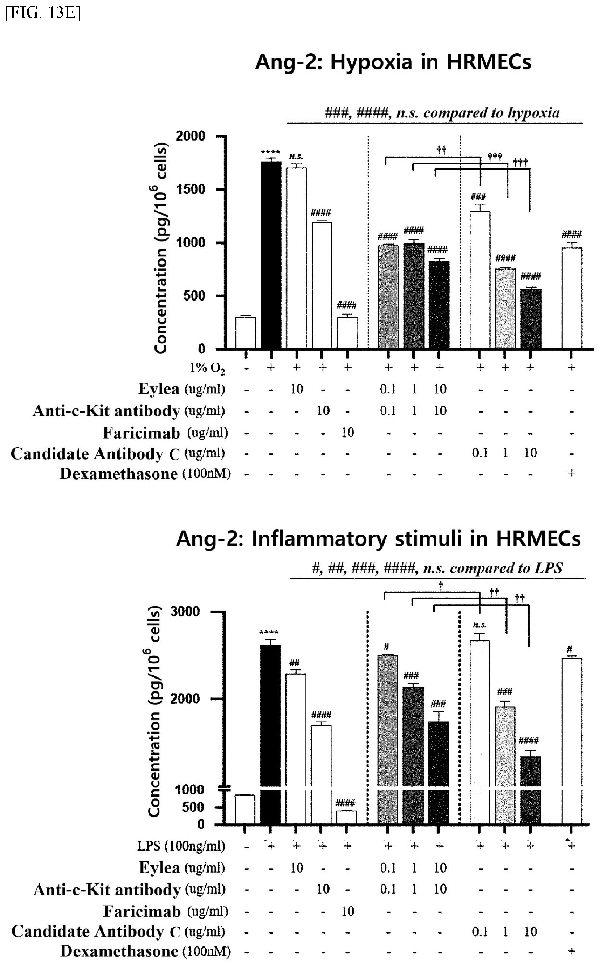

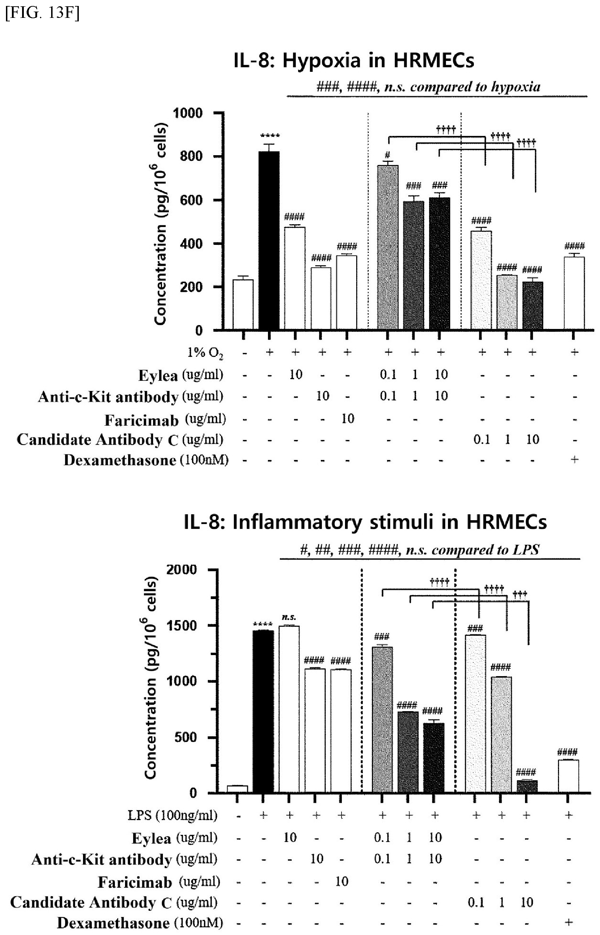

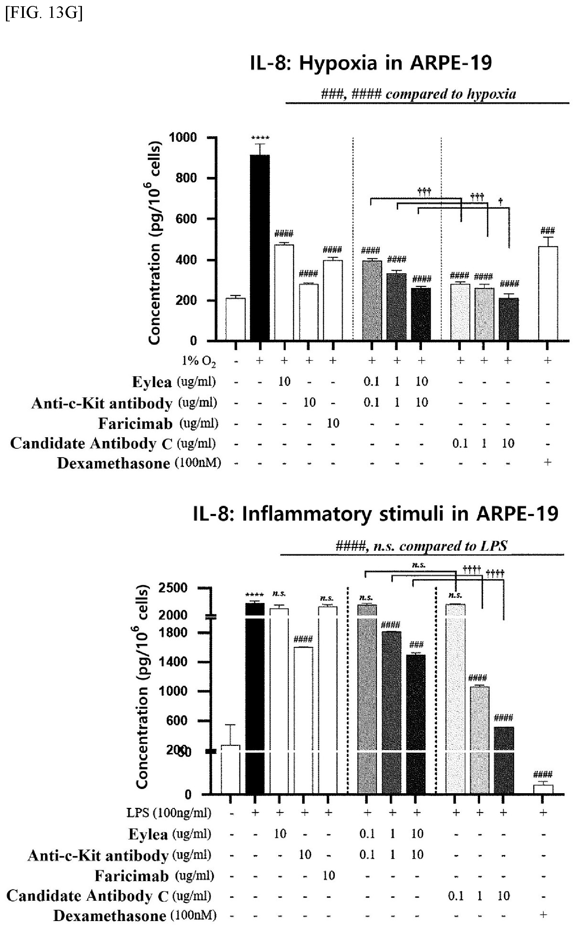

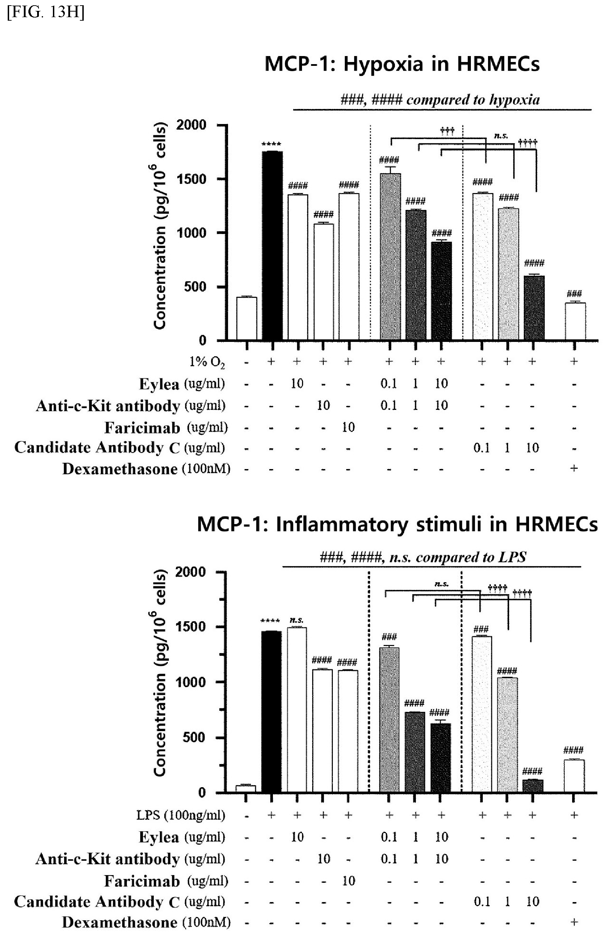

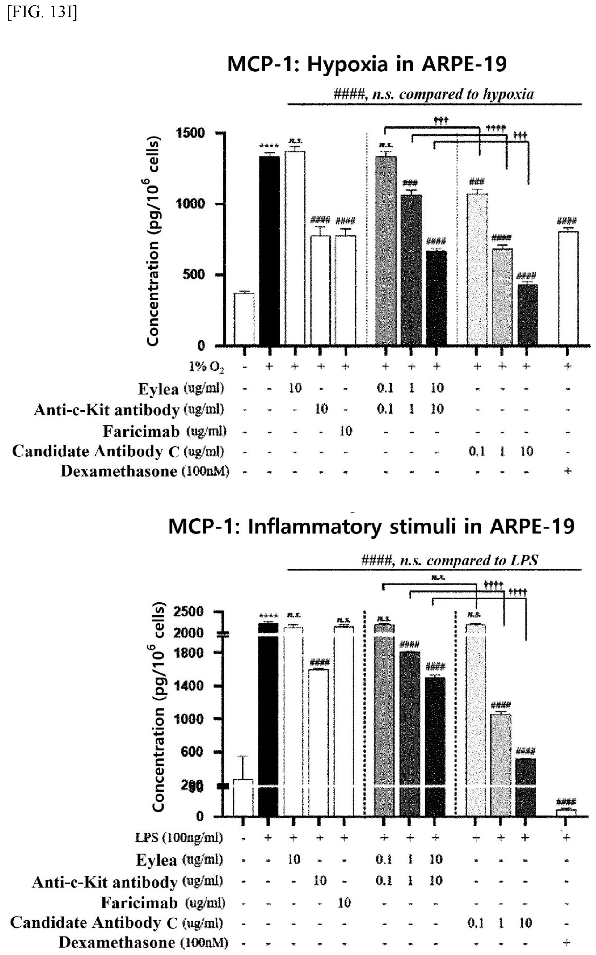

As confirmed in FIGS. 10A to 10B, 11A to 11R and 12A to 12I, Candidate Antibodies A, B, C and E dose-dependently reduced the levels of cytokines induced by hypoxia or LPS in HRMEC and ARPE19 cells *in vitro*, and as confirmed in FIGS. 11A to 11R and 13A to 13I, Candidate Antibodies B and C showed significantly superior cytokine inhibitory effects compared to the combined treatment group of Eylea and anti-c-Kit antibodies and the Faricimab treatment group.

### [Example 7]

### In vivo confirmation of efficacy of bispecific antibodies

### 7-1. Laser-induced choroidal neovascularization (LCNV) and intravitreal injection

General anesthesia was induced in C57BL/6 mice by intraperitoneal injection of 10 mg/kg xylazine (Rompun^{®}, Bayer Korea) and 100 mg/kg ketamine (Ketamine^{®}, Yuhan). Local anesthesia was induced by applying an anesthetic eye drop (Alcaine^{®}, Alcon Korea) to the eye, and then, pupillary dilation was induced by applying a mydriatic agent (Tropherine Eyedrops^{®}, Alcon Korea). After placing the mouse on the stage, the imaging camera of the Micron-IV system (Micron-IV, Phoenix, CA) was focused on the fundus. Lubricant was applied to the eye, and the lens of the Micron-IV system was contacted to the cornea. CNV induction conditions were as follows: wavelength 532 nm, diameter 50 µm, duration 80 ms, and output level 240 mW. One drop of antibiotic (Tobrex^{®}, Novartis Korea) was placed in the eye.

LCNV were generated on experimental Day 0 (D0) in 6- to 8-week-old male C57BL/6 mice, recapitulating the subretinal neovascularization characteristic of human wet age-related macular degeneration (wAMD). One day after laser administration (D1), each bispecific antibody was administered via intravitreal injection.

### 7-2. Fluorescein angiography (qFA)

Lesion patency was assessed by quantitative fluorescein angiography (qFA) on Days 7 and 13 (D7 and D13) post-laser administration. Animals were anesthetized with an IP injection of ketamine/xylazine, and 10% sodium fluorescein was administered via IP injection at a dose of 10 µL/g body weight. Fluorescent fundus images were captured for one eye of each animal with a Micron IV imaging system during lesion filling with fluorescein, and again 2 minutes later. Fluorescence intensity of one lesion per eye was quantified via the integrated density function using ImageJ software. The difference in integrated density between the two hours post-injection was reported as a readout of lesion leakage.

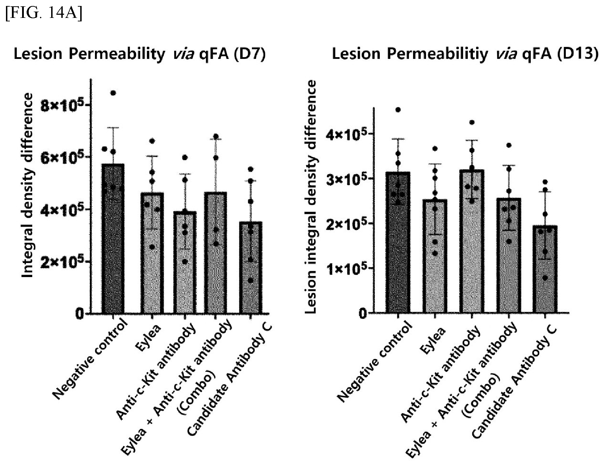

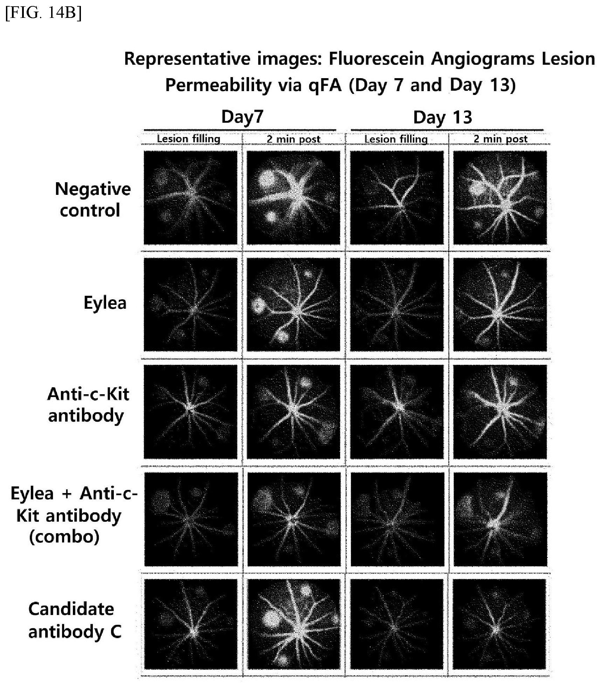

As confirmed in FIGS. 14A and 14B, Candidate Antibody C showed a much more excellent effect on vascular permeability than the combined treatment with Eylea and anti-c-Kit antibodies.

### 7-3. CNV lesions

On Day 14 (D14) after laser administration, animals were euthanized, eyes were enucleated, and choroidal tissue was dissected. The choroids were dissected and stained with FITC-conjugated isolectin B4 (IB4) to visualize the endothelial cells including the CNV lesions before flat-mounting. The area of CNV lesions at each Bruch's membrane rupture site was quantified by using computer-assisted image analysis of high-resolution digital images of these choroidal flat-mounts. The areas for all lesions in each eye were averaged and reported as a single data point per eye (two data points per mouse).

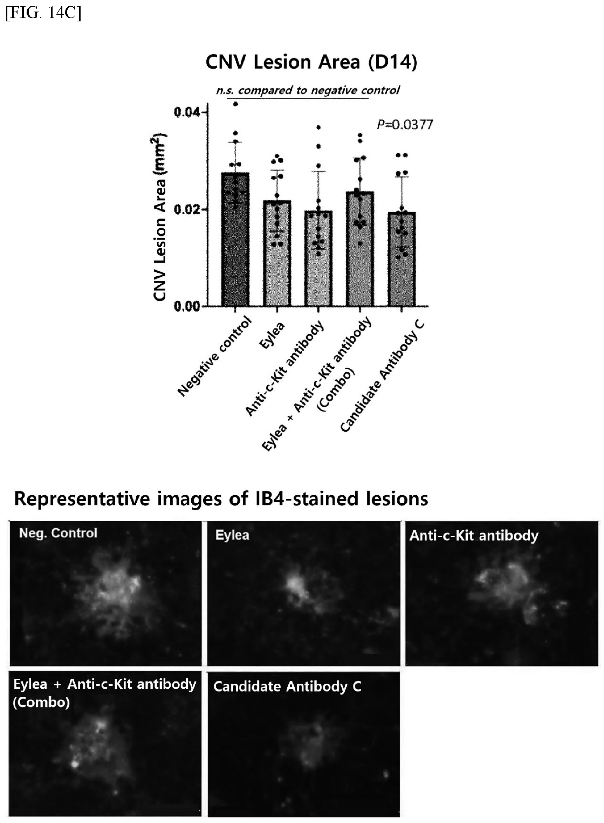

As confirmed in FIG. 14C, Candidate Antibody C showed a statistically significant reduction in CNV lesion area compared to the combined treatment with Eylea and anti-c-Kit antibody.

All qFA and CNV area measurements were analyzed by one-way ANOVA and Tukey post hoc tests, each mean was compared to all other means, and multiple comparisons were corrected using statistical hypothesis testing.

### 7-4. Retinal image evaluation (FFA/OCT)

In order to conduct another efficacy evaluation test, each bispecific antibody was administered into the vitreous of mice at different concentrations (0.5, 1, or 2 µg/µL per eye) immediately after LCNV was induced in Example 7-1, and qFA and optical coherence tomography (OCT) were performed 10 days (D10) after laser administration. For efficacy comparison, the vehicle-treated negative control group was administered with the formulation buffer, and the reference drug-treated positive control group was administered with aflibercept at 2 or 20 µg/µL per eye.

The extent of leakage in LCNV lesions was analyzed by using the Corrected Total Fluorescence (CTF) value based on fundus fluorescein angiography (FFA) images. Measurement of LCNV lesion volume based on OCT images was performed by using the 'InSight' and 'Image-J' programs.

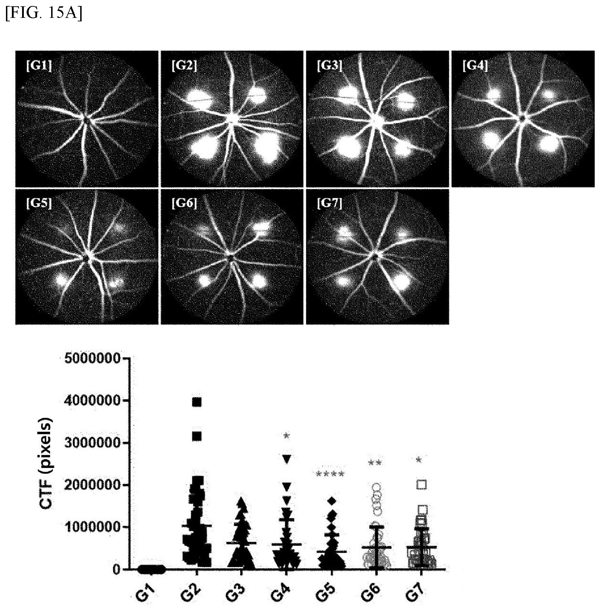

As confirmed in FIG. 15A and Table 32, the Candidate Antibody A administration group showed a significant decrease in CTF value compared to the vehicle-treated negative control group, and there was no statistically significant difference according to the dosage.

**[Table 32]**

| **Group** | **CTF (pixel)** | ***P-value vs G2*** |
|---|---|---|
| G1 (Sham) | 0 ± 0 | |
| G2 (Vehicle-treated negative control) | 1027397 ± 840775 | |
| G3 (Aflibercept 2 µg/µL/eye) | 619330 ± 441069 | 0.3522 |
| G4 (Aflibercept 20 µg/µL/eye) | 587441 ± 586826 | 0.0321 |
| G5 (Candidate Antibody A 0.5 µg/µL/eye) | 417531 ± 392373 | < 0.0001 |
| G6 (Candidate Antibody A 1 µg/µL/eye) | 519124 ± 478599 | 0.0033 |
| G7 (Candidate Antibody A 2 µg/µL/eye) | 522365 ± 434247 | 0.0113 |

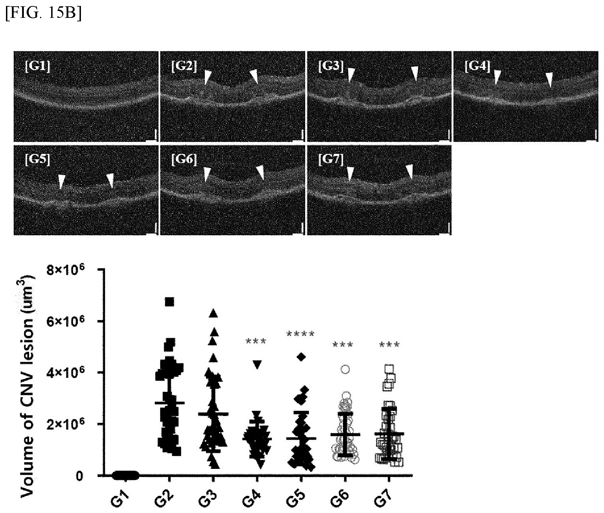

In addition, as confirmed in FIG. 15B and Table 33, the Candidate Antibody A administration group showed a significant decrease in CNV lesion volume compared to the vehicle-treated negative control group.

**[Table 33]**

| Group | Volume (µm³) | *P-value vs G2* |
|---|---|---|
| G1 (Sham) | 0 ± 0 | |
| G2 (Vehicle-treated negative control) | 2833216 ± 1407777 | |
| G3 (Aflibercept 2 µg/µL/eye) | 2389355 ± 1439831 | >0.9999 |
| G4 (Aflibercept 20 µg/µL/eye) | 1413476 ± 679812 | 0.0002 |
| G5 (Candidate Antibody A 0.5 µg/µL/eye) | 1432213 ± 1012822 | < 0.0001 |
| G6 (Candidate Antibody A 1 µg/µL/eye) | 1607532 ± 829899 | 0.0004 |
| G7 (Candidate Antibody A 2 µg/µL/eye) | 1613292 ± 970409 | 0.0005 |

Data in Tables 32 and 33 were expressed as mean ± S.D (G1: n = 48, G2: n = 37, G3: n = 37, G4: n = 33, G5: n = 37, G6: n = 43, and G7: n = 37). Statistically significant differences were compared with G2 (vehicle-treated negative control) through the Kruskal-Wallis multiple comparison test.

## Claims

1. A bispecific antibody that specifically binds to c-Kit and VEGF, comprising (a) and (b) below:
(a) a first antigen binding site that specifically binds to c-Kit, comprising a heavy chain variable region comprising a heavy chain CDR1 comprising an amino acid sequence of SEQ ID NO: 2, a heavy chain CDR2 comprising an amino acid sequence of SEQ ID NO: 4, and a heavy chain CDR3 comprising an amino acid sequence of SEQ ID NO: 6; and a light chain variable region comprising a light chain CDR1 comprising an amino acid sequence of SEQ ID NO: 13, a light chain CDR2 comprising an amino acid sequence of SEQ ID NO: 15, and a light chain CDR3 comprising an amino acid sequence of SEQ ID NO: 17; and
(b) a second antigen binding site that specifically binds to VEGF, wherein a soluble extracellular domain of VEGF and an Fc domain of IgG are fused.

2. The bispecific antibody of claim 1, wherein the first antigen binding site comprises a heavy chain variable region comprising an amino acid sequence having 90% or more sequence homology with an amino acid sequence of SEQ ID NO: 27 and a light chain variable region comprising an amino acid sequence having 90% or more sequence homology with an amino acid sequence of SEQ ID NO: 28.

3. The bispecific antibody of claim 1, wherein the soluble extracellular domain comprises immunoglobulin-like domain 2 of a first VEGF receptor and immunoglobulin-like domain 3 of a second VEGF receptor.

4. The bispecific antibody of claim 1, wherein the second antigen binding site comprises an amino acid sequence of SEQ ID NO: 21.

5. The bispecific antibody of claim 1, wherein the second antigen binding site is bound to a heavy chain C-terminus or N-terminus of the first antigen binding site by a peptide linker; or
the second antigen binding site is bound to a light chain C-terminus of the first antigen binding site by a peptide linker.

6. The bispecific antibody of claim 2, wherein the peptide linker is GGGGG (SEQ ID NO: 20), GGGGGG (SEQ ID NO: 67), GGGGGGG (SEQ ID NO: 68), GGGGGGGG (SEQ ID NO: 23), GGGGGGGGG (SEQ ID NO: 69) or GGGGGGGGGG (SEQ ID NO: 70).

7. The bispecific antibody of claim 1, wherein an Fc domain of the first antigen binding site comprises:
(a) wild-type IgG Fc;
(b) an Fc variant comprising L234A and L235E amino acid substitutions;
(c) an Fc variant comprising L235A and G237A amino acid substitutions; or
(d) an Fc variant comprising L234A, L235A, I253A, H310A, P329G and H435A amino acid substitutions,
wherein residues are numbered according to the EU index of Kabat.

8. The bispecific antibody of claim 1, wherein the bispecific antibody is a tetravalent antibody.

9. The bispecific antibody of claim 1, wherein the first antigen binding site is a full-length divalent antibody, and the second antigen binding site consists of two VEGF antagonists in which a soluble extracellular domain of VEGF and an Fc domain of IgG are fused.

10. A nucleic acid molecule, encoding the bispecific antibody that specifically binds to c-Kit and VEGF according to any one of claims 1 to 9.

11. A recombinant vector, comprising the nucleic acid molecule according to claim 10.

12. A transformant, comprising the recombinant vector according to claim 11.

13. A method for producing a bispecific antibody that specifically binds to c-Kit and VEGF, comprising steps (a) and (b) below:
(a) culturing the transformant of claim 12; and
(b) recovering a bispecific antibody that specifically binds to c-Kit and VEGF from a culture.

14. A pharmaceutical composition for preventing or treating an angiogenic disease, comprising the bispecific antibody that specifically binds to c-Kit and VEGF according to any one of claims 1 to 9 as an active ingredient.

15. The pharmaceutical composition of claim 14, wherein the angiogenic disease is selected from the group consisting of cancer, leukemia, ocular vascular disease, rheumatoid arthritis, psoriasis, chronic wound, chronic inflammation, hemangioma, angiofibroma, vascular malformation, arteriosclerosis, vascular adhesion, vasculitis, pyogenic granuloma, blistering disease, pulmonary hypertension, asthma, nasal polyp, infectious disease, inflammatory bowel disease, periodontal disease, peritoneal adhesion, endometrium, uterine hemorrhage, ovarian cyst, osteomyelitis, osteopathy, sepsis and autoimmune disease.

16. The pharmaceutical composition of claim 15, wherein the cancer is selected from the group consisting of bone cancer, lung cancer, head cancer, neck cancer, thyroid cancer, parathyroid cancer, non-small cell lung cancer, stomach cancer, liver cancer, pancreatic cancer, skin cancer, cutaneous or intraocular melanoma, rectal cancer, proanal cancer, colon cancer, uterine cancer, breast cancer, ovarian cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, adrenal cancer, soft tissue sarcoma, urethral cancer, penile cancer, prostate cancer, bladder cancer, kidney cancer, ureteral cancer, renal cell carcinoma, renopelvic carcinoma, central nervous system tumor, central nervous system lymphoma, spinal cord tumor, glioblastoma, brainstem glioma and pituitary adenoma.

17. The pharmaceutical composition of claim 15, wherein the ocular vascular disease is selected from the group consisting of diabetic retinopathy, macular degeneration, age-related macular degeneration, glaucoma, glaucomatous retinitis pigmentosa, choroidal neovascularization, retinopathy of immaturity, corneal dystrophy and retinal separation.

18. A kit for diagnosing an angiogenic disease, comprising the bispecific antibody that specifically binds to c-Kit and VEGF according to any one of claims 1 to 9.

19. A method for preventing or treating an angiogenic disease, comprising administering a therapeutically effective amount of the bispecific antibody that specifically binds to c-Kit and VEGF according to any one of claims 1 to 9 to a subject in need thereof.

20. Use of the bispecific antibody that specifically binds to c-Kit and VEGF according to any one of claims 1 to 9 in the manufacture of a medicament for preventing or treating an angiogenic disease.
